(19) Europäisches Patentamt
European Patent Office
Office européen des brevets

(11) EP 4 151 235 A1

(12) # EUROPEAN PATENT APPLICATION
published in accordance with Art. 153(4) EPC

(43) Date of publication:
**22.03.2023 Bulletin 2023/12**

(21) Application number: **21804105.1**

(22) Date of filing: **12.05.2021**

(51) International Patent Classification (IPC):
***A61K 47/68*** (2017.01) ***A61K 31/4745*** (2006.01)
***A61K 45/06*** (2006.01) ***A61K 49/00*** (2006.01)
***A61K 51/10*** (2006.01) ***A61P 35/00*** (2006.01)

(52) Cooperative Patent Classification (CPC):
**A61K 31/4745; A61K 45/06; A61K 47/68; A61K 49/00; A61K 51/10; A61P 35/00**

(86) International application number:
**PCT/CN2021/093348**

(87) International publication number:
**WO 2021/228141 (18.11.2021 Gazette 2021/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.05.2020 CN 202010410633**

(71) Applicant: **Sichuan Kelun-Biotech Biopharmaceutical Co., Ltd.**
**Chengdu, Sichuan 611138 (CN)**

(72) Inventors:
- **XIAO, Liang**
  **Chengdu, Sichuan 611138 (CN)**
- **XUE, Tongtong**
  **Chengdu, Sichuan 611138 (CN)**
- **WANG, Jingyi**
  **Chengdu, Sichuan 611138 (CN)**
- **WANG, Cheng**
  **Chengdu, Sichuan 611138 (CN)**
- **LIU, Dengnian**
  **Chengdu, Sichuan 611138 (CN)**
- **TIAN, Qiang**
  **Chengdu, Sichuan 611138 (CN)**
- **SONG, Shuai**
  **Chengdu, Sichuan 611138 (CN)**

(74) Representative: **Jones Day**
**Rechtsanwälte, Attorneys-at-Law, Patentanwälte**
**Prinzregentenstrasse 11**
**80538 München (DE)**

Remarks:
The complete document including Reference Table(s) and the Sequence Listing(s) can be downloaded from the EPO website

(54) **ANTIBODY DRUG CONJUGATE, PREPARATION METHOD THEREFOR AND USE THEREOF**

(57) Related is an anti-CLDN 18.2 antibody-drug conjugate, preparation method and use thereof. The antibody-drug conjugate and the composition thereof is capable of effectively binding CLDN 18.2 and inhibiting the growth of Claudin 18.2 positive tumors. Further related is a pharmaceutical composition comprising the antibody-drug conjugate or the composition, and use of the pharmaceutical composition in the preparation of a medicament for preventing and/or treating tumors.

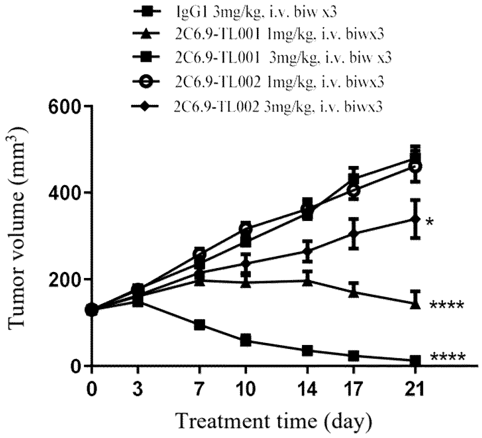


Fig. 12A



Processed by Luminess, 75001 PARIS (FR)

## Description

The application is based on and claims priority to CN application No. 202010410633.5 with filing date of May 15, 2020, the disclosure of which is hereby introduced into the application in its entirety.

## FIELD OF THE INVENTION

The invention belongs to the technical field of biomedicine, specifically relating to an anti-Claudin 18.2 antibody-drug conjugate (ADC) and a composition comprising the ADC, and use thereof.

## BACKGROUND OF THE INVENTION

The treatment of gastric cancer is predominated by surgical excision. For unresectable or recurrent gastric cancer, chemotherapy is the main therapy, but under the state of the art, it can only relieve symptoms and prolong survival. At present, there is no universally recognized standard chemotherapy regimen for advanced gastric cancer. In terms of bio-macromolecular drugs, except for Trastuzumab, Ramucirumab, Pembrolizumab, etc., which have been approved, most of other targeted drugs for gastric cancer are unsatisfactory in efficacy or still in the early stages of clinical research. The core weakness of existing drugs includes the unmet clinical needs of non-surgical treatment, very limited treatment options for advanced or recurrent gastric cancer, extremely poor prognosis, and high death rate. Due to the high incidence of gastric cancer, the current demands for gastric cancer drug are great.

Claudin 18.2 (CLDN 18.2) is a member of the Claudin protein family. Claudin family proteins are a type of proteins that mediate tight junctions between cells. Different Claudin subtypes are expressed in different tissues and are associated with different types of cancer. The expression of Claudin 18.2 in normal tissues is restricted in gastric mucosal cells. Claudin 18.2 is highly expressed in 70% of primary gastric adenocarcinoma and its metastases; it is also expressed in other cancers such as pancreatic cancer, esophageal cancer, and non-small cell lung cancer (NSCLC). The highly specific expression of Claudin 18.2 in tumor tissues makes Claudin 18.2 a very good target for tumor immunotherapy.

## SUMMARY OF THE INVENTION

The inventor of the present application described an antibody with high specificity and high affinity to Claudin 18.2 in PCT/CN2019/126495, the entire content of which is incorporated herein by reference and becomes a part of the present application. Through massive creative work, the inventor of the present application further invented Claudin 18.2 antibody-drug conjugate (ADC), which provides a safe and effective drug option for the treatment of tumors.

Specifically, the inventors conjugate an antibody with high affinity that specifically binds to Claudin 18.2 to a bioactive molecule through a linker, so as to obtain a class of Claudin 18.2 targeting ADCs and a composition comprising the ADC. The ADC or composition has high killing activity against some tumor cells expressed by Claudin 18.2. In animal experiments (*in vivo*), the ADC can effectively inhibit the growth of Claudin 18.2 positive tumors, especially gastric cancer or gastric adenocarcinoma, and is very safe in use.

### Antibody-drug conjugates (ADCs)

In one aspect, the invention provides an antibody-drug conjugate (ADC) that specifically binds to human CLDN 18.2, the structure of which is shown in formula (I):

$$(D\text{-}L)_{\gamma}\text{-}A \qquad \text{Formula (I)}$$

wherein D is a fragment of a bioactive molecule; L is a linker;

$\gamma$ is an integer from 1 to 10; preferably, $\gamma$ is an integer from 1 to 8 (for example, 1, 2, 3, 4, 5, 6, 7 or 8);

A is an antibody or antigen-binding fragment thereof that specifically binds to human CLDN 18.2.

In some embodiments, the antibody or antigen-binding fragment thereof that specifically binds to human CLDN18.2 comprises:

(1) the following three heavy chain CDRs: CDR-H1, CDR-H2, and CDR-H3 comprised in the VH (heavy chain variable region) as set forth in SEQ ID NO: 13, 14 or 23; and/or

the following three light chain CDRs: CDR-L1, CDR-L2, and CDR-L3 comprised in the VL (light chain variable region) as set forth in SEQ ID NO: 15 or 24;
or

(2) the following three heavy chain CDRs: the CDR-H1 described in (1) or a variant thereof containing an amino acid mutation, and the CDR-H2 described in (1) or a variant thereof containing an amino acid mutation, and the CDR-H3 described in (1) or a variant thereof containing an amino acid mutation; and/or

the following three light chain CDRs: the CDR-L1 described in (1) or a variant thereof containing an amino acid mutation, the CDR-L2 described in (1) or a variant thereof containing an amino acid mutation, and the CDR-L3 described in (1) or a variant thereof containing an amino acid mutation;

wherein at least one of the three heavy chain CDRs and/or three light chain CDRs described in (2) contains an amino acid mutation compared with the corresponding CDR in (1), and the amino acid mutation is the substitution, deletion, or addition of one or more amino acids (e.g., substitution, deletion, or addition of 1, 2, or 3 amino acids); the antibody or antigen-binding fragment thereof containing the mutation is still capable of specifically binding to CLDN 18.2, preferably, to human CLDN 18.2; preferably, the substitution is a conservative substitution.

In some embodiments, the CDRs are defined according to the IMGT or AbM numbering system.
In some embodiments, the antibody or antigen-binding fragment thereof further includes the framework regions (FRs) from human immunoglobulins.
In some embodiments, the antibody or antigen-binding fragment thereof comprises:

(1-1) the following three heavy chain CDRs defined by the IMGT numbering system: CDR-H1 having the sequence of SEQ ID No: 1, CDR-H2 having the sequence of SEQ ID No: 2 or 21, and CDR-H3 having the sequence of SEQ ID No: 3; and/or

the following three light chain CDRs defined by the IMGT numbering system: CDR-L1 having the sequence of SEQ ID No: 4, CDR-L2 having the sequence of SEQ ID No: 5, and CDR-L3 having the sequence of SEQ ID No: 6;
or

(1-2) the following three heavy chain CDRs: the CDR-H1 described in (1-1) or a variant thereof containing an amino acid mutation, and the CDR-H2 described in (1-1) or a variant thereof containing an amino acid mutation, and the CDR-H3 described in (1-1) or a variant thereof containing an amino acid mutation; and/or

the following three light chain CDRs: the CDR-L1 described in (1-1) or a variant thereof containing an amino acid mutation, and the CDR-L2 described in (1-1) or a variant thereof containing an amino acid mutation, and the CDR-L3 described in (1-1) or a variant thereof containing an amino acid mutation;

wherein at least one of the three heavy chain CDRs and/or three light chain CDRs contains an amino acid mutation compared with the corresponding CDR in (1-1), and the amino acid mutation is substitution, deletion, or addition of one or more amino acids (e.g., substitution, deletion, or addition of 1, 2, or 3 amino acids); the antibody or antigen-binding fragment thereof containing the mutation is still capable of specifically binding to human CLDN 18.2; preferably, the substitution is a conservative substitution;
or

(2-1) the following three heavy chain CDRs defined by the AbM numbering system: CDR-H1 having the sequence of SEQ ID No: 7, CDR-H2 having the sequence of SEQ ID No: 8 or 22, and CDR-H3 having the sequence of SEQ ID No 9; and/or

the following three light chain CDRs defined by the AbM numbering system: CDR-L1 having the sequence of SEQ ID No: 10, CDR-L2 having the sequence of SEQ ID No: 11, and CDR-L3 having the sequence of SEQ ID No: 12;
or

(2-2) the following three heavy chain CDRs: the CDR-H1 described in (2-1) or a variant thereof containing an amino acid mutation, and the CDR-H2 described in (2-1) or a variant thereof containing an amino acid mutation, and the CDR-H3 described in (2-1) or a variant thereof containing an amino acid mutation; and/or

the following three light chain CDRs: the CDR-L1 described in (2-1) or a variant thereof containing an amino acid mutation, and the CDR-L2 described in (2-1) or a variant thereof containing an amino acid mutation, and the CDR-L3 described in (2-1) or a variant thereof containing an amino acid mutation;

wherein at least one of the three heavy chain CDRs and/or three light chain CDRs contains an amino acid mutation compared with the corresponding CDR in (2-1), and the amino acid mutation is substitution, deletion, or addition of one or more amino acids (e.g., substitution, deletion, or addition of 1, 2, or 3 amino acids); the antibody or antigen-binding fragment thereof containing the mutation is still capable of specifically binding to human CLDN 18.2; preferably, the substitution is a conservative substitution.

In some embodiments, the antibody or antigen-binding fragment thereof further includes the framework regions (FRs) from human immunoglobulins.

In some embodiments, the antibody or antigen-binding fragment thereof comprises:

(1) the following VH and/or VL, wherein CDRs are defined according to the IMGT numbering system:

(1-1): VH comprising the following 3 CDRs: CDR-H1 having the sequence of SEQ ID No: 1, CDR-H2 having the sequence of SEQ ID No: 2 or 21, and CDR-H3 having the sequence of SEQ ID No: 3; and/or

VL comprising the following 3 CDRs: CDR-L1 having the sequence of SEQ ID No: 4, CDR-L2 having the sequence of SEQ ID No: 5, and CDR-L3 having the sequence of SEQ ID No: 6;
or

(1-2): compared with the VH or VL described in (1-1), at least one CDR contains a mutation, which is the substitution, deletion or addition of one or more amino acids, or any combination thereof (for example, the substitution, deletion or addition of 1, 2 or 3 amino acids, or any combination thereof); preferably, the substitution is a conservative substitution; the antibody or antigen-binding fragment containing the mutation is still capable of specifically binding to CLDN 18.2; preferably, to human CLDN 18.2;
or

(2) the following VH and/or VL, wherein CDRs are defined according to the AbM numbering system:

(2-1): VH comprising the following 3 CDRs: CDR-H1 having the sequence of SEQ ID No: 7, CDR-H2 having the sequence of SEQ ID No: 8 or 22, and CDR-H3 having the sequence of SEQ ID No: 9; and/or

VL comprising the following 3 CDRs: CDR-L1 having the sequence of SEQ ID No: 10, CDR-L2 having the sequence of SEQ ID No: 11, and CDR-L3 having the sequence of SEQ ID No: 12;
or

(2-2): compared with the VH or VL described in (2-1), at least one CDR contains a mutation, which is the substitution, deletion or addition of one or more amino acids, or any combination thereof (for example, the substitution, deletion or addition of 1, 2 or 3 amino acids, or any combination thereof); preferably, the substitution is a conservative substitution; the antibody or antigen-binding fragment containing the mutation is still capable of specifically binding to CLDN 18.2; preferably, to human CLDN 18.2.

In some embodiments, the VH and/or VL of the antibody or antigen-binding fragment thereof includes the framework regions (FRs) from human or murine immunoglobulins.

In some embodiments, the antibody or antigen-binding fragment thereof comprises:

(1) the VH as set forth in SEQ ID NO: 13 or 14; and/or the VL as set forth in SEQ ID NO: 15;
or

(2) compared with the VH described in (1), the VH comprised in the antibody or antigen-binding fragment thereof has at least 70%, at least 80%, at least 85%, at least 90%, at least 91%, at least 92%, at least 93%, at least 94%, at least 95%, at least 96%, at least 97%, at least 98%, at least 99%, or 100% identity; and/or, compared with the VL described in (1), the VL compirsed in the antibody or antigen-binding fragment thereof has at least 70%, at least 80%, at least 85%, at least 90%, at least 91%, at least 92%, at least 93%, at least 94%, at least 95%, at least 96%, at least 97%, at least 98%, at least 99%, or 100% identity; the antibody or antigen-binding fragment thereof having

identity to the VL or VH described in (1) is still capable of specifically binding to CLDN 18.2, preferably, to human CLDN 18.2;
or

(3) compared with the VH described in (1), the VH comprised in the antibody or antigen-binding fragment thereof has the substitution, deletion or addition of one or more amino acids, or any combination thereof (for example, the substitution, deletion or addition of 1, 2, 3, 4 or 5 amino acids, or any combination thereof); and/or, compared with the VL described in (1), the VL comprised in the antibody or antigen-binding fragment thereof has the substitution, deletion or addition of one or more amino acids, or any combination thereof (for example, the substitution, deletion or addition of 1, 2, 3, 4 or 5 amino acids, or any combination thereof); preferably, the substitution is a conservative substitution. The antibody or antigen-binding fragment thereof containg the mutation is still capable of specifically binding to CLDN 18.2; preferably, to human CLDN 18.2.

In any of the above aspects, the antibody or antigen-binding fragment thereof may further comprise a constant region derived from a mammalian (e.g., murine or human) immunoglobulin or a variant thereof.
In some embodiments, the antibody may comprise:

(1) the CH (heavy chain constant region) of human immunoglobulin or a variant thereof, wherein the variant comprises a substitution, deletion or addition of one or more amino acids compared with the wild type sequence from which it is derived (for example, the substitution, deletion or addition of up to 20, up to 15, up to 10, or up to 5 amino acids; for example, the substitution, deletion or addition of 1, 2, 3, 4, or 5 amino acids); and/or

(2) the CL (light chain constant region) of human immunoglobulin or a variant thereof, wherein the variant comprises a substitution, deletion or addition of one or more amino acids compared with the wild type sequence from which it is derived (for example, the substitution, deletion or addition of up to 20, up to 15, up to 10, or up to 5 amino acids; for example, the substitution, deletion or addition of 1, 2, 3, 4 or 5 amino acids).

In some embodiments, the constant region is altered, e.g., mutated, to modify the properties of the anti-CLDN18.2 antibody (e.g., to alter one or more of the following properties: binding of Fc receptor, antibody glycosylation, amount of cysteine residues, functions on effector cells or complements). At least one amino acid residue in the constant region of the antibody can be replaced with other ones to alter the function. For example, effector function can be altered (e.g., enhanced) by altering the antibody affinity to an effector ligand (such as FcR or complement C1q). In some embodiments, the constant region is altered (e.g., enhanced) to modify the antibody-dependent cell-mediated cytotoxicity (ADCC), complement-dependent cytotoxicity (CDC), and/or antibody-dependent cellular phagocytosis (ADCP).
In some embodiments, the CH is an IgG heavy chain constant region, such as an IgG1, IgG2, IgG3, or IgG4 heavy chain constant region.
In some embodiments, the antibody or antigen-binding fragment thereof comprises the heavy chain constant region of human IgG1.
In some embodiments, the antibody or antigen-binding fragment thereof comprises the CH as set forth in SEQ ID NO: 16, or a variant thereof, which has a conservative substitution of up to 20 amino acids compared with SEQ ID NO: 16 (e.g., a conservative substitution of up to 15, 10, or 5 amino acids; e.g., a conservative substitution of 1, 2, 3, 4, or 5 amino acids). The CH containing the mutation retains substantially the same function as SEQ ID NO: 16.
In some embodiments, the CL is selected from the κ or λ light chain constant region.
In some embodiments, the CL is a κ light chain constant region (e.g., human κ light chain).
In some embodiments, the antibody or antigen-binding fragment thereof comprises the CL as set forth in SEQ ID NO: 17, or a variant thereof, which has a conservative substitution of up to 20 amino acids compared to SEQ ID NO: 17 (e.g., a conservative substitution of up to 15, 10, or 5 amino acids; e.g., a conservative substitution of 1, 2, 3, 4, or 5 amino acids). The CL containing the mutation retains substantially the same function as SEQ ID NO: 17.
In some embodiments, the antibody or antigen-binding fragment thereof comprises the CH as set forth in SEQ ID NO: 16 and/or the CL as set forth in SEQ ID NO: 17.
In some embodiments, the antibody includes a heavy chain comprising the VH of the sequence set forth in SEQ ID NO: 13 and the CH as set forth in SEQ ID NO: 16; and
a light chain comprising the VL of the sequence as set forth in SEQ ID NO: 15 and the CL as set forth in SEQ ID NO: 17.
In some embodiments, the antibody includes a heavy chain comprising the VH of the sequence as set forth in SEQ ID NO: 14 and the CH as set forth in SEQ ID NO: 16; and
the light chain comprising the VL of the sequence as set forth in SEQ ID NO: 15 and the CL as set forth in SEQ ID NO: 17.
In some embodiments, the antibody or antigen-binding fragment thereof comprises:

(1) a heavy chain, which comprises the amino acid sequence selected from the group consisting of:

(1-1) the sequence as set forth in SEQ ID NO: 18;

(1-2) a sequence having the substitution, deletion, or addition of one or several amino acids (e.g., the substitution, deletion, or addition of 1, 2, 3, 4, or 5 amino acids) compared with the sequence as set forth in SEQ ID NO: 18; or

(1-3) a sequence having at least 80%, at least 85%, at least 90%, at least 91%, at least 92%, at least 93%, at least 94%, at least 95%, at least 96%, at least 97%, at least 98%, at least 99% or 100% identity to the sequence as set forth in SEQ ID NO: 18;
and

(2) a light chain, comprising the amino acid sequence selected from the group consisting of:

(2-1) the sequence as set forth in SEQ ID NO: 20;

(2-2) a sequence having the substitution, deletion, or addition of one or several amino acids (e.g., the substitution, deletion, or addition of 1, 2, 3, 4, or 5 amino acids) compared with the sequence as set forth in SEQ ID NO: 20; or

(2-3) a sequence having at least 80%, at least 85%, at least 90%, at least 91%, at least 92%, at least 93%, at least 94%, at least 95%, at least 96%, at least 97%, at least 98%, at least 99% or 100% identity to the sequence as set forth in SEQ ID NO:20.

In some embodiments, the substitutions described in (1-2) and (2-2) above are conservative substitutions. The CH containing the mutation retains substantially the same function as SEQ ID NO: 16.

In some embodiments, the antibody or antigen-binding fragment thereof comprises:

(1) a heavy chain, comprising the amino acid sequence selected from the group consisting of:

(1-1) the sequence as set forth in SEQ ID NO: 19;

(1-2) a sequence having the substitution, deletion, or addition of one or several amino acids (e.g., the substitution, deletion, or addition of 1, 2, 3, 4, or 5 amino acids) compared with the sequence as set forth in SEQ ID NO: 19; or

(1-3) a sequence having at least 80%, at least 85%, at least 90%, at least 91%, at least 92%, at least 93%, at least 94%, at least 95%, at least 96%, at least 97%, at least 98%, at least 99% or 100% identity to the sequence as set forth in SEQ ID NO: 19;
and

(2) a light chain, comprising the amino acid sequence selected from the group consisting of:

(2-1) the sequence as set forth in SEQ ID NO:20;

(2-2) a sequence having the substitution, deletion, or addition of one or several amino acids (e.g., the substitution, deletion, or addition of 1, 2, 3, 4, or 5 amino acids) compared with the sequence as set forth in SEQ ID NO: 20; or

(2-3) a sequence having at least 80%, at least 85%, at least 90%, at least 91%, at least 92%, at least 93%, at least 94%, at least 95%, at least 96%, at least 97%, at least 98%, at least 99% or 100% identity to the sequence as set forth in SEQ ID NO:20.

In some embodiments, the substitutions described in (1-2) and (2-2) are conservative substitutions. Preferably, the antibody containing mutations or identity sequences or the antigen binding fragment thereof is still capable of specifically binding to human CLDN18.2.

In some embodiments, the antibody or antigen-binding fragment thereof is selected from scFv, Fab, Fab', $(Fab')_2$, Fv fragment, disulfide bond-linked Fv (dsFv), diabody, bispecific antibody, and multispecific antibody.

In some embodiments, the structure of $(D\text{-}L)_\gamma\text{-}A$ is shown in formula (II):

$$\{D\text{-}[L_1\text{-}(L_2)_{m1}\text{-}(L_3)_{m2}\text{-}(L_4)_{m3}\text{-}E]\}_\gamma\text{-}A \qquad \text{Formula (II)}$$

wherein

$L_1$ is

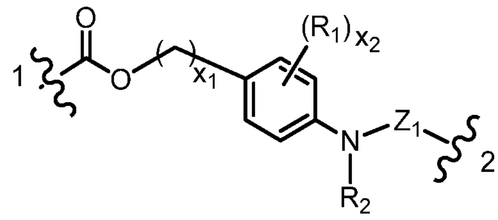

wherein $R_1$ and $R_2$ each independently are hydrogen (such as protium or deuterium), halogen, carboxylic acid group, sulfonic acid group, cyano, $C_{1-6}$ alkyl, halogenated $C_{1-6}$ alkyl, cyano- substituted $C_{1-6}$ alkyl (e.g., $-CH_2CN$), $C_{1-6}$ alkoxy, $C_{2-10}$ alkenyl or $C_{2-10}$ alkynyl; $Z_1$ is an amino acid or a peptide composed of 2-10 amino acids; $x_1$ and $x_2$ each independently are 0, 1, 2, 3, 4, 5 or 6; $L_1$ is connected to D at position 1, and connected to $L_2$ at position 2;

$L_2$ is

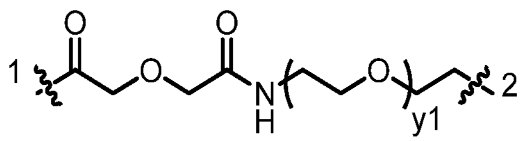

wherein yi is 0, 1, 2, 3, 4, 5, 6, 7, 8, 9 or 10; $L_2$ is connected to $L_1$ at position 1, and connected to $L_3$ at position 2;

$L_3$ is selected from a 5-12 membered aromatic heterocycle;

$L_4$ is

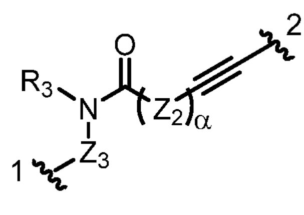

wherein $Z_2$ is selected from $C_{1-6}$ alkylene, $C_{2-10}$ alkenylene, $C_{2-10}$ alkynylene, and $C_{3-8}$ cycloalkylene; $R_3$ is selected from hydrogen (such as protium or deuterium) and $C_{1-6}$ alkyl; $Z_3$ does not exist or is selected from $C_{1-6}$ alkylene; alternatively, $R_3$ and $Z_3$ together with the nitrogen atom to which they are attached form a 4-8 membered heterocyclyl; $\alpha$ is 0, 1, 2, 3, 4, 5 or 6, and $L_4$ is connected to E at position 2, and connected to $L_3$ at position 1;

E is

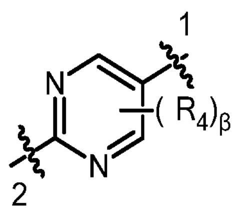

wherein each $R_4$ independently is hydrogen (e.g., protium or deuterium), $\beta$ is 0, 1, 2, 3, 4, 5 or 6, and E is connected

to A at position 2, and connected to $L_4$ at position 1;

$m_1$, $m_2$ and $m_3$ are each independently 0, 1, 2, 3, 4, 5, 6, 7, 8, 9 or 10;

A, D and $\gamma$ are as defined above.

In some embodiments, the structure of $(D-L)_\gamma$-A is as shown in formula (III):

$$\{D-[(L_1')_{m4}-L_1-(L_5)_{m5}-(L_3)_{m2}-(L_4)_{m3}-E]\}_\gamma-A \qquad \text{Formula (III)}$$

wherein

$L_1'$ is

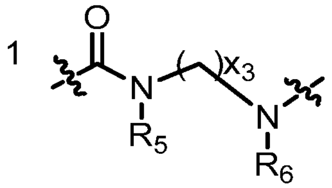

,

wherein $R_5$ and $R_6$ each independently are hydrogen (such as protium or deuterium) or $C_{1-6}$ alkyl; $x_3$ is 1, 2, 3, 4, 5 or 6; and, if $L_1'$ is present, it connects with D at position 1 and connects with $L_1$ at position 2;

$L_1$ is

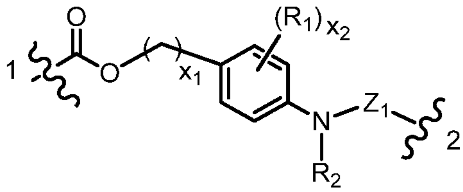

,

wherein $R_1$ and $R_2$ each independently are hydrogen (such as protium or deuterium), halogen, carboxylic acid group, sulfonic acid group, cyano, $C_{1-6}$ alkyl, halogenated $C_{1-6}$ alkyl, cyano- substituted $C_{1-6}$ alkyl (e.g., $-CH_2CN$), $C_{1-6}$ alkoxy, $C_{2-10}$ alkenyl or $C_{2-10}$ alkynyl; $Z_1$ is an amino acid or a peptide composed of 2-10 amino acids ; $x_1$ and $x_2$ each independently are 0, 1 , 2, 3, 4, 5 or 6; $L_1$ is connected to $L_1'$ (when $L_1'$ exists) at position 1, or to D (when $L_1'$ does not exist) at position 1; $L_1$ is connected to $L_5$ at position 2;

$L_5$ is

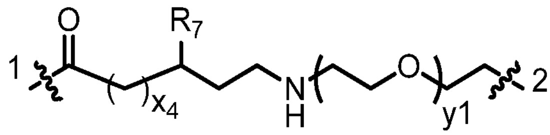

,

wherein $R_7$ is hydrogen or $C_{1-6}$ alkyl, or $R_7$ is connected to the atom N on its $\gamma$-C to form a 5-6 membered heterocyclyl; $x_4$ is 1, 2, 3, 4, 5 or 6; yi is 0, 1, 2, 3, 4, 5, 6, 7, 8, 9 or 10; and $L_5$ is connected to $L_1$ at position 1, and connected to $L_3$ at position 2;

$L_3$ is selected from a 5-12 membered aromatic heterocycle;

$L_4$ is

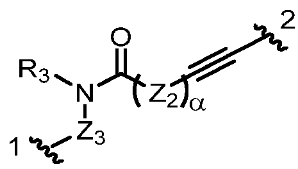

,

wherein $Z_2$ is selected from $C_{1-6}$ alkylene, $C_{2-10}$ alkenylene, $C_{2-10}$ alkynylene, and $C_{3-8}$ cycloalkylene; $R_3$ is selected from hydrogen (such as protium and deuterium) and $C_{1-6}$ alkyl; $Z_3$ does not exist or is selected from $C_{1-6}$ alkylene; or, $R_3$ and $Z_3$ together with the nitrogen atom to which they are attached form a 4-8 membered heterocyclic radical; $\alpha$ is 0, 1, 2, 3, 4, 5 or 6, and $L_4$ is connected to E at position 2, and connected to $L_3$ at position 1;

E is

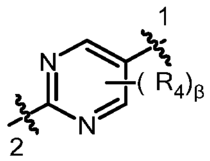

,

wherein each $R_4$ is independently hydrogen (such as protium or deuterium), $\beta$ is 0, 1, 2, 3, 4, 5, or 6, and E is connected to A at position 2, and connected to $L_4$ at position 1;

$m_1$, $m_2$, $m_3$ and $m_4$ each independently are 0, 1, 2, 3, 4, 5, 6, 7, 8, 9 or 10;

A, D and $\gamma$ are as defined above.

In some embodiments, $L_1$ in the ADC represented by formula (II) or formula (III) is

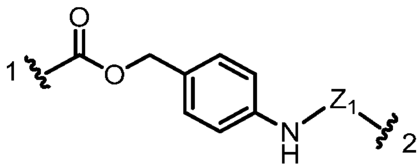

,

wherein $Z_1$ is an amino acid or a peptide composed of 2-5 amino acids, wherein the amino acid is selected from Lys, Cit, Val, D-Val, Phe, Leu, Gly, Ala and Asn; preferably, $Z_1$ is selected from Cit, Lys, Cit-Val, and Ala-Val.

In some embodiments, $L_1$ is

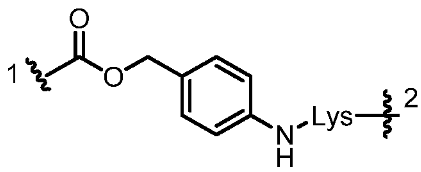

.

In some embodiments, $L_1$ is

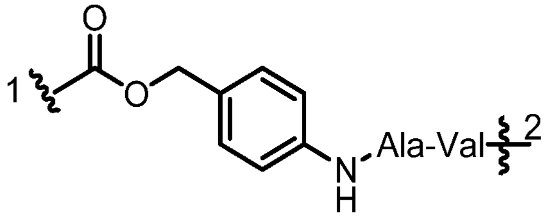

.

In some embodiments, $L_2$ in the ADC represented by formula (II) is

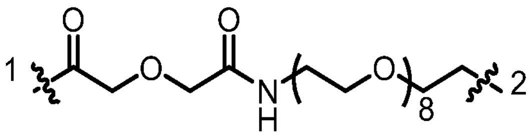

,

and $m_1$ is 1.

In some embodiments, $L_3$ in the ADC represented by formula (II) or formula (III) is a 5-6 membered aromatic heterocycle, and $m_2$ is 1.

In some embodiments, $L_3$ is triazole, and $m_2$ is 1.

In some embodiments, $L_4$ in the ADC represented by formula (II) or formula (III) is

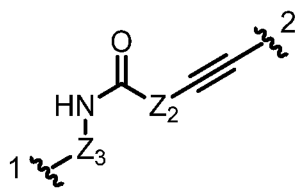

,

wherein $Z_2$ is $C_{1-6}$ alkylene, $Z_3$ is $C_{1-6}$ alkylene; and $m_3$ is 1.

In some embodiments, $L_4$ is

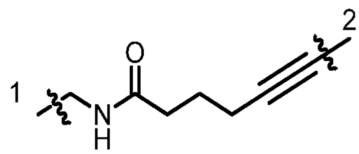

,

and $m_3$ is 1.

In some embodiments, $L_1$' in the ADC represented by formula (III) is

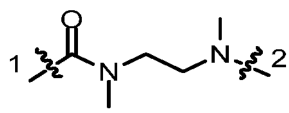

.

In some embodiments, $L_5$ in the ADC represented by formula (III) is

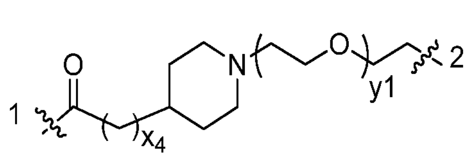

,

wherein $x_4$ is 1, 2, 3, 4, 5 or 6; $y_1$ is 1, 2, 3, 4, 5, 6, 7, 8, 9 or 10.

In some embodiments, $L_5$ in the ADC represented by formula (III) is

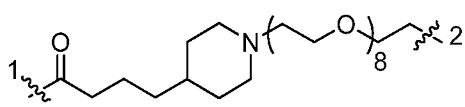

.

In some embodiments, D in the antibody-drug conjugate represented by formula (I) is

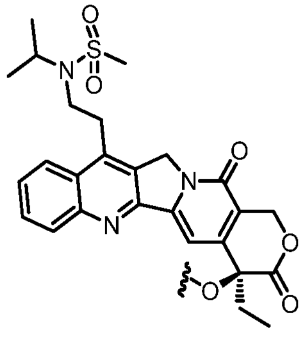,
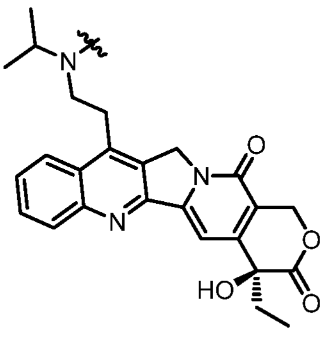 or
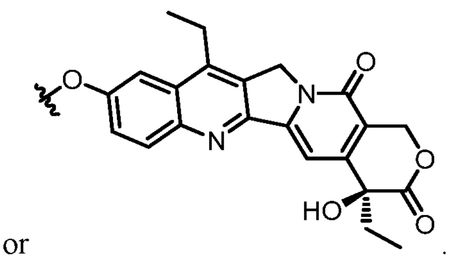.

In some embodiments, D in the antibody-drug conjugate represented by formula (II) is

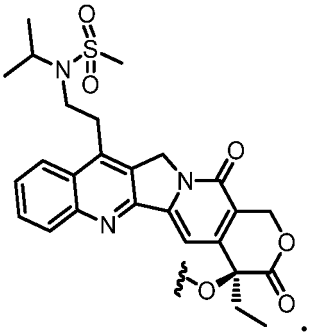.

In some embodiments, D in the ADC represented by formula (III) is

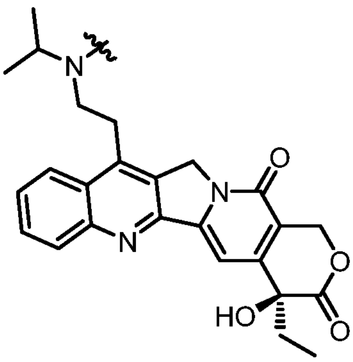 or
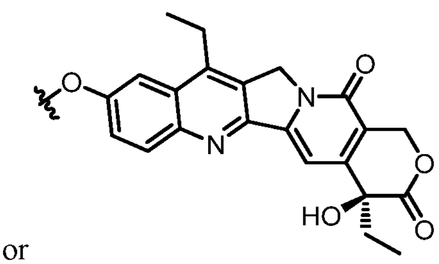.

In some embodiments, $D-[L_1-(L_2)_{m1}-(L_3)_{m2}-(L_4)_{m3}-E]-$ in the ADC represented by formula (II) is

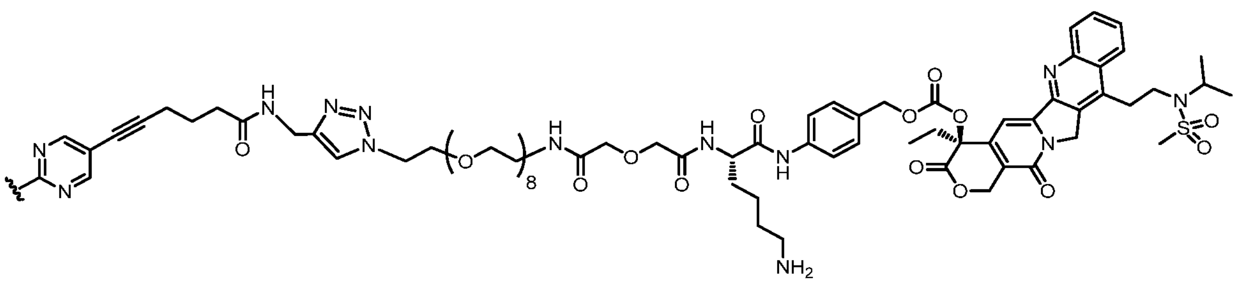.

In some embodiments, $D-[(L_1')_{m4}-L_1-(L_5)_{m5}-(L_3)_{m2}-(L_4)_{m3}-E]-$ in the ADC represented by formula (III) is

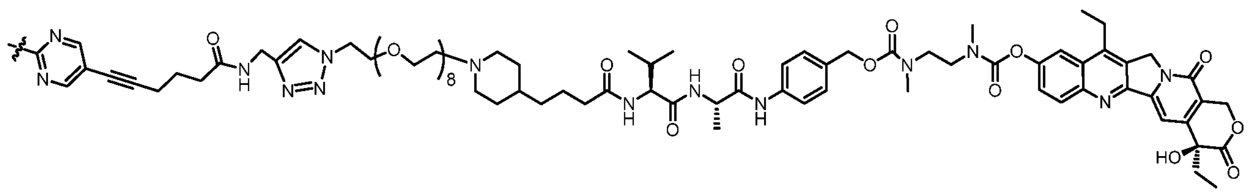

or

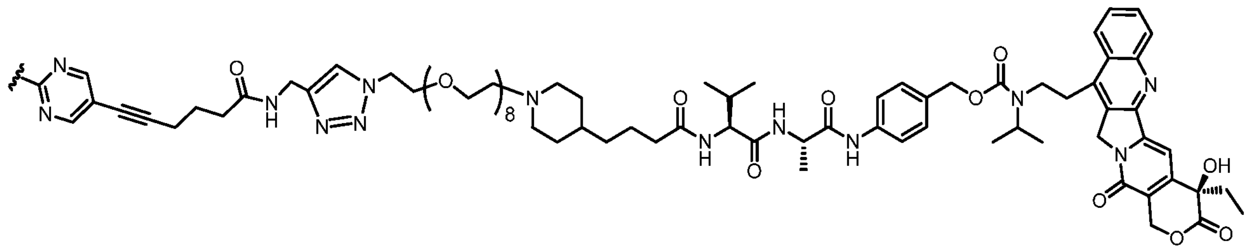

In some embodiments, the structure of the ADC represented by formula (II) is:

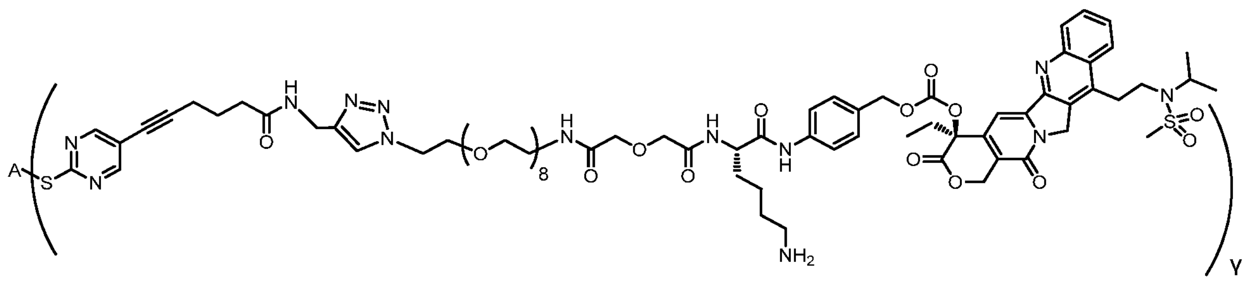

wherein γ is selected from an integer from 1 to 10, for example, γ is 1, 2, 3, 4, 5, 6, 7, 8, 9 or 10, and A is the aforementioned antibody or antigen-binding fragment thereof that specifically binds to human CLDN 18.2.

In some embodiments, the ADC represented by formula (II) is shown in the following formula:

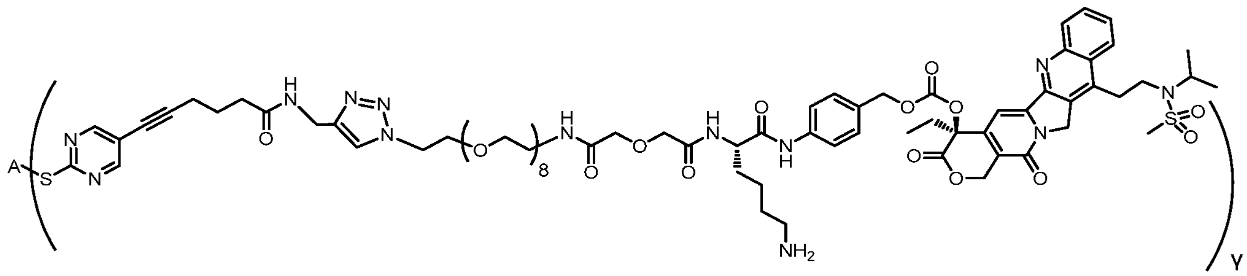

wherein γ is an integer from 1 to 10, for example, γ is 1, 2, 3, 4, 5, 6, 7, 8, 9 or 10, and A is 2C6.9-hz21.

In some embodiments, the structure of the ADC represented by formula (III) is:

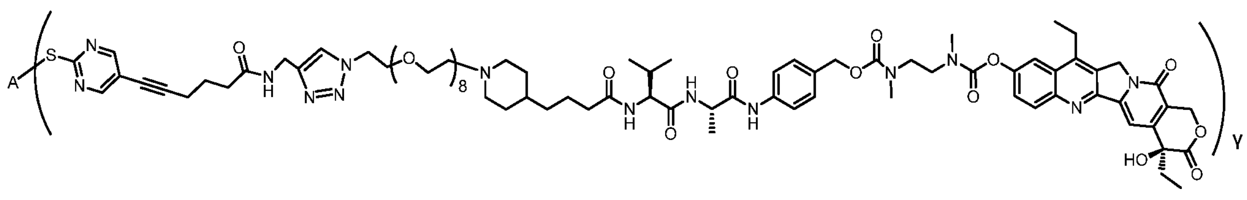

wherein γ is selected from an integer from 1 to 10, for example, γ is 1, 2, 3, 4, 5, 6, 7, 8, 9 or 10, and A is the aforementioned antibody or antigen-binding fragment thereof that specifically binds to human CLDN 18.2.

In some embodiments, the ADC represented by formula (III) is shown in the following formula:

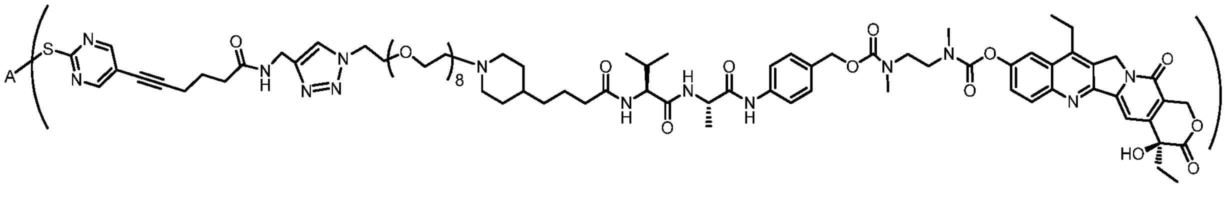

wherein γ is an integer from 1 to 10, for example, γ is 1, 2, 3, 4, 5, 6, 7, 8, 9 or 10, and A is 2C6.9-hz21.

In some embodiments, the structure of the ADC represented by formula (III) is:

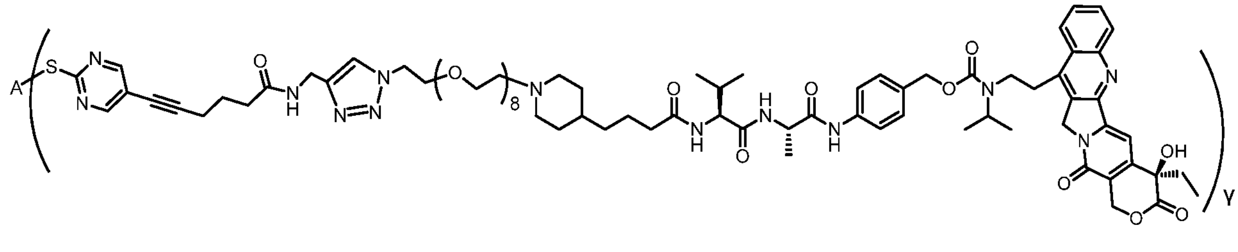

wherein γ is an integer from 1 to 10, for example, γ is 1, 2, 3, 4, 5, 6, 7, 8, 9 or 10, and A is the aforementioned antibody or antigen-binding fragment thereof that specifically binds to human CLDN 18.2.

In some embodiments, the ADC represented by formula (III) is represented by the following formula:

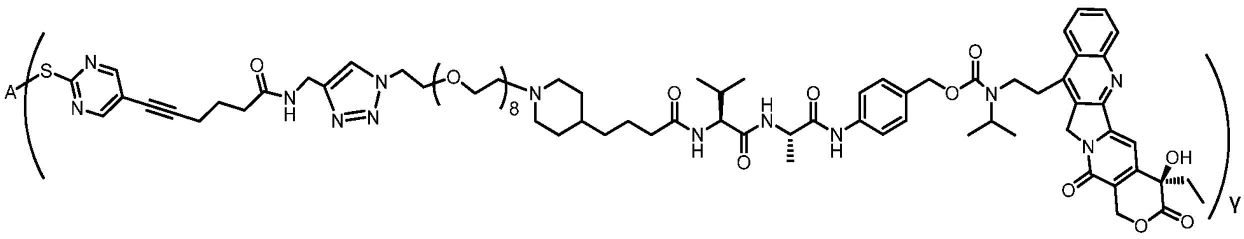

wherein γ is an integer from 1 to 10, for example, γ is 1, 2, 3, 4, 5, 6, 7, 8, 9 or 10, and A is 2C6.9-hz21.

The present invention also provides a composition comprising one or more of the aforementioned antibody-drug conjugates (ADCs), the molar ratio (DAR value) of the fragment of the bioactive molecule (i.e., D in formula (I)) to the antibody or antigen-binding fragment thereof that specifically binds to CLDN18.2 (i.e., A in formula (I)) in the composition is a decimal or integer from 1 to 10 (e.g., a decimal or integer between 1 and 8, such as 1.0, 1.5, 2.0, 2.5, 3.0, 3.1, 3.2, 3.3, 3.4, 3.5, 3.6, 3.7, 3.79, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 6.95, 7.0, 7.03, 7.1, 7.12, 7.2, 7.3, 7.40, 7.5, 7.6, 7.7, 7.8, 7.9 or 8.0).

The DAR value is also known as drug-antibody ratio, which refers to the average number of (D-L) attached to each antibody, and can be determined and calculated by the methods known in the art. For example, by the molecular weight of the conjugated antibody and (D-L) determined by LC-MS, HIC, etc., the ratio of light chains and heavy chains conjugated with different numbers of (D-L) was calculated, and the DAR value was calculated from the formula DAR (light chain DAR1*1+... +light chain $DAR_{n1}*_{n1}$)*2+(heavy chain DAR1*1+...+$DAR_{n2}*_{n2}$ )*2. In the formula, $DAR_{n1}$ means the proportion of the light chains conjugated with $n_1$ (D-L) accounted in the light chain, and $DARn_2$ means the proportion of the heavy chains conjugated $n_2$ (D-L) accounted in the heavy chain.

In some embodiments, the composition further comprises the aforementioned antibody or antigen-binding fragment thereof.

In some embodiments, the composition comprises 2C6.9-TL001. In some embodiments, the composition is 2C6.9-TL001.

In some embodiments, the composition comprises 2C6.9-TL002. In some embodiments, the composition is 2C6.9-TL002.

In some embodiments, the composition comprises 2C6.9-TL003. In some embodiments, the composition is 2C6.9-TL003.

An antibody or antigen-binding fragment thereof of the invention can be derivatized, for example, linked to another molecule (e.g., another polypeptide or protein). In general, the derivatization (e.g., labeling) of an antibody or antigen-binding fragment thereof does not adversely affect its binding to CLDN 18.2 (particularly human CLDN18.2). Thus, an antibody or antigen-binding fragment thereof of the invention is also intended to include such derivatized forms.

One type of derivatized antibody (e.g., a bispecific antibody) is produced by cross-linking two or more antibodies (of the same type or different types). Methods for obtaining bispecific antibodies are well known in the art, and examples thereof include, but are not limited to, chemical cross-linking, cell engineering (hybrid hybridoma) or genetic engineering.

Another type of derivatized antibody is a labeled antibody. For example, an antibody or antigen-binding fragment thereof herein of the invention is ligated to a detectable label. The detectable label of the present invention can be any substance detectable by fluorescence, spectroscopic, photochemical, biochemical, immunological, electrical, optical or chemical means. Such labels are well known in the art, examples of which include, but are not limited to, enzymes (e.g., horseradish peroxidase, alkaline phosphatase, beta-galactosidase, urease, glucose oxidase, etc.), radionuclides (e.g., 3H, 125I, 35S, 14C or 32P), fluorescent dyes (e.g., fluorescein isothiocyanate (FITC), fluorescein, tetramethylrhodamine isothiocyanate (TRITC), phycoerythrin (PE), Texas Red, Rhodamine, quantum dot or cyanine dye derivatives (e.g., Cy7, Alexa 750), acridinium esters, magnetic beads (e.g. , Dynabeads®), calorimetric labels such as colloids Gold or tinted glass or plastic (e.g., polystyrene, polypropylene, latex, etc.) beads, and biotin for binding to the above-described label modified avidin (e.g., streptavidin). Patents that teach the use of such markers include, but are not limited to, U.S. Patent Nos. 3,817,837; 3,850,752; 3,939,350; 3,996,345; 4,277,437; 4,275,149, and 4,366,241 (each incorporated herein by references). The detectable label as described above can be detected by methods known in the art. For example, the radioactive label can be detected using a photographic film or a scintillation counter, and the fluorescent label can be detected using a photodetector to detect the emitted light. Enzyme labels are typically detected by providing a substrate for the enzyme and detecting the reaction product produced by the action of the enzyme on the substrate, and the thermo-label is detected by simply visualizing the stained label. In certain embodiments, such markers can be adapted for use in immunological assays (e.g., enzyme-linked immunoassays, radio-immunoassays, fluorescent immunoassays, chemiluminescent immunoassays, etc.). In some embodiments, a detectable label as described above can be linked to an antibody or antigen-binding fragment thereof of the invention by a linker of varying length to reduce potential steric hindrance.

In certain embodiments, the antibody or antigen-binding fragment thereof comprised in the antibody-drug conjugate (ADC) of the present invention is one or more of the aforementioned derivatized antibodies or antigen-binding fragments thereof.

## Therapeutic methods and pharmaceutical compositions

In another aspect, the present invention also provides a pharmaceutical composition.

In some embodiments, the pharmaceutical composition comprises one or more of the aforementioned antibody-drug conjugates (ADCs) or compositions.

In some preferred embodiments, the pharmaceutical composition further comprises a pharmaceutically acceptable carrier and/or excipient.

In some preferred embodiments, the pharmaceutical composition further comprises other ingredients with anti-tumor activity. In some embodiments, the ADC or composition and other ingredients with anti-tumor activity are in the same formulation unit, or in different formulation units. Therefore, the ADC or composition of the present invention and other ingredients with anti-tumor activity can be administered simultaneously, separately or sequentially.

In some embodiments, the other ingredients with anti-tumor activity are bioactive polypeptides or active fragments thereof or chemotherapeutic drugs. In some embodiments, the bioactive polypeptide is selected from immune checkpoint inhibitors (PD-1 antibody, PD-L1 antibody, CTLA-4 antibody, LAG-3 antibody etc.), or cytokines (interferon, IL-2, IL-15, GM-CSF, IL-7, IL-12, IL-18, IL-21 etc.). In some embodiments, the chemotherapeutic drug is one or more selected from epirubicin, oxaliplatin, capecitabine, 5-fluorouracil, folinic acid, paclitaxel, and albumin-bound paclitaxel. In some embodiments, the other ingredients with anti-tumor activity are a combination of epirubicin, oxaliplatin, and 5-fluorouracil, or a combination of oxaliplatin, leucovorin, and 5-fluorouracil.

In another aspect, when being administered to a subject, the ADC or composition in the pharmaceutical composition of the present invention is sufficient to:

(a) induce the apoptosis of tumor cells (especially gastric cancer cells, such as gastric adenocarcinoma cells);

(b) inhibit the proliferation of tumor cells (especially gastric cancer cells, such as gastric adenocarcinoma cells);

(c) induce and/or increase complement dependent cytotoxicity (CDC);

(d) induce and/or increase antibody-dependent cell-mediated cytotoxicity (ADCC);

(e) inhibit the expression and activation of CLDN18.2;

(f) inhibit CLDN18.2-mediated cell signaling pathway; or

(g) any combination of (a) to (f).

In another aspect, the present invention provides use of the antibody-drug conjugate, the composition, or the pharmaceutical composition in the preparation of a medicament for the prevention and/or treatment and/or adjuvant treatment of tumors.

In some embodiments, the tumor is selected from solid tumors, hematological malignancies, and metastatic, refractory, or recurrent lesions of cancers.

In some embodiments, the tumor or cancer is selected from the group consisting of esophageal cancer, gastrointestinal cancer, gastric adenocarcinoma, pancreatic cancer, thyroid cancer, colorectal cancer, kidney cancer, lung cancer (e.g., non-small cell lung cancer, NSCLC), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell tumor, bone cancer, skin cancer, thymus cancer, cholangiocarcinoma, gallbladder cancer, melanoma, mesothelioma, lymphoma, myeloma (e.g., multiple myeloma), sarcoma, glioblastoma, and leukemia.

In some embodiments, the tumor is selected from gastric cancer, gastric adenocarcinoma, gastroesophageal junction (GEJ) adenocarcinoma, esophageal cancer, gastrointestinal cancer, pancreatic cancer, and lung cancer (e.g., NSCLC).

In some embodiments, the tumor is gastric cancer, gastric adenocarcinoma, or gastroesophageal junction (GEJ) adenocarcinoma, such as a locally advanced unresectable or metastatic tumor of gastric cancer, gastric adenocarcinoma or gastroesophageal junction (GEJ) adenocarcinoma.

In some embodiments, the tumor is CLDN18.2 positive, and further, the tumor is HER2 negative.

In some embodiments, the tumor is HER2 negative.

In another aspect, the present invention provides a method of preventing and/or treating a tumor in a subject. In another aspect, the present invention provides a method of delaying tumor progression in a subject. In another aspect, the present invention provides a method of reducing or inhibiting tumor recurrence in a subject. The method comprises a step of administrating to a subject in need thereof an effective amount of the antibody-drug conjugate, composition or the pharmaceutical composition of the present invention.

In some embodiments, the method described above also comprises a step of applying a second therapy to the subject, wherein the second therapy is selected from the group consisting of surgery, chemotherapy, radiation therapy, immunotherapy, gene therapy, DNA therapy, RNA therapy, nanotherapy, viral therapy, adjuvant therapy, and any combination thereof.

In some embodiments, the second therapy can be applied separately or in combination with the method described above; or, the second therapy can be applied separately, in combination, simultaneously or sequentially with the method described above.

In some embodiments, the second therapy is chemotherapy. In some embodiments, the chemotherapeutic drug is selected from one or more of epirubicin, oxaliplatin, capecitabine, 5-fluorouracil, folinic acid, paclitaxel, and albumin-bound paclitaxel. In some embodiments, the chemotherapeutic drug is a combination of epirubicin, oxaliplatin, and 5-fluorouracil, or a combination of oxaliplatin, leucovorin, and 5-fluorouracil. In some embodiments, the combined dosage regimen of oxaliplatin, leucovorin, and 5-fluorouracil is selected from FOLFOX4, FOLFOX6, or mFOLFOX6.

In some embodiments, the second therapy is immunotherapy. In some embodiments, the drug for immunotherapy is selected from immune checkpoint inhibitors (e.g., PD-1 antibody, PD-L1 antibody, CTLA-4 antibody, and LAG-3 antibody), or cytokines (e.g., interference Vegetarian, IL-2, IL-15, GM-CSF, IL-7, IL-12, IL-18, and IL-21).

In some embodiments, the tumor is selected from solid tumors, hematological malignancies, and the metastatic, refractory, or recurring lesions of cancers.

In some embodiments, the tumor or cancer is selected from the group consisting of esophageal cancer, gastrointestinal cancer, gastric adenocarcinoma, pancreatic cancer, thyroid cancer, colorectal cancer, kidney cancer, lung cancer (e.g., NSCLC), liver cancer, gastric cancer, gastroesophageal junction (GEJ) adenocarcinoma, head and neck cancer, bladder cancer, breast cancer, uterine cancer, cervical cancer, ovarian cancer, prostate cancer, testicular cancer, germ cell tumor, bone cancer, skin cancer, thymus cancer, cholangiocarcinoma, gallbladder cancer, melanoma, mesothelioma, lymphoma, myeloma (e.g., multiple myeloma), sarcoma, glioblastoma, and leukemia.

In some embodiments, the tumor is selected from gastric cancer, gastric adenocarcinoma, gastroesophageal junction (GEJ) adenocarcinoma, esophageal cancer, gastrointestinal cancer, pancreatic cancer, and lung cancer (e.g., NSCLC).

In some embodiments, the tumor is gastric cancer, gastric adenocarcinoma, or gastroesophageal junction (GEJ) adenocarcinoma, such as a locally advanced unresectable or metastatic tumor of gastric cancer, gastric adenocarcinoma, or gastroesophageal junction (GEJ) adenocarcinoma.

In some embodiments, the tumor is CLDN18.2 positive, and further, the tumor is HER2 negative.

In some embodiments, the tumor is HER2 negative.

The ADC, composition or pharmaceutical composition of the present invention can be formulated into any pharmaceutical formulations known in the medical field, such as tablets, pills, suspensions, emulsions, solutions, gels, capsules, powders, granules, elixirs, lozenges, suppositories, injections (including injection, sterile powder for injection and concentrated solution for injection), inhalant, spray, or the like. Preferred dosage form depends on the intended mode of administration and therapeutic use. The pharmaceutical composition of the invention should be sterile and stable under manufacture and storage condition. A preferred pharmaceutical formulation is injection. The injection may be sterile injection solution. For example, the following methods could be used to prepare sterile injection solution: a necessary dosage of the ADC or composition is dispersed in a proper carrier, optionally with other desired ingredients (including but not limited to pH adjusting agent, surfactant, adjuvant, ionic strength enhancer, isotonicity agent, preservative, diluent, or any combination thereof), followed by filtration sterilization. In addition, sterile injection solution can be used to prepare sterile lyophilized powder (such as by vacuum drying or lyophilizing) to facilitate storage and usage. The sterile lyophilized powder can be dispersed in a suitable carrier before use, such as sterile pyrogen-free water.

Furthermore, the ADC of the present invention may be present in a pharmaceutical composition in the form of a unit dose to facilitate administration.

The ADC, composition or pharmaceutical composition of the invention may be administered by any suitable method known in the art, including but not limited to, oral, buccal, sublingual, ocular, topical, parenteral, rectal, intrathecal, intra-cisterna, in the groin, intravesical, local (such as powder, ointment or drops), or nasal route. However, for many therapeutic uses, the preferred route/mode of administration is parenteral (such as intravenous, subcutaneous, intraperitoneal, intramuscular). The skill person will appreciate that the route and/or manner of administration will vary depending on the intended purpose. In a preferred embodiment, the antibody or antigen-binding fragment thereof, pharmaceutical composition of the invention is administered by intravenous infusion or injection.

The pharmaceutical compositions of the invention may comprise a "therapeutically effective dose" or a "prophylactically effective dose" of the ADC or composition of the invention. "Prophylactically effective dose" means an amount sufficient to prevent, inhibit, or delay the onset of a disease. "Therapeutically effective amount" means an amount sufficient to cure or at least partially inhibit the disease and its complications in a patient already suffering from the disease. The therapeutically effective dose of the ADC or composition of the present invention may vary depending on factors such as the severity of the disease to be treated, the overall state of the patient's own immune system, the general condition of the patient such as age, weight and sex, and administration of drug, as well as other treatments for simultaneous administration, and the like.

In the present invention, the dosage regimen can be adjusted to achieve an optimal intended response (such as therapeutic or prophylactic response). For example, it may be administered in a single administration, may be administered multiple times over a period of time, or may be administrated in proportionally reduced or increased dosage according to the urgency of the treatment.

A typical non-limiting therapeutically or prophylactically effective dose range of the ADC or composition of the invention is from 0.02 to 100 mg/kg, such as from 0.1 to 100 mg/kg, from 0.1 to 50 mg/kg, or from 1 to 50 mg/kg. It should be noted that the dosage may vary depending on the type and severity of the condition to be treated. Moreover, it will be understood by those skilled in the art that for any particular patient, the particular dosage regimen should be adjusted over time according to the needs of the patient and the professional evaluation by the physician; the dosage ranges given herein are for illustrative purposes only and are not for limiting the use or range of the pharmaceutical compositions of the invention.

In the present invention, the subject may be a mammal, such as a human.

## ABBREVIATION

| | |
|---|---|
| CDR | Complementarity-determine region of immunoglobulins |
| FR | Framework region of antibody: including amino acid residues in antibody variable region other than CDR |
| VH | Variable region of antibody heavy chain |
| VL | Variable region of antibody light chain |
| IgG | Immunoglobulin G |

| | |
|---|---|
| AbM | The definition of AbM CDR comes from Martin's related research (Martin ACR, Cheetham JC, Rees AR (1989) Modelling antibody hypervariable loops: A combined algorithm. Proc Natl Acad Sci USA 86:9268-9272), this definition method integrates the partial definitions of Kabat and Chothia. |
| Kabat | An Immunoglobulin reference and numbering system proposed by Elvin A. Kabat (see, for example, Kabat et al., Sequences of Proteins of Immunological Interest, 5th Ed. Public Health Service, National Institutes of Health, Bethesda, Md., 1991) |
| Chothia | An Immunoglobulin numbering system proposed by Chothia et al., which is a classic rule for identifying the boundaries of CDR regions based on the location of the structural loop region. (see, for example, Chothia & Lesk (1987) J. Mol. Biol. 196:901-917; Chothia et al. (1989) Nature 342:878-883) |
| IMGT | A numbering system based on the international ImMunoGeneTics information system ® (IMGT) initiated by Lefranc et al., See, Lefranc et al., Dev. Comparat. Immunol. 27:55-77, 2003 |
| mAb | Monoclonal antibody |
| EC50 | A concentration at which 50% efficacy or binding is produced |
| HRP | Horseradish peroxidase |
| CDR-H1 | Complementarity-determining region 1 of heavy chain variable region |
| CDR-H2 | Complementarity-determining region 2 of heavy chain variable region |
| CDR-H3 | Complementarity-determining region 3 of heavy chain variable region |
| CDR-L1 | Complementarity-determining region 1 of light chain variable region |
| CDR-L2 | Complementarity-determining region 2 of light chain variable region |
| CDR-L3 | Complementarity-determining region 3 of light chain variable region |

In the present invention, unless otherwise specified, scientific and technical terms used herein have the meanings commonly understood by those skilled in the art. Moreover, laboratory procedures of cell culture, biochemistry, nucleic acid chemistry, immunology used herein are all routine steps widely used in the corresponding fields. At the same time, in order to better understand the present invention, definitions and explanations of related terms are provided below.

As used herein, the terms "ADC (antibody-drug conjugate)" or "conjugate" are interchangeable and refer to a substance obtained by conjugating a bioactive molecule to an antibody through a linker.

The linker can be connected to the antibody through various chemical bonds. For example, in some embodiments, the linker is connected by forming a thioether bond with the sulfhydryl group of the antibody. In the structural formula of some specific ADC molecules (such as 2C6.9-TL001, 2C6.9-TL002, or 2C6.9-TL003 ADC molecules), -S- merely represents the thioether bond formed by the linker and the sulfhydryl group of the antibody, and does not mean that -Sis part of the linker.

The structural formula of the ADC in the present application can be represented by $(D-L)_{\gamma}$-A, wherein D is a bioactive molecular fragment; L is a linker; A is an antibody or antigen-binding fragment thereof that specifically binds to human CLDN 18.2; γ, which refers to the number of (D-L) attached to each antibody molecule, is selected from an integer from 1 to 10, such as 1, 2, 3, 4, 5, 6, 7, 8, 9 or 10. However, in the course of ADC preparation, each antibody molecule may be connected with different numbers of (D-L). Therefore, the ADC product is generally a composition of antibodies coupled with different numbers of (D-L). In practice, DAR is often used to represent the average value of the number of (D-L) linked to the antibody.

As used herein, the term "antibody" refers to an immunoglobulin molecule usually composed of two pairs of polypeptide chains (each pair has a light chain (LC) and a heavy chain (HC)). Antibody light chains can be classified into κ (kappa) and λ (lambda) light chains. Heavy chains can be classified into μ, δ, γ, α or ε heavy chains, and the isotypes of antibody are therefore defined as IgM, IgD, IgG, IgA and IgE, respectively. Within the light and heavy chains, the variable and constant regions are connected by a "J" region of about 12 or more amino acids, and the heavy chain also includes a "D" region of about 3 or more amino acids. Each heavy chain is composed of a heavy chain variable

region (VH) and a heavy chain constant region (CH). The heavy chain constant region is composed of 3 domains (CH1, CH2 and CH3). Each light chain is composed of a light chain variable region (VL) and a light chain constant region (CL). The light chain constant region consists of one domain CL. The constant domains are not directly involved in the binding of antibodies and antigens, but exhibit a variety of effector functions, such as mediating the binding of immunoglobulins to host tissues or factors, including various cells (for example, effector cells) of immune system and the first component (C1q) of classical complement system. The VH and VL regions can also be subdivided into hypervariable regions (called complementarity determining regions (CDR)), interspersed with more conservative regions (called framework regions (FR)). Each VH and VL consists of 3 CDRs and 4 FRs arranged in the following order: FR1, CDR1, FR2, CDR2, FR3, CDR3, FR4 from the amino terminus to the carboxy terminus. The variable regions (VH and VL) of each heavy chain/light chain pair form antigen binding sites respectively. The assignment of amino acids in each region or domain may follow the definition of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883, or AbM, Martin's related research (Martin ACR, Cheetham JC, Rees AR (1989) Modelling antibody hypervariable loops: A combined algorithm. Proc Natl Acad Sci USA 86:9268-9272).

In this context, unless the context clearly dictates otherwise, when the term "antibody" is referred to, it includes not only intact antibody but also antigen-binding fragments of antibody.

As used herein, the term "complementarity determining region" or "CDR" refers to the amino acid residues in antibody variable region that are responsible for antigen binding. The precise boundaries of these amino acid residues can be defined according to various numbering systems known in the art, for example, according to the Kabat numbering system (Kabat et al., Sequences of Proteins of Immunological Interest, 5th Ed. Public Health Service, National Institutes of Health, Bethesda, Md., 1991), Chothia numbering system (Chothia & Lesk (1987) J. Mol. Biol. 196:901-917; Chothia et al. (1989) Nature 342:878-883), IMGT numbering system ( Lefranc et al., Dev. Comparat. Immunol. 27:55-77, 2003), or the definition of Martin's related research (Martin ACR, Cheetham JC, Rees AR (1989) Modelling antibody hypervariable loops: A combined algorithm. Proc Natl Acad Sci USA 86:9268-9272), the definition method of which integrates the parts of definitions of Kabat and Chothia, and was first applied in Oxford Molecular antibody modeling software (Martin AC R. Protein sequence and structure analysis of antibody variable domains[M] //Antibody engineering. Springer, Berlin, Heidelberg, 2010: 33-51.). For a given antibody, those skilled in the art will easily identify the CDRs defined by each numbering system. Moreover, the correspondence between different numbering systems is well known to those skilled in the art (for example, see Lefranc et al., Dev. Comparat. Immunol. 27:55-77, 2003).

The CDR contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia, IMGT or AbM numbering system.

As used herein, the term "framework region" or "FR" residue refers to the amino acid residues in the variable region of the antibody other than the CDR residues as defined above.

As used herein, the term "antigen-binding fragment" of antibody refers to a polypeptide of antibody fragment, such as a polypeptide of fragment of full-length antibody, which retains the ability to specifically bind to the same antigen to which the full-length antibody binds, and/or compete with the full-length antibody for specific binding to the antigen, which is also called "antigen-binding portion". See generally, Fundamental Immunology, Ch. 7 (Paul, W., ed., 2nd edition, Raven Press, NY (1989), which is incorporated herein by reference in its entirety for all purposes. Recombinant DNA technology or enzymatic or chemical cleavage of an intact antibody can be used to produce an antigen-binding fragment of the antibody. Non-limiting examples of antigen-binding fragment include Fab, Fab', $F(ab')_2$, Fd, Fv, dAb, and complementarity determining region (CDR) fragment, single chain antibody (e.g., scFv), chimeric antibody, diabody, linear antibody, nanobody (technology of which is from Domantis), domain antibody (technology of which is from Ablynx), and such polypeptides, which contain at least a portion of antibody that is sufficient to confer the polypeptide a specific antigen binding ability. Engineered antibody variants are reviewed by Holliger et al., 2005; Nat Biotechnol, 23:1126-1136.

As used herein, the term "full-length antibody" refers to an antibody composed of two "full-length heavy chains" or "heavy chains" and two "full-length light chains" or "light chains", wherein the "full-length heavy chain" or "heavy chain" refers to a polypeptide chain that consists of a heavy chain variable region (VH), a heavy chain constant region CH1 domain, a hinge region (HR), a heavy chain constant region CH2 domain, and a heavy chain constant region CH3 domain in the N-terminal to C-terminal direction; and, when the full-length antibody is of the IgE isotype, it optionally also comprises a heavy chain constant region CH4 domain. Preferably, the "full-length heavy chain" is a polypeptide chain composed of VH, CH1, HR, CH2 and CH3 in the N-terminal to C-terminal direction. The "full-length light chain" or "light chain" is a polypeptide chain composed of a light chain variable region (VL) and a light chain constant region (CL) in the N-terminal to C-terminal direction. The two pairs of full-length antibody chains are connected by a disulfide bond between the CL and CH1 and a disulfide bond between the HR of the two full-length heavy chains. The full-length antibody of the present invention can be from a single species, such as a human; it can also be a chimeric antibody or a humanized antibody. The full-length antibody of the present invention comprises two antigen binding sites formed by

a pair of VH and VL respectively, and the two antigen binding sites specifically recognize/bind the same antigen.

As used herein, the term "Fd fragment" refers to an antibody fragment composed of VH and CH1 domains; the term "dAb fragment" refers to an antibody fragment composed of VH domains (Ward et al., Nature 341:544 546 (1989)); the term "Fab fragment" refers to an antibody fragment composed of VL, VH, CL and CH1 domains; the term "$F(ab')_2$ fragment" refers to an antibody fragment comprising two Fab fragments connected by a disulfide bridge of the hinge region; the term "Fab' fragment" refers to a fragment obtained by reducing the disulfide bond connecting the two heavy chain fragments in $F(ab')_2$ fragment, consisting of an intact light chain and a heavy chain Fd fragment (consisting of VH and CH1 domains).

As used herein, the term "Fv fragment" refers to an antibody fragment composed of the VL and VH domains of a single arm of antibody. Fv fragment is generally considered to be the smallest antibody fragment that can form a complete antigen binding site. It is generally believed that six CDRs can confer antigen binding specificity to antibody. However, even a variable region (e.g., a Fd fragment, which contains only three antigen-specific CDRs) can recognize and bind an antigen, although its affinity may be lower than that of the complete binding site.

As used herein, the term "Fc fragment" refers to an antibody fragment formed with the second and third constant regions of first heavy chain and the second and third constant regions of second heavy chain of an antibody that are bound through a disulfide bond. The Fc fragment of antibody has many different functions, but does not participate in antigen binding.

As used herein, the term "scFv" refers to a single polypeptide chain comprising VL and VH domains, wherein the VL and VH are connected by a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Volume 113, edited by Roseburg and Moore, Springer-Verlag, New York, pp. 269-315 (1994)). Such scFv molecule may have a general structure: $NH_2$-VL-linker-VH-COOH or $NH_2$-VH-linker-VL-COOH. A suitable linker of prior art consists of a repeated GGGGS amino acid sequence or variants thereof. For example, a linker having amino acid sequence $(GGGGS)_4$ can be used, but variants thereof can also be used (Holliger et al. (1993), Proc. Natl. Acad. Sci. USA 90: 6444-6448). Other linkers that can be used in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol. In some cases, there may also be a disulfide bond between the VH and VL of scFv. As used herein, the term "di-scFv" refers to an antibody fragment formed by linking two scFvs.

As used herein, the term "diabody" refers to that its VH and VL domains are expressed on a single polypeptide chain, but the linker used is too short to allow the pairing between the two domains of the same chain, so that the domains are forced to pair with the complementary domains of another chain and create two antigen binding sites (see, for example, Holliger P. et al., Proc. Natl. Acad. Sci. USA 90: 6444-6448 (1993), and Poljak RJ et al., Structure 2:1121-1123 (1994)).

Each of the aforementioned antibody fragments maintains the ability to specifically bind to the same antigen to which the full-length antibody binds, and/or compete with the full-length antibody for specific binding to the antigen.

As used herein, "bispecific antibody" refers to a conjugate formed by a first antibody (fragment) and a second antibody (fragment) or antibody mimetics through a coupling arm. The coupling mode includes but is not limited to chemical reaction, gene fusion, protein fusion, polypeptide fusion and enzymatic reaction. "Multispecific antibody" comprises, for example, trispecific antibody and tetraspecific antibody, in which the former is an antibody with three different antigen binding specificities, and the latter is an antibody with four different antigen binding specificities.

As used herein, "antibody mimetics" refers to substances that specifically bind to an antigen as an antibody does, but do not have the structure of antibody. They are usually artificial peptides or proteins with a molar mass of about 3 to 20 kDa, such as ankyrin repeat protein (DARPin) and fynomer. The designed ankyrin repeat protein (DARPin) is linked to IgG antibody, scFv-Fc antibody fragment or a combination thereof, as in CN104341529A. The anti-IL-17a fynomer binds to anti-IL-6R antibody, as in WO2015141862A1.

In this context, the antigen-binding fragment (e.g., the above-mentioned antibody fragment) of antibody can be obtained from a given antibody (e.g., the antibody provided by the present invention) using a conventional technique known to those skilled in the art (e.g., recombinant DNA technology or enzymatic or chemical fragmentation methods), and can be screened for specificity in the same manner by which intact antibodies are screened.

As used herein, the terms "monoclonal antibody" and "mAb" have the same meaning and are used interchangeably, which refer to an antibody or a fragment of an antibody derived from a group of highly homologous antibody molecules, that is, a group of identical antibody molecules except for natural mutations that may occur spontaneously. The monoclonal antibody has high specificity for a single epitope on antigen. Polyclonal antibodies are mentioned relative to the monoclonal antibody, and usually comprise at least two or more different antibodies, and these different antibodies usually recognize different epitopes on the antigen. In addition, the modifier "monoclonal" merely indicates the character of the antibody as being obtained from a highly homogeneous population of antibodies, and is not to be construed as requiring production of the antibody by any particular method.

The monoclonal antibody of the present invention can be prepared by a variety of techniques, such as hybridoma technology (see, for example, Kohler et al. Nature, 256:495, 1975), recombinant DNA technology (see, for example, US Patent Application 4,816,567), or phage antibody library technology (see, for example, Clackson et al. Nature352:624-628, 1991, or Marks et al. J. Mol. Biol. 222:581-597, 1991).

For example, monoclonal antibodies can be prepared as follows. The mice or other suitable host animals are first immunized with immunogen (with addition of an adjuvant if necessary). The method of injection of immunogen or adjuvant is usually multi-point subcutaneous injection or intraperitoneal injection. Immunogen can be pre-coupled to certain known proteins, such as serum albumin or soybean trypsin inhibitors, to enhance the immunogenicity of the antigen in the host. The adjuvant may be Freund's adjuvant or MPL-TDM or the like. After the animal is immunized, the body will produce lymphocytes that secrete antibodies that specifically bind to the immunogen. In addition, lymphocytes can also be obtained by in vitro immunization. The lymphocytes of interest are collected and fused with myeloma cells using a suitable fusing agent, such as PEG, to obtain hybridoma cells (Goding, Monoclonal Antibodies: Principles and Practice, pp. 59-103, Academic Press, 1996). The hybridoma cells prepared above may be inoculated into a suitable culture medium which preferably contains one or more substances capable of inhibiting the growth of unfused, parental myeloma cells. For example, for parental myeloma cells lacking hypoxanthine guanine phosphotransferase (HGPRT or HPRT), the addition of hypoxanthine, aminopterin, and thymine (HAT medium) to the culture medium will inhibit growth of HGPRT- defective cells. Preferred myeloma cells should have a high fusion rate, stable antibody secretion capacity, and sensitivity to HAT culture medium. Among them, murine myeloma is preferred for myeloma cells, such as MOP-21 or MC-11 mouse tumor-derived strains (THE Salk Institute Cell Distribution Center, San Diego, Calif. USA), and SP-2/0 or X63-Ag8-653 cell line (American Type Culture Collection, Rockville, Md. USA). In addition, studies have also reported the use of human myeloma and human-murine heterologous myeloma cell lines to prepare human monoclonal antibodies (Kozbor, J. Immunol., 133: 3001 (1984); Brodeur et al., Monoclonal Antibody Production Techniques and Applications, pp 51-63, Marcel Dekker, Inc., New York, 1987). The culture medium for growing hybridoma cells is used to detect the production of monoclonal antibodies against specific antigens. Methods for determining the binding specificity of monoclonal antibodies produced by hybridoma cells include, for example, immunoprecipitation or in vitro binding assays such as radioimmunoassay (RIA), enzyme-linked immunosorbent assay (ELISA). For example, the affinity of the monoclonal antibody can be determined using the Scatchard assay described by Munson et al., Anal. Biochem. 107: 220 (1980). After determining the specificity, affinity, and reactivity of the antibody produced by the hybridoma, the cell strain of interest can be subcloned by the standard limiting dilution method described in Goding, Monoclonal Antibodies: Principles and Practice, pp. 59-103, Academic Press, 1996. A suitable culture medium may be DMEM or RPMI-1640 or the like. In addition, hybridoma cells can also be grown in animals in the form of ascites tumors. Utilizing traditional immunoglobulin purification methods, such as protein A agarose gel, hydroxyapatite chromatography, gel electrophoresis, dialysis or affinity chromatography, the monoclonal antibodies secreted by the subcloned cells can be isolated from the cell culture medium, ascites or serum.

Monoclonal antibodies can also be obtained by genetic engineering recombinant techniques. A DNA molecule encoding the heavy chain and light chain genes of a monoclonal antibody can be isolated from a hybridoma cell by PCR amplification using a nucleic acid primer that specifically binds to the monoclonal antibody heavy chain and light chain genes. The obtained DNA molecule is inserted into an expression vector, and then transfected into a host cell (such as E. coli cells, COS cells, CHO cells, or other myeloma cells that do not produce immunoglobulin), and cultured under appropriate conditions, thereby obtaining the desired recombinant antibody.

The antibody can be purified by well-known techniques, for example, affinity chromatography using Protein A or Protein G. Subsequently or alternatively, the specific antigen (the target molecule recognized by the antibody) or its antigenic epitope can be immobilized on a column and the immunospecific antibody can be purified by immunoaffinity chromatography. Purification of immunoglobulins can be found, for example, in D. Wilkinson (The Scientist, published by The Scientist, Inc., Philadelphia Pa., Vol. 14, No. 8 (Apr. 17, 2000), pp. 25-28).

As used herein, the term "murine antibody" refers to an antibody that is prepared by fusing B cells of immunized mice with myeloma cells, selecting murine hybrid fusion cells that can proliferate indefinitely and secrete the antibody, followed by screening, antibody preparation and antibody purification; or an antibody that is secreted by plasma cells which is formed by differentiation and proliferation of B cells after antigen invades the mouse body. For the antibody produced under the stimulation of specific antigen, the production of antibody is due to the interaction of various immune cells caused by the antigen invading the human body, resulting in differentiation and proliferation of B cells into plasma cells, which can produce and secrete the antibody.

As used herein, the term "chimeric antibody" refers to an antibody in which a portion of the light or/and heavy chain thereof is derived from one antibody (which may be derived from a specific species or belong to a certain specific antibody class or subclass), and another portion of the light chain or/and heavy chain is derived from another antibody (which may be derived from the same or different species or belong to the same or different antibody class or subclass), nonetheless, it still retains binding activity to the antigen of interest (USP 4,816,567 to Cabilly et al.; Morrison et al., Proc. Natl. Acad. Sci. USA, 81:6851 6855 (1984)). For example, the term "chimeric antibody" can include an antibody (e.g.,

a human-murine chimeric antibody), wherein the heavy and light chain variable regions of the antibody are derived from a first antibody (e.g., a murine antibody), while the heavy chain and light chain variable regions of the antibody are derived from a second antibody (e.g., a human antibody).

As used herein, the term "humanized antibody" refers to a genetically engineered non-human antibody whose amino acid sequence has been modified to increase homology to the sequence of a human antibody. Generally, all or part of the CDR regions of a humanized antibody are derived from a non-human antibody (donor antibody), and all or part of the non-CDR regions (e.g., variable region FRs and/or constant region) are derived from a human immunoglobulin (receptor antibody). Humanized antibodies typically retain the desired properties of the donor antibody, including, but not limited to, antigen specificity, affinity, reactivity, ability to increase immune cell activity, ability to enhance an immune response, and the like. A donor antibody can be an antibody from mouse, rat, rabbit, or non-human primate (e.g., cynomolgus) having desirable properties (e.g., antigen specificity, affinity, reactivity, ability to increase immune cell activity and/or enhance immune response).

Humanized antibody can not only retain the expected properties of non-human donor antibody (e.g., murine antibody), but also effectively reduce the immunogenicity of non-human donor antibody (e.g., murine antibody) in a human subject, and thus is particularly advantageous. However, due to the matching problem between the CDR of donor antibody and the FR of receptor antibody, the expected properties of humanized antibody (e.g., antigen specificity, affinity, reactivity, ability to improve immune cell activity, and/or ability to enhance immune response) are generally lower than that of the non-human donor antibody (e.g., murine antibody).

Therefore, although researchers in the field have carried out in-depth research on the humanization of antibodies and have made some progresses (see, for example, Jones et al., Nature, 321:522 525 (1986); Reichmann et al., Nature, 332: 323 329 (1988); Presta, Curr. Op. Struct. Biol., 2:593 596 (1992); and Clark, Immunol. Today 21: 397 402 (2000)), the prior art does not provide detailed guidance on how to fully humanize donor antibody so that the humanized antibody produced not only has the highest degree of humanization, but also retains the expected properties of the donor antibody as much as possible. Technicians need to perform exploration, investigation and modification for a specific donor antibody, and pay a lot of creative work to obtain a humanized antibody that not only has a high degree of humanization (for example, a humanization degree of at least 75%, at least 80%, at least 85%, at least 90%, at least 91%, at least 92%, at least 93%, at least 94%, at least 95%, at least 96%, at least 97%, at least 98%, at least 99%), but also retains the expected properties of the specific donor antibody.

In the present invention, in order to make the humanized antibody retain the properties (including, for example, antigen specificity, affinity, reactivity, ability to improve immune cell activity and/or ability to enhance immune response) of the donor antibody as much as possible, the framework region (FR) of the humanized antibody of the present invention may comprise both the amino acid residues of human receptor antibody and the amino acid residues of corresponding non-human donor antibody.

The humanized antibody of the present invention can be prepared based on the sequence of the murine monoclonal antibody prepared above. The DNA encoding the heavy and light chains can be obtained from the target murine hybridoma and engineered using standard molecular biology techniques to contain non-mouse (e.g., human) immunoglobulin sequences.

To prepare a chimeric antibody, a variable region of murine immunoglobulin can be linked to a constant region of human immunoglobulin using a method known in the art (see, for example, US Patent No. 4,816,567 to Cabilly et al.). For example, a DNA encoding VH is operably linked to another DNA molecule encoding heavy chain constant region so as to obtain a full-length heavy chain gene. The sequence of human heavy chain constant region gene is known in the art (see, for example, Kabat, EA et al. (1991) Sequences of Proteins of Immunological Interest, Fifth Edition, U.S. Department of Health and Human Services, NIH Publication No. 91-3242), a DNA fragment containing these regions can be obtained by standard PCR amplification. The heavy chain constant region may be an IgG1, IgG2, IgG3, IgG4, IgA, IgE, IgM or IgD constant region, but is generally preferably an IgG1 or IgG4 constant region. For example, a DNA encoding VL is operably linked to another DNA molecule encoding light chain constant region CL so as to obtain a full-length light chain gene (as well as a Fab light chain gene). The sequence of human light chain constant region gene is known in the art (see, for example, Kabat, EA et al. (1991) Sequences of Proteins of Immunological Interest, Fifth Edition, US Department of Health and Human Services, NIH Publication No. 91-3242), a DNA fragment containing these regions can be obtained by standard PCR amplification. The light chain constant region may be a κ or λ constant region, but is generally preferably a κ constant region.

To prepare a humanized antibody, murine CDR regions can be grafted onto a human framework sequence by using any methods known in the art (see US Patent No. 5,225,539 to Winter; US Patent Nos. 5,530,101, 5,585,089, 5,693,762, and 6,180,370 to Queen et al.; And Lo, Benny, KC, editor, in Antibody Engineering: Methods and Protocols, volume 248, Humana Press, New Jersey, 2004). Alternatively, a transgenic animal can also be used, which is capable of producing a complete human antibody library without producing an endogenous immunoglobulin after immunization. For example, it has been reported that the homozygous deletion of the antibody heavy chain joining region (JH) gene in chimeric and germ-line mutant mice results in complete inhibition of endogenous antibody production, and transfer

of the human germ-line immunoglobulin gene array in such germ-line mutant mice will result in the production of human antibodies upon antigen challenge (see, for example, Jakobovits et al., 1993, Proc. Natl. Acad. Sci. USA 90: 2551; Jakobovits et al., 1993, Nature 362 : 255-258; Bruggermann et al., 1993, Year in Immunology 7: 33; and Duchosal et al., 1992, Nature 355: 258). Non-limiting examples of the above-mentioned transgenic animal include HuMAb mice (Medarex, Inc.) which comprises human immunoglobulin gene miniloci encoding unrearranged human heavy chains ($\mu$ and $\gamma$) and $\kappa$ light chain immunoglobulin sequences, and a targeted mutation that inactivates endogenous $\mu$ and $\kappa$ chain loci (see, for example, Lonberg et al. (1994) Nature 368 (6474): 856-859); or "KM mouse™" which carries a human heavy chain transgene and human light chain transchromosome (see: patent application WO02/43478). Other methods of humanizing antibodies include phage display technology (Hoogenboom et al., 1991, J. Mol. Biol. 227: 381; Marks et al., J. Mol. Biol. 1991, 222: 581-597; Vaughan et al., 1996, Nature Biotech 14: 309).

As used herein, the term "humanization degree" refers to an index used to evaluate the number of non-human amino acid residues in a humanized antibody. The humanization degree of a humanized antibody can be assessed, for example, by predicting the homology of the variable region sequence to the human V domain with the DomainGapAlign of IMGT website.

As used herein, the term "homologous antibody" refers to a variant of an antibody, in which the amino acid sequences in the heavy and light chain variable regions contained therein are homologous to the amino acid sequence of the antibody or antigen-binding fragment thereof provided herein, and the variant retains the desired functional properties of the anti-CLDN18.2 antibody of the present invention.

Methods of sequence alignment for comparison are well known in the art. Various procedures and alignment algorithms are described in: Smith TF and Waterman MS, Adv.Appl.Math., 2:482, 1981; Higgins DG and Sharp PM, CABIOS5:151, 1989. Altschul SF et al., Nature Genet., 6:119, 1994 provides detailed ideas for sequence alignment and homology calculations.

As used herein, the term "specific binding" refers to a non-random binding reaction between two molecules, such as a reaction between an antibody and an antigen to which it is directed. The strength or affinity of a specific binding interaction can be expressed in term of the equilibrium dissociation constant (KD) or half-maximum effect concentration (EC50) of the interaction.

The specific binding properties between two molecules can be determined using methods known in the art. One method involves measuring the rate of formation and dissociation of the antigen binding site/antigen complex. Both the "binding rate constant" (ka or kon) and the "dissociation rate constant" (kdis or koff) can be calculated from the concentration and the actual rate of association and dissociation (see Malmqvist M, Nature, 1993, 361: 186-187). The ratio of kdis/kon is equal to the dissociation constant $K_D$ (see Davies et al, Annual Rev Biochem, 1990; 59: 439-473). The $K_D$, kon and kdis values can be measured in any effective way. In certain embodiments, the dissociation constant can be measured using bioluminescence interferometry (e.g., the ForteBio Octet method). In addition, the dissociation constant can be measured by surface plasmon resonance techniques (for example, Biacore) or Kinexa.

As used herein, the term "identity" refers to the match degree between two polypeptides or between two nucleic acids. When two sequences for comparison have the same monomer subunit of base or amino acid at a certain site (e.g., two DNA molecules each have an adenine at a certain site, or two polypeptides each have a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

As used herein, the term "conservative substitution" means an amino acid substitution that does not adversely affect or alter the expected properties of a protein/polypeptide comprising an amino acid sequence, and the antibody variant obtained by conservative substitution retains the biological activity of its original sequence, such as specifically binding to CLDN 18.2. For example, conservative substitutions can be introduced by standard techniques known in the art, such as site-directed mutagenesis and PCR-mediated mutagenesis. Conservative amino acid substitutions include substitutions wherein an amino acid residue is substituted with another amino acid residue having a similar side chain, for example, a residue physically or functionally similar (e.g., having similar size, shape, charge, chemical properties, including ability of forming a covalent bond or a hydrogen bond, etc.) to the corresponding amino acid residue. A family

of amino acid residues having similar side chains has been defined in the art. These families include amino acids having basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g. alanine, valine, leucine, isoleucine, valine, phenylalanine, methionine), beta branch side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al, Biochem. 32: 1180-1187 (1993); Kobayashi et al., Protein Eng. 12(10): 879-884 (1999); and Burks et al. Proc. Natl Acad. Set USA 94: 412-417 (1997), which is incorporated herein by reference).

The twenty conventional amino acids involved herein are expressed in routine manners. See, for example, Immunology-A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. Also in the present disclosure, amino acids are generally represented by single letter and three letter abbreviations as known in the art. For example, alanine can be represented by A or Ala.

The term "pharmaceutically acceptable carrier and/or excipient" as used herein refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995) and includes, but is not limited to, pH adjusting agents, surfactants, adjuvants, ionic strength enhancers, diluents, agents that maintain osmotic pressure, agents that delay absorption, preservatives. For example, pH adjusting agents include, but are not limited to, phosphate buffers. Surfactants include, but are not limited to, cationic, anionic or nonionic surfactants such as Tween-80. Ionic strength enhancers include, but are not limited to, sodium chloride. Preservatives include, but are not limited to, various antibacterial and antifungal agents, such as parabens, chlorobutanol, phenol, sorbic acid, and the like. Agents that maintain osmotic pressure include, but are not limited to, sugars, NaCl, and the like. Agents that delay absorption include, but are not limited to, monostearate and gelatin. Diluents include, but are not limited to, water, aqueous buffers (such as buffered saline), alcohols and polyols (such as glycerin), and the like. Preservatives include, but are not limited to, various antibacterial and antifungal agents, such as thimerosal, 2-phenoxyethanol, parabens, chlorobutanol, phenol, sorbic acid, and the like. Stabilizers have the meaning commonly understood by those skilled in the art which can stabilize the desired activity of the active ingredient in the drug, including but not limited to sodium glutamate, gelatin, SPGA, sugars (e.g., sorbitol, mannitol, starch, sucrose, lactose, dextran, or glucose), amino acids (such as glutamic acid, glycine), proteins (such as dried whey, albumin or casein) or degradation products thereof (such as lactalbumin hydrolysate).

As used herein, the term "prevention" refers to a method performed to prevent or delay the occurrence of a disease or disorder or symptom (e.g., a tumor, infection, or autoimmune disease) in a subject. As used herein, the term "treatment" refers to a method performed to obtain a beneficial or desired clinical result. For the purposes of the present invention, the beneficial or desired clinical result includes, but is not limited to, alleviating symptom, reducing the extent of disease, stabilizing (i.e., no longer exacerbating) the state of disease, delaying or slowing the development of disease, improving or alleviating the status of disease, and alleviating a symptom (either in part or in whole), either detectable or undetectable. In addition, the term "treatment" can also refer to prolonging survival time as compared to the expected survival time (if not receiving treatment).

As used herein, the term "subject" refers to a mammal, such as a primate mammal, such as a human. In certain embodiments, the subject (e.g., a human) suffers from tumor, infection or autoimmune disease, or is at risk of suffering from the aforementioned diseases.

As used herein, the term "effective amount" refers to an amount sufficient to obtain or at least partially obtain a desired effect. For example, an effective amount to prevent a disease (e.g., tumor, infection, or autoimmune disease) refers to an amount sufficient to prevent, stop or delay the occurrence of a disease (e.g., tumor, infection, or autoimmune disease); an effective amount for treating a disease refers to an amount sufficient to cure or at least partially prevent a disease and complications thereof in a patient who has already suffered from the disease. It is completely within the ability of those skilled in the art to determine such an effective amount. For example, the effective amount for a therapeutic use will depend on the severity of the disease to be treated, the overall state of the patient's own immune system, the patient's general conditions such as age, weight and gender, the manner in which the drug is administered, and other therapies administered simultaneously, etc.

As used herein, the term "immune cell" includes cells that have a hematopoietic origin and play a role in immune response, for example, lymphocytes, such as B cells and T cells; natural killer cells; myeloid cells, such as monocytes, macrophages, eosinophils, mast cells, basophils and granulocytes.

As used herein, the term "immune response" refers to an effect of immune cells (e.g., lymphocytes, antigen-presenting cells, phagocytes, or granulocytes) and soluble macromolecules (including antibodies, cytokines, and complements) produced by the immune cells or liver, which leads to selective damage or destruction of invasive pathogens, cells or tissues infected with pathogens, cancer cells, or normal human cells or tissues in the context of autoimmune or

pathological inflammation, or removal of them from the body. In the present invention, the term "antigen-specific T cell response" refers to an immune response produced by a T cell, which is generated when the T cell is stimulated by the T cell specific antigen. Non-limiting examples of response produced by T cell upon antigen-specific stimulation include the proliferation of T cell and the production of cytokine such as IL-2.

As used herein, the term "effector function" refers to those biological activities attributable to the Fc region of an antibody (Fc region of a natural sequence or an amino acid sequence variant), which varies as the isotype of an antibody. Examples of antibody effector functions include, but are not limited to, Fc receptor binding affinity, antibody-dependent cell-mediated cytotoxicity (ADCC), complement dependent cytotoxicity(CDC), antibody-dependent cellular phagocytosis (ADCP), downregulation of cell surface receptors (e.g., B cell receptors), B cell activation, cytokine secretion, half-life/clearance of antibodies and antigen-antibody complexes, and the like. Methods for altering the effector function of an antibody are known in the art, for example by introducing a mutation in the Fc region.

The terms "cancer" and "tumor" are used interchangeably and refer to a large group of diseases characterized by the uncontrolled growth of abnormal cells in the body. Unregulated cell division may lead to the formation of malignant tumors or cells that invade adjacent tissues, and may metastasize to distant parts of the body through the lymphatic system or bloodstream. Cancers include benign and malignant cancers as well as dormant tumors or micrometastasis. Cancers also include blood cancers, especially hematological malignancies.

The term "hematological malignancy" or "hematological tumor" includes lymphoma, leukemia, myeloma or lymphoid malignancies, as well as spleen cancer and lymph node tumors. Exemplary lymphomas include B-cell lymphoma and T-cell lymphoma. B-cell lymphoma includes, for example, Hodgkin's lymphoma. T cell lymphoma includes, for example, skin T cell lymphoma. Hematological malignancies also include leukemia, such as secondary leukemia or acute lymphocytic leukemia. Hematological malignancies also include myeloma (e.g., multiple myeloma) and other hematological and/or B cell or T cell related cancers.

As used herein, the term "pharmaceutically acceptable" means that when a molecular itself, molecular fragment or composition is suitably administered to an animal or a human, it does not produce an adverse, allergic or other untoward reaction. Specific examples of some substances which can be used as a pharmaceutically acceptable carrier or a component thereof include sugars such as lactose, starch, cellulose and derivatives thereof, vegetable oils, gelatin, polyols such as propylene glycol, alginic acid, and the like.

In this context, a combination therapy includes the use of the combinations of the ADC, or composition, or pharmaceutical composition covered by the present invention with one or more therapeutic agents (e.g., chemotherapeutics) of the second therapy, or other preventive or therapeutic modalities (e.g., radiotherapy).

Exemplary therapeutic agents for the second therapy may include chemotherapeutic agents (e.g., mitotic inhibitors), alkylating agents (e.g., Nitrogen Mustard), antimetabolites (e.g., folate analogs), natural products (e.g., Vinca Alkaloid), various reagents (e.g., platinum coordination complexes), hormones and antagonists (e.g., adrenal corticosteroids), immunomodulators (e.g., Bropirimine, Upjohn), etc. Other anti-cancer treatments include other antibodies that specifically target cancer cells.

In this type of combination therapy, various therapeutic agents often have different complementary mechanisms of action, and the combination therapy may result in a synergistic effect. The combination therapy includes therapeutic agents that affect the immune response (e.g., enhancing or activating the response) and therapeutic agents that affect (e.g., inhibiting or killing) tumor/cancer cells. The combination therapy can reduce the possibility of occurrence of drug-resistant cancer cells. The combination therapy may allow the dosage of one or more than one of the agents to be reduced so as to reduce or eliminate an adverse effect associated with one or more than one of the agents. Such combination therapy may have a synergistic therapeutic or preventive effect on a potential disease, disorder or condition.

In this context, the "combination" includes therapies that can be administered separately, such as separately formulated for separate administration (for example, can be provided in a kit), and therapies that can be administered together as a single formulation (i.e., "co-formulation"). In some embodiments, the ADC, composition, or pharmaceutical composition of the present invention can be administered sequentially. In other embodiments, they can be administered simultaneously. The ADC of the present invention can be used in any combination with at least one other (active) agent.

HER2 negative means that there is no significant amount of HER2 proteins on the cell surface, including IHC 1+ or IHC 2+/FISH negative, as well as the range of IHC 0 to 1+ and IHC 1+ to 2+.

As used herein, the term "halogen" includes fluorine, chlorine, bromine, and iodine.

As used herein, the term "$C_{1-6}$ alkyl" refers to a straight- or branched-chain alkyl (group) containing 1-6 carbon atoms, including, for example, "$C_{1-4}$ alkyl" and "$C_{1-3}$ alkyl". Specific examples include but are not limited to: methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1,2-dimethylpropyl.

As used herein, the term "$C_{1-6}$ alkylene" refers to a divalent group obtained by losing two hydrogen atoms of a straight- or branched-chain alkane containing 1-6 carbon atoms, including, for example, "$C_{1-4}$ alkylene " and "$C_{1-3}$ alkylene". Specific examples include but are not limited to: methylene, ethylene, prop-1,3-ylene, but-1,4-ylene, pent-1,5-ylene, and hex-1,6-ylene.

As used herein, the term "$C_{2-10}$ alkenyl" refers to a straight- or branched-chain alkenyl having at least one double bond and a carbon number of 2-10, including, for example, "$C_{2-6}$ alkenyl" and " $C_{2-4}$ alkenyl". Examples include but are not limited to: vinyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, 1,4-hexadienyl, cyclopentenyl, 1,3-cyclopentadienyl, cyclohexenyl, and 1,4-cyclohexadienyl.

As used herein, the term "$C_{2-10}$ alkenylene" refers to a divalent group obtained by losing two hydrogen atoms of an olefin containing 2-10 carbon atoms, including, for example, "$C_{2-8}$ alkenylene" and "$C_{4-6}$ alkenylene". Examples include but are not limited to: penten-1,5-ylene, 2-penten-1,5-ylene, and hexen-1,6-ylene.

As used herein, the term "$C_{2-10}$ alkynyl" refers to a straight- or branched-chain alkynyl having at least one triple bond and a carbon number of 2-10, including, for example, "$C_{2-6}$ alkynyl" and " $C_{2-4}$ alkynyl". Examples include but are not limited to: ethynyl, propynyl, 2-butynyl, 2-pentynyl, 3-pentynyl, 4-methyl-2-pentynyl, 2-hexynyl, 3-hexynyl, and 5-methyl-2-hexynyl.

As used herein, the term "$C_{2-10}$ alkynylene" refers to a divalent group obtained by losing two hydrogen atoms of an alkyne containing 2-10 carbon atoms, including, for example, "$C_{2-8}$ alkynylene" and "$C_{4-6}$ alkynylene". Examples include but are not limited to: pentyn-1,5-ylene, 2-pentyn-1,5-ylene, and hexyn-1,6-ylene.

As used herein, the term "$C_{1-6}$ alkoxy" refers to a group having a structure of $C_{1-6}$ alkyl-O-, wherein $C_{1-6}$ alkyl is as defined above. Specific examples include, but are not limited to, methoxy, ethoxy, propoxy, isopropoxy, n-propoxy, isopropoxy, n-butoxy, isobutoxy, tert-butoxy, pentoxy, and hexoxy.

As used herein, the term "5-12 membered heteroaryl" refers to an aromatic cyclic group containing 5-12 ring members, one of which is at least a heteroatom selected from N, O, and S. Specific examples include but are not limited to: 5-10 membered heteroaryl, 5-10 membered nitrogen-containing heteroaryl, and 5-6 membered oxygen-containing heteroaryl, such as furyl, thienyl, pyrrolyl, thiazolyl, isothiazole, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl, and 1,2,4,5-tetrazinyl.

The present invention is not limited to the specific methodologies, protocols, cell lines, vectors/carriers, or reagents described herein, as they can vary. In addition, the terms used herein are only used for the purpose of describing specific embodiments, not for limiting the scope of the present invention.

In this context (Specification and Claims), the singular forms "a", "an", "the", and "said", unless the context clearly specified otherwise, include the plural forms thereof. For example, "a host cell" includes a plurality of such host cells. In addition, there are no corresponding English singular and plural grammatical rules in Chinese, where the singular and plural forms of a noun must be judged according to the context or actual situation. Therefore, in the English translation, the prefix "one or more" of a noun is probably correct.

## BRIEF DESCRIPTION OF THE DRAWINGS

Figure 1A shows the detection of the monoclonal stable cell line of HEK293T-Claudin18.2 by flow cytometry.

Figure 1B shows the detection of the monoclonal stable cell line of L929-Claudin18.2 by flow cytometry.

Figure 1C shows the detection of the monoclonal stable cell line of KATOIII-Claudin18.2 by flow cytometry.

Figure 1D shows the detection of the monoclonal stable cell line of NCI-N87-Claudin18.2 by flow cytometry.

Figure 1E shows the detection of the monoclonal stable cell line of HEK293T-Claudin18.1 by Western blot.

Figure 2 shows the determination of the affinity of 2C6.9-hz21 and IMAB362 by flow cytometry.

Figure 3 shows the determination of the affinity of 2C6.9-hz21 and IMAB362 binding to L929-Claudin 18.2 cells.

Figure 4 shows the determination of the specificity of 2C6.9-hz21 by flow cytometry.

Figure 5 shows the determination of the CDC killing activity of 2C6.9-hz21 and IMAB362 against HEK293T-Claudin18.2 cells.

Figure 6 shows the determination of the ADCC activity of 2C6.9-hz21 and IMAB362 against HEK293T-Claudin18.2

cells.

Figure 7 shows the HPLC-SEC spectrum of 2C6.9-TL001 (DAR: 3.79).

Figure 8 shows the HPLC-SEC spectrum of 2C6.9-TL001 (DAR: 7.12).

Figure 9 shows the test results of the affinity of 2C6.9-TL001 (DAR: 7.12) to Claudin 18.2 on the cell membrane surface.

Figure 10A shows the test results of the cytotoxicity effect of 2C6.9-TL001 (DAR: 7.12) on HEK293T-Claudin 18.2.

Figure 10B shows the test results of the cytotoxicity effect of 2C6.9-TL001 (DAR: 3.79) on HEK293T-Claudin 18.2.

Figure 10C shows the test results of the cytotoxicity effect of 2C6.9-TL001 (DAR: 7.12) on HEK293T-Claudin 18.1.

Figure 10D shows the test results of the cytotoxicity effect of 2C6.9-TL002 and 2C6.9-TL003 on HEK293T-Claudin 18.2.

Figure 10E shows the test results of the cytotoxicity effect of 2C6.9-TL002 and 2C6.9-TL003 on HEK293T-Claudin 18.1.

Figure 11A shows the test results of the cytotoxicity effect of 2C6.9-TL001 (DAR: 7.12) on NUGC-4 cells.

Figure 11B shows the test results of the cytotoxicity effect of 2C6.9-TL001 (DAR: 3.79) on NUGC-4 cells.

Figure 11C shows the test results of the cytotoxicity effect of 2C6.9-TL002 and 2C6.9-TL003 on NUGC-4 cells.

Figure 12A shows the tumor volume changes of mouse in each group in subcutaneous transplanted NCI-N87-Claudin18.2 cell tumor model in Balb/c Nude mice (*: $P<0.05$; ****: $P < 0.0001$).

Figure 12B shows the body weight changes of mouse in each group in subcutaneous transplanted NCI-N87-Claudin18.2 cell tumor model in Balb/c Nude mice.

Figure 12C shows the comparison of the 11-day *in vivo* efficacy of 2C6.9-TL001 and 2C6.9 mAb + chemotherapy in CDX model (****: $P<0.0001$).

Figure 12D shows the comparison of the 21-day *in vivo* efficacy of 2C6.9-TL001 and 2C6.9 monoclonal antibody + chemotherapy in CDX model (****: $P < 0.0001$).

Figure 12E shows the tumor volume changes within 21 days ofmouse in each group treated with 2C6.9-TL001 with different DAR values in CDX (NUGC-4) model (****: $P < 0.001$).

Figure 12F shows the body weight changes within 21 days of mouse in each group treated with 2C6.9-TL001 with different DAR values in CDX (NUGC-4) model.

Figure 12G shows the tumor volume changes within 17 days of mouse in each group in the subcutaneous HuPrime® gastric cancer GA0006 PDX model of Balb/c Nude mice (****: $P < 0.0001$).

Figure 12H shows the body weight changes within 17 days of mouse in each group in the subcutaneous HuPrime® gastric cancer GA0006 PDX model of Balb/c Nude mice.

Figure 12I shows the tumor volume changes within 24 days of mouse in each group in the subcutaneous HuPrime® gastric cancer GA0006 PDX model of Balb/c Nude mice (****: $P < 0.0001$).

Figure 12J shows the body weight changes within 24 days of mouse in each group in the subcutaneous HuPrime® gastric cancer GA0006 PDX model of Balb/c Nude mice.

Figure 12K shows the tumor volume changes of mouse in each group in the subcutaneous NCI-N87-Claudin18.2

cell transplantation tumor model of Balb/c Nude mice (***: *P* < 0.001; ****: P < 0.0001).

Figure 12L shows the body weight changes of mouse in each group in the subcutaneous NCI-N87-Claudin18.2 cell transplantation tumor model of Balb/c Nude mice.

Figure 12M shows the tumor volume changes of mouse in each group in the subcutaneous HEK293T-Claudin18.2 cell transplantation tumor model of Balb/c Nude mice (**: *P* < 0.01; ****: P < 0.0001).

Figure 12N shows the body weight changes of mouse in each group in the subcutaneous HEK293T-Claudin18.2 cell transplantation tumor model of Balb/c Nude mice.

Figure 12O shows the tumor volume changes of mouse in each group in the subcutaneous NUGC-4 cell transplantation tumor model of Balb/c Nude mice (****: *P* < 0.0001).

Figure 12P shows the body weight changes of mouse in each group in the subcutaneous NUGC-4 cell transplantation tumor model of Balb/c Nude mice.

Figure 13 shows the HPLC-SEC spectrum of 2C6.9-TL001 (DAR:7.40).

## SPECIFIC MODE FOR CARRYING OUT THE INVENTION

The embodiments of the present invention will be described in detail below with reference to the examples, but those skilled in the art will understand that the following examples are only used to illustrate the present invention and should not be regarded as limiting the scope of the present invention. The specific conditions which are not indicated in the examples are subject to the conventional conditions or the conditions suggested by the manufacturers. The reagents or instruments used without indicating any manufacturer are all commercially available conventional products.

Example 1. Synthesis of bioactive molecule (TOXIN-1)

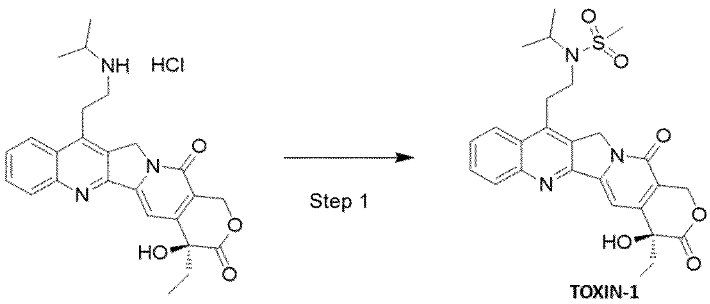


TOXIN-1

Methanesulfonyl chloride (462 mg, 12.77 mmol, about 70% purity) was added dropwise to the dichloromethane (40 mL) solution of Belotecan hydrochloride (3g, 6.38 mmol) and triethylamine (2.58 g, 25.54 mmol), the resulting mixture was allowed to react for 2h at room temperature. After filtration by suction, the filter cake was washed three times with dichloromethane (3 mL) to obtain 2.2 g of (S)-N-(2-(4-ethyl-4-hydroxy-3,14-dione-3,4,12,14-tetrahydro-1H-pyrano[3',4':6,7] indozino[1,2-b] quinolin-11-yl) ethyl)-N-isopropyl methane sulfonamide (TOXIN-1).

Structural characterization data is as follows:

$^1$H NMR (400 MHz, DMSO-$d_6$) δ 8.32 (d, *J* = 8.4 Hz, 1H), 8.20 (dd, *J* = 8.4, 1.2 Hz, 1H), 7.93-7.84 (m, 1H), 7.79 (t, *J*= 7.6 Hz, 1H), 7.35 (s, 1H), 6.56 (s, 1H), 5.44 (d, *J*= 9.2 Hz, 4H), 3.98 (p, *J* = 6.7 Hz, 1H), 3.50 (t, *J* = 8.0 Hz, 2H), 3.42-3.35 (m, 2H), 3.00 (s, 3H), 1.93-1.82 (m, 2H), 1.15 (d, *J*= 6.7 Hz, 6H), 0.88 (t, *J*= 7.3 Hz, 3H).

ESI-MS (m/z): 512.2 $[M+H]^+$.

$[\alpha]_D^{20}$ is +28.19° (c =0.101 g/100 mL, $CH_3CN$).

Example 2. Synthesis of 6-(2-(methylsulfonyl) pyrimidin-5-yl)-5-hexynoic acid (compound 3-4)

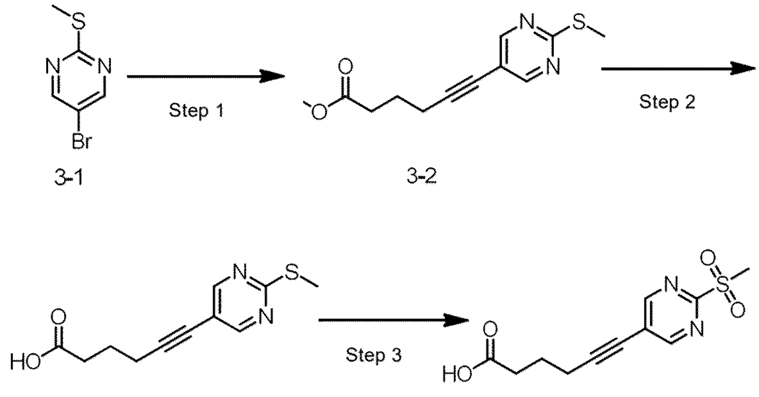


3-1 3-2 3-3 3-4

Step 1: Synthesis of 6-(2-(methylthio) pyrimidin-5-yl)-5-hexynoic acid methyl ester (compound 3-2)

At room temperature, methyl 5-hexynoate (500 mg, 3.97 mmol) and 5-bromo-2-methylthiopyrimidine were dissolved in N, N-dimethylformamide (3 mL), triethylamine (3 mL), cuprous iodide (75 mg, 0.4 mmol), and palladium (II) bis(triphenylphosphine) dichloride (279 mg, 0.4 mmol) were added successively. Then, the mixure was heated to 95°C under the protection of nitrogen and allowed to react with stirring for 6h. The reaction was quenched with water. The reactant was extracted with ethyl acetate (20 mL x 3). The organic phases were combined, washed with saturated brine (20 mL x 2), dried with anhydrous sodium sulfate, filtered to remove the drying agent, concentrated in vacuo, and purified by preparative liquid chromatography to obtain 300 mg of the title compound. ESI-MS (*m/z*): 251.3 $[M + H]^+$.

Step 2: Synthesis of 6-(2-(methylthio) pyrimidin-5-yl)-5-hexynoic acid (compound 3-3)

At room temperature, compound 3-2 (200 mg, 0.8 mmol) was dissolved in a mixed solution of tetrahydrofuran and water (4 mL/4 mL), lithium hydroxide monohydrate (235 mg, 5.6 mmol) was added, the resulting mixture was allowed to react with stirring for 4h at room temperature, followed by dilution with water, and extraction with ethyl acetate (20 mL x 2); the aqueous phase was adjusted with 1N hydrochloric acid to pH = 3, extracted with ethyl acetate (20 mL x 3); the organic phases were combined, washed with saturated brine (20 mL x 2), dried with anhydrous sodium sulfate, filtered to remove the drying agent, and concentrated in vacuo to obtain 120 mg of the title compound.

Step 3: Synthesis of 6-(2-(methylsulfonyl) pyrimidin-5-yl)-5-hexynoic acid (compound 3-4)

At room temperature, compound 3-3 (20 mg, 0.085 mmol) was dissolved in dichloromethane (4 mL), m-chloroperoxybenzoic acid (22 mg, 0.127 mmol) was added, the resulting mixture was allowed to react with stirring at room temperature overnight, and purified with preparative liquid chromatography to obtain 20 mg of the title compound. ESI-MS (m/z): 269.1 $[M + H]^+$.

Example 3. (4-((S)-2-(4-aminobutyl)-35-(4-((6-(2-(methylsulfonyl)pyrimidin-5-yl)hex-5-ynamido)methyl)-1H-1,2,3-triazol-1-yl)-4,8-dioxo-6,12,15,18,21,24,27,30,33-nonaoxa-3,9-diazapentatriacontanamido)benzyl) ((S)-4-ethyl-11-(2-(N-isopropylmethylsulfonamido)ethyl)-3,14-dioxo-3,4,12,14-tetrahydro-1H-pyrano[3',4':6,7]indozino[1,2-b]quinolin-4-yl)carbonate (compound TL001)

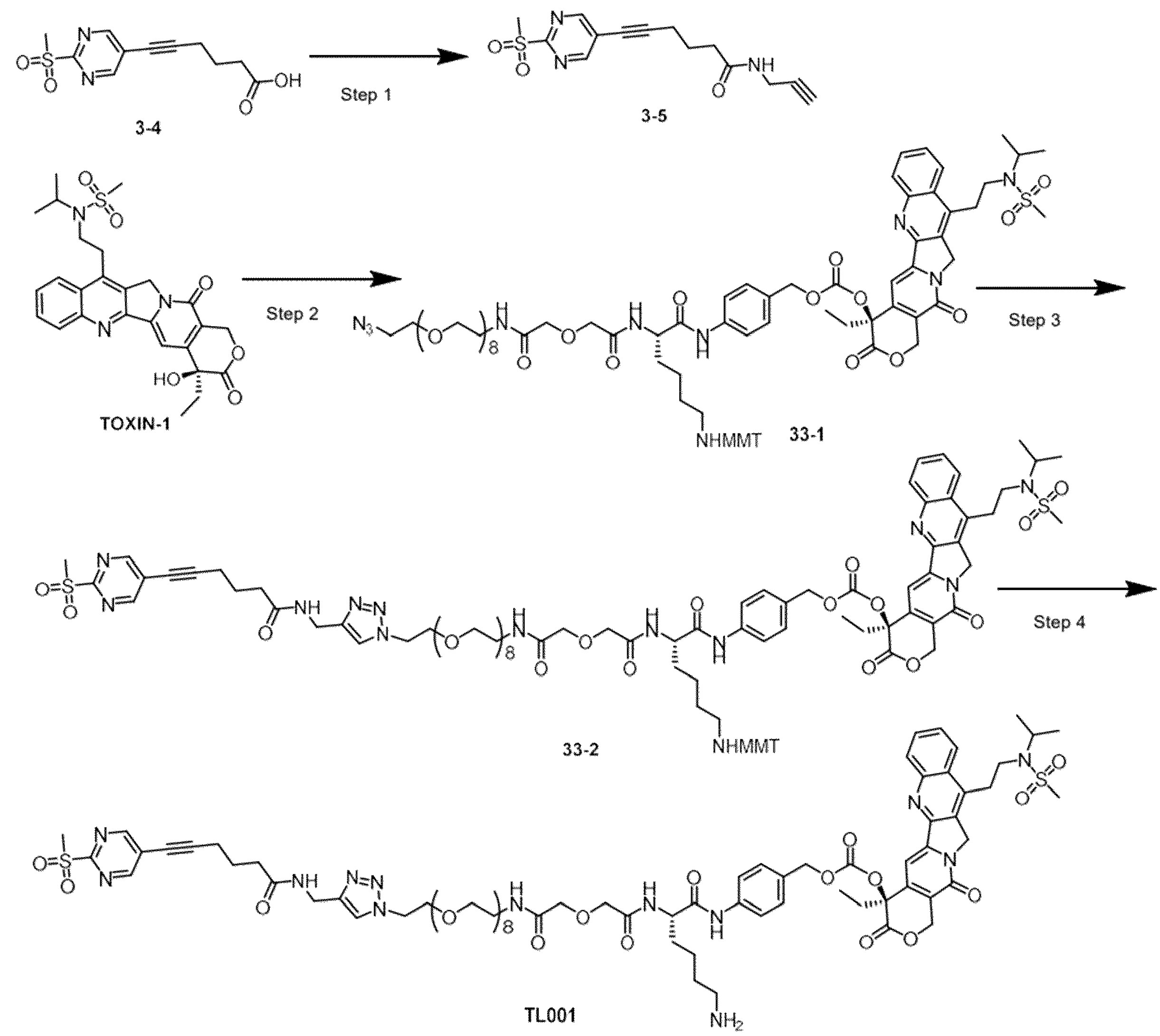


3-4 3-5 TOXIN-1 33-1 33-2 TL001

Step 1: Synthesis of 6-(2-(methylsulfonyl) pyrimidin-5-yl)-N-propargyl-hex-5-ynamide (compound 3-5)

Propynylamine (189 mg, 3.4 mmol) and compound 3-4 (800 mg, 2.83 mmol) were dissolved in dichloromethane (10 mL) under 25°C, then N, N-diisopropylethylamine (738 mg, 5.67 mmol) and O-(7-aza-benzotriazol-1-yl)-N, N, N', N'-tetramethyl urea hexafluorophosphate (1.63 g, 4.25 mmol) were added sequentially. The resulting mixture was allowed to react with stirring for 2h. The reaction solution was concentrated in vacuo, the residue was purified by flash silica gel column (ethyl acetate/petroleum ether = 3/1) to obtain 700 mg of the title compound. ESI-MS (m/z): 306.1 $[M + H]^+$.

Step 2: Synthesis of (4-((S)-35-azido-2-(4-(((4-methoxyphenyl) diphenylmethyl) amino) butyl)-4,8-dioxo-6,12,15,18,21,24,27,30,33-nonaoxa-3,9-diazapentatriacontanamido) benzyl)((S)-4-ethyl-11-(2-(N-isopropylmethylsulfonamido) ethyl)-3,14-dioxo-3,4,12,14-tetrahydro-1H-pyrano[3',4':6,7] indozino[1,2-b] quinolin-4-yl) carbonate (compound 33-1).

TOXIN-1 (250 mg, 0.49 mmol) was dissolved in dichloromethane (10 mL) under the protection of nitrogen at 25°C, cooled to 0°C, added with the dichloromethane (3 mL) solution of 4-dimethylaminopyridine (478 mg, 3.91 mmol), followed by an addition of the dichloromethane (10 mL) solution of triphosgene (72 mg, 0.24 mmol) in a slow dropwise manner. After the addition, the resulting mixture was allowed to react with stirring for 20 min at 0°C, and purged with nitrogen for 20 min. The dichloromethane (7 mL) solution of (S)-2-(32-azido-5-oxo-3,9,12,15,18,21,24,27,30-nonaoxa-6-aza-dotriacontanamido)-N-(4-(hydroxymethyl)phenyl)-6-(((4-methoxyphenyl) diphenylmethyl)amino) hexanamide (518 mg, 0.49 mmol) was then added and stirred at 0°C for 1h. The reaction solution was concentrated under reduced pressure, the residue was purified with preparative liquid chromatography to obtain 500 mg of the title compound. ESI-MS (m/z): 1597.5 $[M+H]^+$.

Step 3: Synthesis of ((S)-4-ethyl-11-(2-(N-isopropylmethylsulfonamido) ethyl) -3,14-dioxo -3,4,12,14-tetrahydro-1H-pyrano[3',4':6,7]indolizino[1,2-b]quinolin-4-yl)(4-((S)-2-(4-(((4-methoxyphenyl)diphenylmethyl)amino)butyl)-35-(4-((6-(2-(methylsulfonyl)pyrimidin-5-yl)hex-5-ynamido)methyl)-1H-1,2,3-triazol-1-yl)-4,8-dioxo-6,12,15,18,21,24,27,30,33-nonoxy-3,9-diaza pentatriacontanamido) benzyl) carbonate (compound 33-2)

At room temperature, compound 33-1 (80 mg, 0.05 mmol) and the fragment 3-5 (23 mg, 0.075 mmol) were dissolved in the solution of dimethyl sulfoxide and water (2.0 mL: 0.5 mL), then cuprous bromide (11 mg, 0.08 mmol) was added. The resulting mixture was stirred for 1h, and purified with preparative high-performance liquid chromatography to obtain 30 mg of the title compound. ESI-MS (m/z): 815.9 [(M-273)/2+H $]^+$.

Step 4: Synthesis of 4-((S)-2-(4-aminobutyl)-35-(4-((6-(2-(methylsulfonyl) pyrimidin-5-yl) hex-5-ynamido) methyl)-1H-1,2,3-triazol-1-yl)-4,8-dioxo-6,12,15,18,21,24,27,30,33-nonaoxa-3,9-diazapentatriacontanamido)benzyl) ((S)-4-ethyl-11-(2-(N-isopropylmethylsulfonamido) ethyl)-3,14-dioxo-3,4,12,14-tetrahydro-1H-pyrano[3',4':6,7]indolizino[1,2-b]quinolin-4-yl)carbonate (compound TL001).

Compound 33-2 (30 mg, 0.02 mmol) was dissolved in dichloromethane (1.0 mL), followed by an addition of trifluoroacetic acid (0.2 mL). The resulting mixture was allowed to react for 30 min at room temperature, purified with preparative HPLC to obtain 20.0 mg trifluoroacetic acid salt of the title compound. The structure is characterized as follows:

$^1$H NMR (400 MHz, DMSO-$d_6$) δ 10.18 (s, 1H), 9.10 (s, 2H), 8.38 (t, *J* = 5.56 Hz, 1H), 8.32 (d, J = 8.40 Hz, 1H), 8.22 - 8.20 (m, 2H), 8.09 (t, *J* = 5.68 Hz, 1H), 7.91 - 7.87 (m, 2H), 7.82 - 7.78 (m, 1H), 7.69 (brs, 3H), 7.61 (d, *J*= 8.56 Hz, 2H), 7.32 (d, *J*= 8.56 Hz, 2H), 7.06 (s, 1H), 5.56 (d, *J* = 16.96 Hz, 1H), 5.51 (d, *J* = 16.96 Hz, 1H), 5.47 (d, *J* = 19.28 Hz, 1H), 5.42 (d, *J* = 19.28 Hz, 1H), 5.14 (d, *J* = 12.20 Hz, 1H), 5.07 (d, *J*= 12.16 Hz, 1H), 4.48 (t, *J* = 5.24 Hz, 2H), 4.46 - 4.43 (m, 1H), 4.29 (d, *J* = 5.60 Hz, 2H), 4.08 - 3.95 (m, 5H), 3.79 (t, *J* = 5.28 Hz, 2H), 3.51 - 3.43 (m, 32H), 3.40 (s, 3H), 3.39 - 3.35 (m, 2H), 3.30 - 3.26 (m, 2H), 3.00 (s, 3H), 2.82 - 2.74 (m, 2H), 2.56 (t, *J* = 7.08 Hz, 2H), 2.29 (t, *J* = 7.36 Hz, 2H), 2.23 - 2.13 (m, 2H), 1.82 (p, *J*= 7.24 Hz, 2H), 1.78 - 1.63 (m, 2H), 1.61 - 1.49 (m, 2H), 1.42 - 1.27 (m, 2H), 1.15 (d, *J* = 6.80 Hz, 3H), 1.13 (d, *J* = 6.76 Hz, 3H), 0.90 (t, *J* = 7.32 Hz, 3H).

ESI-MS (m/z): 816.0[M/2+H$]^+$.

$[\alpha]_D^{20}$ is -19.55° (c =1.000 g/100 mL, $CH_3CN$).

Example 4. Synthesis of ((S)-4,1 1-diethyl-4-hydroxy-3,14-dioxo -3,4,12,14-tetrahydro -1H-pyrano[3',4':6,7] indozino[1,2-b]quinolin-9-yl)(4-((S)-2-((S)-3-methyl-2-(4-(1-(26-(4-((6-(2-(methylsulfonyl)pyrimidin-5-yl)hex-5-ynamido) methyl)-1H-1,2,3-triazol-1-yl)-3,6,9,12,15,18,21,24-octaoxahexacosanyl) piperidin-4-yl) butyramido) butyramido)propionamido)benzyl)ethan-1,2-diyl bis(methylcarbamate) trifluoroacetate (TL002)

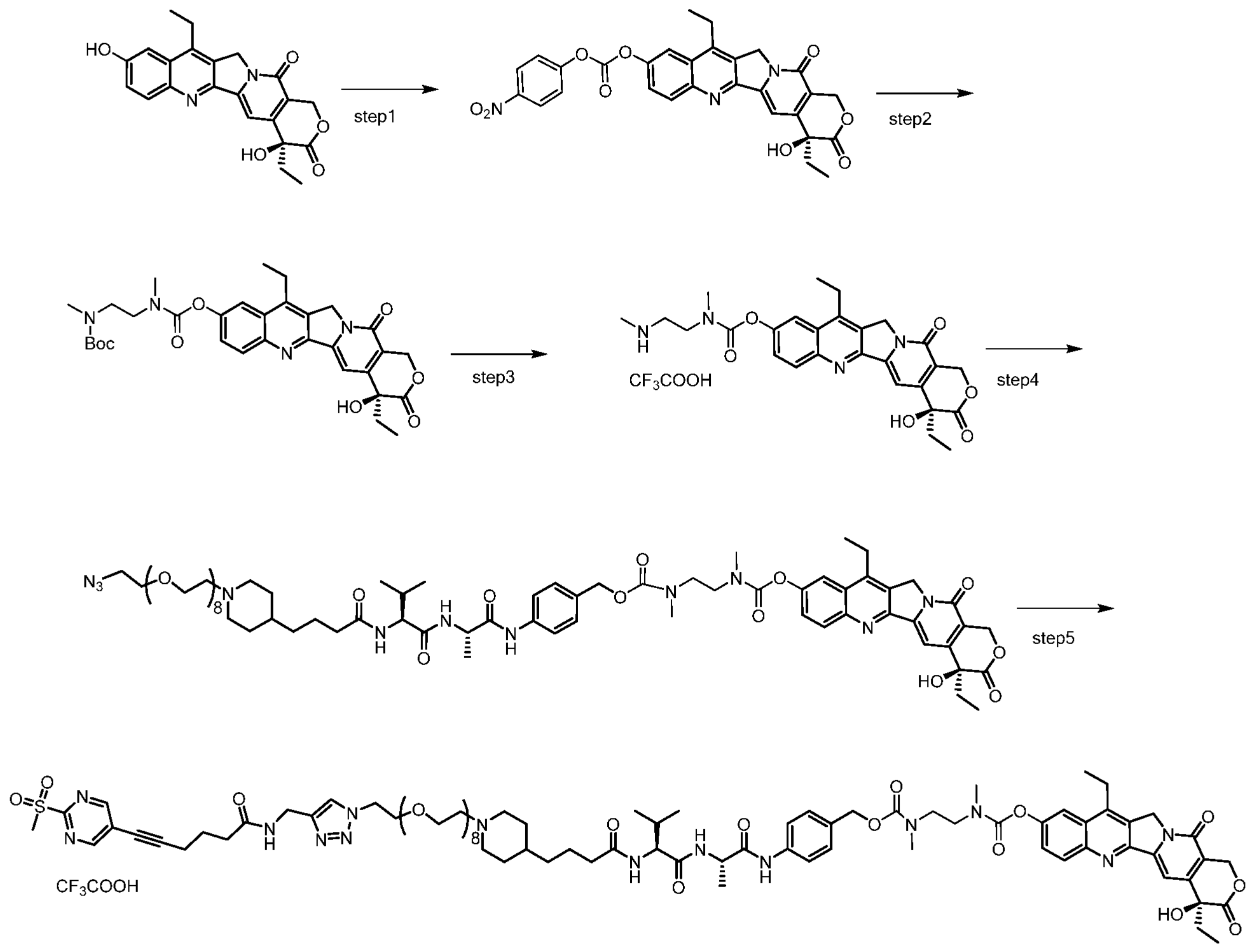


Step 1: Synthesis of (S)-(4,11-diethyl-4-hydroxy-3,14-dioxo-3,4,12,14-tetrahydro-1H-pyrano[3',4':6,7] indozino[1,2-b] quinolin-9-yl(4-nitrophenyl) carbonate

(S)-4,11-diethyl-4,9-dihydroxy-1H-pyrano[3',4':6,7] indozino[1,2-b] quinolin-3,14(4H, 12H)-dione (150 mg, 0.37 mmol) was dissolved in dichloromethane (15 mL), diisopropylethylamine (96.81 mg, 0.74 mmol) was added, followed by an addition of the solution of bis (4-nitrophenyl) carbonate (127.92 mg, 0.41 mmol) in dichloromethane (15mL), the resulting mixture was allowed to react at 25°C for 3h. The reaction solution was concentrated to obtain 207 mg of the title compound, which was directly used for next reaction.

ESI-MS (m/z):558.1[M+H]$^+$.

Step 2: Synthesis of (S)-tert-butyl (4,11-diethyl-4-hydroxy-3,14-dioxo-3,4,12,14-tetrahydro-1H-pyrano[3',4':6,7] indozino[1,2-b] quinolin-9-yl) ethan-1,2-diyl bis (methylcarbamate)

(S)-(4,11-diethyl-4-hydroxy-3,14-dioxo-3,4,12,14-tetrahydro-1H-pyrano[3',4':6,7] indozino[1,2-b] quinolin-9-yl) (4-nitrophenyl) carbonate (207 mg, 0.33 mmol) was dissolved in dichloromethane (10 mL), diisopropyl ethylamine (130.87 mg, 1.00 mmol) and tert-butyl N-methyl-N-[2-(methylamino) ethyl] carbamate (71.34 mg, 0.37 mmol) were added, the resulting mixture was stirred at 25°C for 12h. The reaction solution was concentrated, the residue was purified by silica gel column chromatography (eluent: dichloromethane/methanol = 9/1) to obtain 207 mg of the title compound.

ESI-MS (m/z):607.3[M+H]$^+$.

Step 3: Synthesis of (S)-N-methyl-(4,11-diethyl-4-hydroxy-3,14-dioxo-3,4,12,14-tetrahydro-1H-pyrano[3',4':6,7] indozino[1,2-b] quinolin-9-yl) (2- (methylamino) ethyl) carbamate trifluoroacetate

(S)-tert-butyl (4,11-diethyl-4-hydroxy-3,14-dioxo-3,4,12,14-tetrahydro-1H-pyrano[3',4':6,7] indozino[1,2-b] quinoline-9-yl) ethan-1,2-diyl bis (methylcarbamate) (207 mg, 0.19 mmol) was dissolved in dichloromethane (8 mL), trifluoroacetic acid (2 mL) was added, the resulting mixture was allwed to react at 25°C for 2h. The reaction solution was

concentrated to obtain 200 mg of the title compound, which was directly used for next reaction.

ESI-MS (m/z):507.2[M+H]$^+$.

Step 4: Synthesis of 4-((S)-2-((S)-2-(4-(1-(26-azido-3,6,9,12,15,18,21,24-octaoxahexacosanyl) piperidin-4-yl) butyramido)-3-methylbutyramido) propionamido) benzyl) ((S)-4,11-diethyl-4-hydroxy-3,14-dioxo-3,4,12,14-tetrahydro-1H-pyrano[3',4': 6,7] indozino[1,2-b] quinolin-9-yl) ethan-1,2-diyl bis (methylcarbamate)

(4-((S)-2-((S)-2-(4-(1-(26-azido-3,6,9,12,15,18,21,24-octaoxahexacosanyl)piperidin-4-yl)butyramido)-3-methylbutyramido) propionamido)benzyl) (4-nitrophenyl) carbonate (150 mg, 0.12 mmol) was dissolved in N,N-dimethylformamide (3 mL), 1-hydroxybenzotriazole (34.03 mg, 0.25 mmol) and diisopropyl ethylamine (48.82 mg, 0.37 mmol) were added, followed by an addition of (S)-N-methyl-(4,11-diethyl-4-hydroxy-3,14-dioxo-3,4,12,14-tetrahydro-1H-pyrano[3',4':6,7] indozino[1,2-b] quinolin-9-yl) (2-(methylamino) ethyl) carbamate trifluoroacetate (102.11 mg, 0.12 mmol), the resulting mixture was allowed to react at 25°C for 16h. The reaction solution was purified by preparative HPLC, the collected fractions were subjected to lyophilization to obtain 44 mg of the title compound.
ESI-MS (m/z):1400.7[M+H]$^+$.
Chromatographic column: Waters XBridge Prep C18 OBD 19 mm×150 mm×5.0 μm
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 3.00 | 10 | 90 | 28 |
| 19.00 | 90 | 10 | 28 |

Step 5: Synthesis of ((S)-4,11-diethyl-4-hydroxy-3,14-dioxo-3,4,12,14-tetrahydro-1H-pyrano[3',4':6,7] indozino[1,2-b] quinolin-9-yl) (4-((S)-2-((S)- 3-methyl-2-(4-(1-(26-(4-((6-(2-(methylsulfonyl)pyrimidin-5-yl)hex-5-ynamido)methyl)-1H-1,2,3-triazol-1-yl)-3,6,9,12,15,18,21,24-octaoxahexacosanyl)piperidin-4-yl)butyramido)butyramido) propionamido)benzyl)ethan-1,2-diyl bis(methylcarbamate)trifluoroacetate

4-((S)-2-((S)-2-(4-(1-(26-azido-3,6,9,12,15,18,21,24-octaoxahexacosanyl)piperidin-4-yl)butyramido)-3-methylbutyramido) propionamido)benzyl)((S)-4,1 1-diethyl-4-hydroxy-3,14-dioxo-3,4,12,14-tetrahydro-1H-pyrano[3',4':6,7] indozino[1,2-b]quinolin-9-yl)ethan-1,2-diyl bis(methylcarbamate) (44 mg, 0.30 mmol) and 6-(2-(methylsulfonyl) pyrimidin-5-yl)-N-propyl)hexamide (14.09 mg, 0.045 mmol) were dissolved in a solution of dimethyl sulfoxide (3 mL) and water (0.75 mL), cuprous bromide (8.65 mg, 0.06 mmol) was added, the resulting mixture was allowed to react at 25°C for 1h. The reaction solution was purified by preparative HPLC, the collected fractions were subjected to lyophilization, which was then dissolved in dichloromethane (2 mL), added with trifluoroacetic acid (0.2 mL), and stirred at 25°C for 0.5h, purified by preparative HPLC, and the collected fractions were subjected to lyophilization to obtain 16 mg of the title compound.
ESI-MS (m/z): 853.6[M/2+H]$^+$.
Chromatographic column: Waters SunFire Prep C18 ODS 5 μm 19×50 mm
Mobile phase A: acetonitrile; Mobile phase B: water (0.05% trifluoroacetic acid)

| Time [min] | Mobile phase A [%] | Mobile phase B [%] | Flow rate [mL/min] |
|---|---|---|---|
| 0.00 | 10 | 90 | 28 |
| 4.00 | 10 | 90 | 28 |
| 20.00 | 90 | 10 | 28 |

Example 5. Synthesis of (4-((S)-2-((S)-3-methyl-2-(4-(1-(26-(4-((6-(2-(methylsulfonyl) pyrimidin-5-yl) hex-5-ynamido) methyl)-1H-1,2,3-triazol-1-yl)-3,6,9,12,15,18,21,24-octaoxahexacosanyl)piperidin-4-yl) butyramido)butyramido)propionamido)benzyl)(2-((S)-4-ethyl-4-hydroxy-3,14-dioxo-3,4,12,14-tetrahydro-1H-pyrano[3',4':6,7]indozino[1,2-b] quinolin-11-yl) N-ethyl-N-isopropylcarbamate trifluoroacetate (TL003)

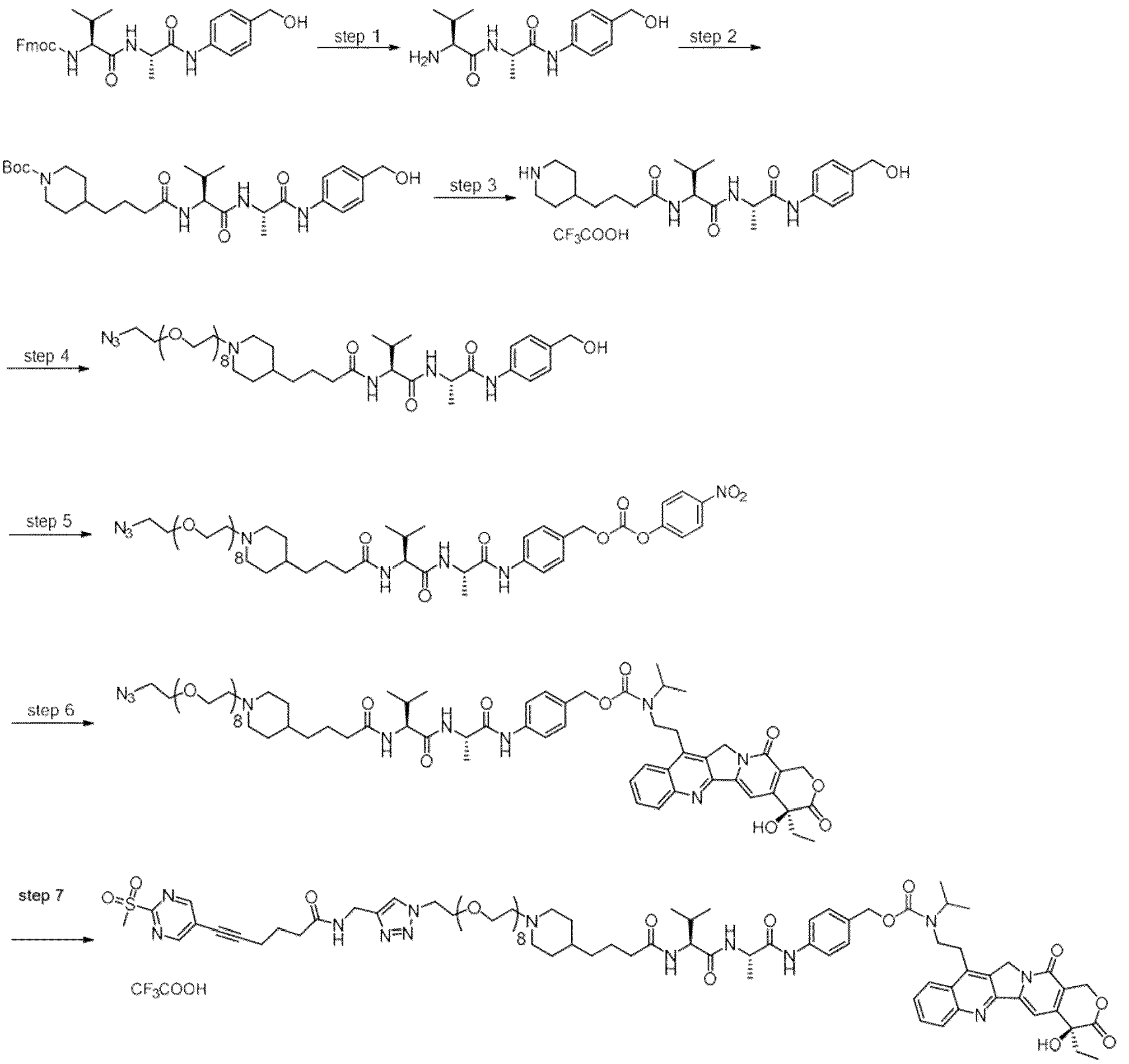


Step 1: Synthesis of (S)-2-amino-N-((S)-1-((4-(hydroxymethyl)phenyl) amino)-1-oxoprop-2-yl)-3-methylbutyramide

At room temperature, ((9H-fluoren-9-yl) methyl)(((S)-1-((S)-1-((4-(hydroxymethyl)phenyl)amino)-1-oxoprop-2-yl)amino)-3-methyl-1-oxobut-2-yl) carbamate (2.00 g, 3.88 mmol) was dissolved in N,N-dimethylformamide (12 mL), piperidine (3 mL) was added, the resulting mixture was allowed to react at 25°C for 2h. The reaction solution was then poured into water to precipitate solids, and the solids were filtered, purified by preparative HPLC. The collected fractions were subjected to lyophilization to obtain 810 mg of the title compound.
ESI-MS (*m*/*z*): 294.0$[M+H]^+$.
Chromatographic column: Waters SunFire Prep C18 ODS 8 μm 45×450 mm
Mobile phase A: acetonitrile; Mobile phase B: water

| Time [min] | Mobile phase A [%] | Mobile phase B [%] | Flow rate [mL/min] |
|---|---|---|---|
| 0.00 | 7 | 93 | 60 |
| 7.00 | 7 | 93 | 60 |
| 50.00 | 50 | 50 | 60 |

Step 2: Synthesis of *tert*-butyl 4-(4-((S)-1-((S)-1-((4-(hydroxymethyl) phenyl)amino)-1-oxoprop-2-yl) amino-3-methyl-1-oxobut-2-yl) amino-4-oxobutyl) piperidin-1-carboxylate

At room temperature, (S)-2-amino-N-((S)-1-((4-(hydroxymethyl) phenyl) amino)-1-oxoprop-2-yl)-3-methylbutyramide (810 mg, 2.76 mmol), 4-(1-(tert-butoxycarbonyl) piperidin-4-yl) butyric acid and 2-ethoxy-1-ethoxycarbo-1,2-dihydroquinoline were dissolved in dichloromethane (8 mL) and methanol (8 mL), and allowed to react at 45°C for 2h. The reaction solution was concentrated in vacuo, and the residue was purified by silica gel chromatography column (eluent: dichloromethane/methanol = 15/1) to obtain 1.31 g of the title compound.
ESI-MS *(m/z):* 546.8$[M+H]^+$.

Step 3: Synthesis of (S)-N-((S)-1-((4-(hydroxymethyl) phenyl) amino)-1-oxoprop-2-yl)-3-methyl-2-(4-(piperidin-4-yl) butyryl amido) butyramide trifluoroacetate

At room temperature, *tert*-butyl 4-(4-((S)-1-((S)-1-((4-(hydroxymethyl) phenyl amino)-1-oxoprop-2-yl) amino-3-methyl-1-oxobut-2-yl) amino-4-oxobutyl) piperidin-1-formate (1.3 g, 2.14 mmol) was dissolved in dichloromethane (20 mL), trifluoroacetic acid (5 mL) was added and allowed to react at 25°C for 2h. The reaction solution was concentrated under reduced pressure, and the residue was dissolved in acetonitrile (30 mL), added with potassium carbonate (1.22 g, 8.85 mmol) and allowed to react at 25°C for 2h. The reaction mixture was filtered by suction, the filter cake was washed with acetonitrile, the filtrate was collected, and concentrateed under reduced pressure to obtain 900 mg of the title compound.
ESI-MS *(m/z):* 446.9$[M+H]^+$.

Step 4: Synthesis of (S)-2-(4-(1-(26-azido-3,6,9,12,15,18,21,24-octaoxahexacosanyl) piperidin-4-yl) butyramido)-N-((S)-1-((4-(hydroxymethyl) phenyl) amino)-1-oxoprop-2-yl)-3-methylbutyramide

At room temperature, (S)-N-((S)-1-((4-(hydroxymethyl) phenyl) amino)-1-oxoprop-2-yl)-3-methyl-2-(4-(piperidin-4-yl) butyryl amido) butyramide trifluoroacetate (487 mg, 0.78 mmol) and 26-azido-3,6,9,12,15,18,21,24-octaoxahexacosanyl p-toluenesulfonate (619 mg, 0.94 mmol) were dissolved in acetonitrile (20 mL), added with potassium carbonate (655 mg, 4.69 mmol) and allowed to react at 16°C for 6h. The reaction solution was concentrated under reduced pressure, the residue was purified by silica gel chromatography column (eluent: dichloromethane/methanol = 8/1) to obtain 586 mg of the title compound.
ESI-MS *(m/z):* 868.5$[M+H]^+$.

Step 5: Synthesis of (4-((S)-2-((S)-2-(4-(1- (26-azido-3,6,9,12,15,18,21,24-octaoxahexacosanyl) piperidin-4-yl) butyramido)-3-methylbutyramido) propionamido) benzyl) (4-nitrophenyl) carbonate

At room temperature, (S)-2-(4-(1-(26-azido-3,6,9,12,15,18,21,24-octaoxahexacosanyl) piperidin-4-yl) butyramido)-N-((S)-1-((4-(hydroxymethyl) phenyl) amino)-1-oxoprop-2-yl)-3-methylbutyramide (585 mg, 0.64 mmol) was dissolved in dichloromethane (30 mL), N,N-Diisopropyl ethylamine (334.31 mg, 2.56 mmol) was added, followed by an addition of dichloromethane (30 mL) solution of di(p-nitrobenzene) carbonate (602.35 mg, 1.92 mmol) in a dropwise manner, the resulting mixture was allowed to react at 25°C for 6h. The reaction solution was concentrated under reduced pressure, methyl *tert*-butyl ether was added into the residue, the mixture was subjected to filtration to obtain 760 mg of the title compound.
ESI-MS (*m/z*):1033.4$[M+H]^+$.

Step 6: Synthesis of (4-((S)-2-((S)-2-(4-(1- (26-azido-3,6,9,12,15,18,21,24-octaoxahexacosanyl) piperidin-4-yl) butyramido)-3-methylbutyramido) propionamido) benzyl) (N-(2-((S)-4-ethyl-4-hydroxy-3,14-dioxo-3,4,12,14-tetrahydro-1H-pyrano[3',4':6,7] indozino[1,2-b] quinolin-11-yl) ethyl)-N-isopropyl) carbamate

At room temperature, (4-((S)-2-((S)-2-(4-(1-(26-azido-3,6,9,12,15,18,21,24-octaoxahexacosanyl) piperidin-4-yl) butyramido)-3-methylbutyramido) propionamido) benzyl) (4-nitrophenyl) carbonate (150 mg, 0.12 mmol) was dissolved in N,N-dimethylformamide (3 mL), 1-hydroxybenzotriazole (34.03 mg, 0.25 mmol) and N,N-Diisopropyl ethylamine (48.82 mg, 0.37 mmol) were added, followed by an addition of (S)-4-ethyl-4-hydroxy-11-(2-(isopropylamino) ethyl)-1,12-dihydro-14H-pyrano[3', 4': 6,7] indozino[1,2-b] quinolin-3,14 (4H)-dione hydrochloride (59.79 mg, 0.12 mmol), the reaction solution was stirred at room temperature overnight, and then purified by preparative HPLC, the collected fractions were subjected to lyophilization to obtain 64 mg of the title compound.
ESI-MS (*m/z*):1327.6$[M+H]^+$.

Step 7: Synthesis of (4-((S)-2-((S)-3-methyl-2-(4-(1-(26-(4-((6-(2- (methylsulfonyl) pyrimidin-5-yl) hex-5-ynamido) methyl)-1H-1,2,3-triazol-1-yl)-3,6,9,12,15,18,21,24-octaoxahexacosanyl)piperidin-4-yl) butyramido) butyramido) propionamido) benzyl)(2-((S)-4-ethyl-4-hydroxy-3,14-dioxo-3,4,12,14-tetrahydro-1H-pyrano[3',4':6,7] indozino[1,2-b] quinolin-11-yl) N-ethyl-N-isopropylcarbamate trifluoroacetate

At room temperature, (4-((S)-2-((S)-2-(4-(1-(26-azido-3,6,9,12,15,18,21,24-octaoxahexacosanyl) piperidin-4-yl) butyramido)-3-methylbutyramido) propionamido) benzyl) (N-(2-((S)-4-ethyl-4-hydroxy-3,14-dioxo-3,4,12,14-tetrahydro-1H-pyrano[3',4':6,7] indozino[1,2-b] quinolin-11-yl) ethyl)-N-isopropyl) carbamate (64 mg, 0.046 mmol) and 6-(2-(methylsulfonyl) pyrimidin-5-yl)-N-(prop-2-ynyl) hexamide (21.63 mg, 0.069 mmol) were dissolved in dimethyl sulfoxide (4 mL) and water (1 mL), cuprous bromide (13.27 mg, 0.092 mmol) was added, the resulting mixture was allowed to react at 25°C for 1h. The reaction solution was purified by preparative HPLC, the collected fractions were subjected to lyophilization to obtain 35 mg of the title compound. ESI-MS (*m/z*): 1632.8$[M+H]^+$.
Chromatographic column: Waters SunFire Prep C18 OBD 5 μm 19×150 mm
Mobile phase A: acetonitrile;
Mobile phase B: water (0.05% trifluoroacetic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

Example 6. Preparation of monoclonal antibody targeting human Claudin 18.2

The monoclonal antibody targeting human claudin 18.2 in the invention is a humanized monoclonal antibody, including 2C6.9-hz11 and 2C6.9-hz21, and the CDRs, variable region sequence, and constant region sequence thereof are shown in Table 1:

Table 1: Sequence information

| SEQ ID NO | Description | SEQ ID NO | Description |
|---|---|---|---|
| 1 | IMGT 2C6.9 CDR-H1 | 13 | heavy chain variable region of humanized antibody 2C6.9-hz11 |
| 2 | IMGT 2C6.9 CDR-H2 | 14 | heavy chain variable region of humanized antibody 2C6.9-hz21 |
| 3 | IMGT 2C6.9 CDR-H3 | 15 | light chain variable region of humanized antibody 2C6.9-hz11/2C6.9-hz21 |
| 4 | IMGT 2C6.9 CDR-L1 | 16 | Human IgG1 heavy chain constant region |
| 5 | IMGT 2C6.9 CDR-L2 | 17 | Human κ light chain constant region |
| 6 | IMGT 2C6.9 CDR-L3 | 18 | Full length amino acid sequence of heavy chain of humanized antibody 2C6.9-hz11 |
| 7 | AbM 2C6.9 CDR-H1 | 19 | Full length amino acid sequence of heavy chain of humanized antibody 2C6.9-hz21 |
| 8 | AbM 2C6.9 CDR-H2 | 20 | Full length amino acid sequence of light chain of humanized antibody 2C6.9-hz 11/2C6.9-hz21 |
| 9 | AbM 2C6.9 CDR-H3 | 21 | IMGT 2C6.9 CDR-H2 (after modification) |
| 10 | AbM 2C6.9 CDR-L1 | 22 | AbM 2C6.9 CDR-H2 (after modification) |

(continued)

| SEQ ID NO | Description | SEQ ID NO | Description |
|---|---|---|---|
| 11 | AbM 2C6.9 CDR-L2 | 23 | heavy chain variable region of murine antibody 2C6.9M |
| 12 | AbM 2C6.9 CDR-L3 | 24 | light chain variable region of murine antibody 2C6.9M |

6.1 Construction and identification of human Claudin18.2 and human Claudin 18.1 overexpression cell lines

6.1.1 Construction of human Claudin18.2 and human Claudin18.1 overexpression cell lines

To determine the specificity and function of anti-human Claudin18.2 antibody, the complete coding sequences of human Claudin18.2 (Gene accession number: NM_001002026.2, synthesized by Nanjing Genscript Biotech Corporation) and human Claudin18.1 (Gene accession number: NM_016369.3, synthesized by Nanjing Genscript Biotech Corporation) were cloned into lentivirus vector pLVX-IRES-puro and the viruses were prepared by lentivirus packaging system according to the published method (Mohammadi Z etl., Mol Biotechnol. 2015 Sep;57(9):793-800.). The viruses obtained were used to infect HEK293T, L929, KATOIII and NCI-N87 cells. Monoclonal stable cell lines of HEK293T-Claudin 18.1, HEK293T-Claudin 18.2, L929-Claudin 18.2, KATOIII- Claudin 18.2, and NCI-N87-Claudin 18.2 were obtained by puromycin screening and single clone selection. BaF/3 cells (DSMZ, Cat# ACC300) were transfected with plasmids coding human Claudin18.2 or human Claudin18.1 using 4D-Nucleofector X transfection kit (Lonza, Cat# V4XC-3012). 48h after transfection, cells were screened by addition of 1.25 mg/mL hygromycin (Thermo Fisher Sci. Cat# 10687010). Single clones were selected after 12 days of screening, thereby obtaining monoclonal cell lines BaF/3-Claud18.1 and BaF/3-Claud18.2.

6.1.2 Detection of human Claudin18.2 and human Claudin18.1 overexpression cell lines

Western Blot was used to detect HEK293T-Claudin 18.1 (Detection Antibody: Proteintech, 66167-1-Ig) and FACS was used to detect other cell lines (Flow cytometer: Beckman, CytoFlex; Detection Antibody: IMAB362, of which the sequences are from patent: CN 101312989 B). As shown in Figures 1A-1D, FACS results demonstrated that HEK293T-Claudin 18.2, L929-Claudin 18.2, KATOIII- Claudin 18.2 and NCI-N87- Claudin 18.2 monoclonal cell lines with high positive rate (close to 100%) and good homogeneity were obtained and were used in the following experiments. Western blot results (Figure 1E) demonstrated that the three stable cell lines of HEK293T-Claudin 18.1 all overexpressed human Claudin 18.1, wherein the single clone of HEK293T-Claudin 18.1-1C2 showed higher expression level than others and was used in the following experiments.

6.2 Preparation of mouse anti-human Claudin18.2 monoclonal antibodies

DNA/cell immunization was performed in wild type mice to generate mouse anti-human Claudin 18.2 monoclonal antibodies. Each Balb/c mouse was injected with 100 $\mu$g of plasmid containing the complete coding sequence of human Claudin 18.2 through tail vein. After the fourth and sixth immunizations, serum titers were determined by FACS. Mice with high serum titers were boosted with BaF/3-Claudin18.2 overexpression cell lines 3-5 days prior to fusion. PEG mediated fusion of mouse splenocytes and mouse myeloma cell line Sp2/0 (ATCC, Cat# CRL-1581) was performed with standard fusion protocol, followed by HAT pressurized selection. FACS screening was carried out 10-14 days after fusion.

Supernatants of about 6000 hybridomas were screened using flow cytometry (available from Sartorius, as Model iQue Screener Plus) and 43 positive hybridomas binding HEK293T-Claudin18.2 cell line were obtained and subcloned. 14 hybridomas which specifically bound to human Claudin18.2 but not to human Claudin18.1 were selected by FACS using HEK293T-Claudin18.2 and HEK293T-Claudin18.1 cell lines. Single clones were obtained by limited dilution and subclone selection.

Human gastric cancer cell line NUGC4 (purchased from Japan JCRB cell bank, catalog number: JCRB0834) expresses Claudin18.2 endogenously and is widely used to evaluate the binding between antibodies and endogenous Claudin18.2 and develop functional assays. NUGC4 cells were used to evaluate the candidate clones and 7 sub-clones were finally selected. After further affinity detection, 2C6.9M was selected for variable region amplification and humanization.

In order to detect the antibody subtypes of candidate hybridoma clones, the Pierce Rapid Isotyping Kit (Thermo Fisher SCI.Cat #26179) was used to identify 2C6.9M. The identification results showed that the heavy chain was IgG1 subtype and the light chain was Kappa subtype.

Hybridoma cells were expanded and around 8000 cells were harvested and lysed. The first cDNA chain was synthesized by cDNA reverse transcription kit (Thermo Fisher Sci. Cat# 18080-200). VH and VK (VL Kappa) genes were amplified by PCR from the cDNA using primers. The PCR products were purified by DNA purification kit (Qiagen, Cat#28104) and ligated to TOPO vector (Thermo Fisher Sci. Cat#K457540). About 12 clones were picked from each ligation reaction and sequenced. The sequences were then analyzed by Vector NTI 11.5 (Thermo Fisher Sci.) and Sequencer 5.4.6 (Genecodes). The variable region sequence and CDR sequence of the obtained murine antibody 2C6.9M were shown in Table 2.

Table 2: Variable region and CDR amino acid sequence of 2C6.9 (SEQ ID No:)

| Clone No. | V H | VL | Description method | CDR-H1 | CDR-H2 | CDR-H3 | CDR-L1 | CDR-L2 | CDR-L3 |
|---|---|---|---|---|---|---|---|---|---|
| 2C6.9M | 23 | 24 | IMGT | 1 | 2 | 3 | 4 | 5 | 6 |
| | | | Abm | 7 | 8 | 9 | 10 | 11 | 12 |

6.3 Humanization of 2C6.9M murine antibody

CDR-grafting method was used to humanize the murine (mouse) antibody 2C6.9M. Briefly, the humanization process was involved in the following steps: the amino acid sequences of mouse monoclonal antibodies were aligned with the amino acid sequences of human germline antibody to identify human germline frameworks with high homology and good physical-chemical properties; the affinity to HLA-DR was determined and the human germline frameworks with low affinity to HLA-DR were then selected; the six CDR regions of mouse antibodies were then grafted to the selected heavy chain and light chain frameworks.

Specifically, the heavy chain and light chain CDRs of the mouse antibody 2C6.9M were grafted to the frameworks (FR) of the corresponding humanization templates. The humanization templates for the heavy chain and light chain of 2C6.9M are human germline sequence IGHV4-59*01 (IMGT reference number AB019438) and IGKV4-1*01 (IMGT reference number Z00023), respectively.

Moreover, with computer simulation, molecular docking was performed to analyze the variable region and its surrounding framework amino acid sequences, so as to determine the spatial stereoscopic binding mode of the antibodies. By calculating electrostatic forces, Van der Waals forces, hydrophobic interactions and entropy, critical amino acid residues which may interact with Claudin18.2 or maintain spatial structure in the amino acid sequence of the mouse antibody were identified, and these mouse amino acids were maintained after grafting. In other words, a series of back mutations were taken in the FR region residues of humanization template so that the affinities of the mouse antibodies could be kept in humanized antibodies to the maximal extent.

The variable region sequences of the mouse antibody 2C6.9M are shown in SEQ ID No: 23 and SEQ ID No: 24, and its CDR sequences are shown in SEQ ID No: 1 to SEQ ID No: 12. In order to avoid the occurrence of isomerization without affecting the affinity, the amino acid sequence of 2C6.9 CDR-H2 was modified in the invention. The modified sequences are shown in SEQ ID No: 21 and SEQ ID No: 22. Finally, 2 humanized antibodies were constructed, and named as 2C6.9-hz11 and 2C6.9-hz21 respectively. The heavy chain constant region of each antibody is human wild-type IgG1 heavy chain constant region (SEQ ID No: 16), and the light chain constant region of the antibody is human wild-type IgG1 κ Light chain constant region (SEQ ID No: 17).

The variable region, constant region, and heavy/light chain amino acid sequence of each 2C6.9 antibody are shown in Table 1.

The codon optimized cDNAs of the heavy chain and light chain amino acid sequences of the 2C6.9 humanized antibody were synthesized and ligated into plasmid pcDNA3.4 (by Nanjing Genscript Biotech Corporation on commission). The pcDNA3.4 corresponding to the heavy chain and light chain was simultaneously transfected into Expi293F cells (purchased from Thermo company), and the cell supernatant was purified by the protein A affinity column (MabSelect SuRe, GE) to obtain a 2C6.9 humanized monoclonal antibody.

6.4 Affinity detection of humanized monoclonal antibody 2C6.9

The affinity of 2C6.9-hz21 to human Claudin 18.2 on cell membrane was detected using HEK293T-Claudin 18.2 cells. The specific procedures were as follows: HEK293T-Claudin 18.2 cells were detached and centrifuged, followed by being washed twice with PBS. And then the cells were resuspended in PBS containing 1% BSA and were plated into a 96-well tip bottom plate at 300000 cells per well in a volume of 50μl for 20 wells in total. 50μl of 2C6.9-hz21 and

IMAB362 antibody were separately then added with 11 concentrations starting from 1000nM with three-fold dilution to each well. Human IgG was served as negative control. The reactions were mixed well and incubated for 1h at 4°C in the dark, followed by three times of washing with PBS, the FITC-labelled anti-human Fc secondary antibody (Biolegend, 409322) was added and incubated for 0.5h at 4°C in the dark. Detection was performed by flow cytometry (Beckman, Cytoflex) after being washed for three times with PBS.

As shown in Figure 2 and Table 3, the EC50 value of the binding affinity of 2C6.9-hz21 to HEK293T-Claudin 18.2 was lower than that of IMAB362; and the max fluorescence value of 2C6.9-hz21 was higher than that of IMAB362, indicating that the affinity of 2C6.9-hz21 to Claudin 18.2 on cell membrane was stronger than that of IMAB362.

Table 3: Measurement of affinity for humanized antibody 2C6.9-hz21 to HEK293T-Claudin 18.2 by flow cytometry

| Antibody name | EC50 (nM) | Max MFI |
|---|---|---|
| 2C6.9-hz21 | 5.767 | 165866.3 |
| IMAB362 | 8.217 | 156715.7 |

6.5 Determination of the affinity and specificity of humanized antibody 2C6.9

Human Claudin18.2 belongs to the tetraspanin family and has a complex structure. Cell ELISA was thus carried out to maintain the structure of Claudin18.2. The stable cell lines L929-Claudin 18.2 constructed in 6.1 were used for detection. Specifically, L929-Claudin 18.2 adherent cells were detached by 2mM EDTA treatment. The cells were resuspended and adjusted to $2 \times 10^5$/mL and 100 μL of the resuspension was plated into a 96-well plate and incubated overnight at 37°C. The next day, the media was removed, and the plate was washed with PBS once. 100 μL/well 4% formaldehyde was added to the plate. After 30min of incubation at room temperature, formaldehyde was removed, followed by being washed twice with PBS. Blocking buffer (PBS containing 2% BSA) was then added to the plate at 100 μL/well, and incubated at 37°C for 2h. After removal of blocking buffer, 100 μL/well of serially diluted antibodies (starting from 1 μM, with 4-fold dilution, for a total of 11 concentrations) were added to the corresponding wells, which were incubated at 37°C for 2h afterwards. The plate was washed with 250 μL PBST for 5 times, and was allowed to stand for 2min each time. 100 μL/well of horseradish peroxidase labeled anti-Human IgG secondary antibody (HRP-anti-Human IgG, Jackson ImmunoResearch, 109-035-003) diluted 1:10000 in PBS (containing 2% BSA) was added to the plate. The plate was incubated at 37°C for 1h and washed with 250 μL of PBST 6 times, wherein the plate was allowed to stand for 2min each time. 100 μL/well of TMB solution (Thermo, 34029) was added. The reactions were incubated at 37°C for 20min and stopped with 50 μL 2 mol/L $H_2SO_4$. The OD450 nm absorbance values were obtained on a plate reader (MD, SpectraMax M2) and the results were subjected to curve fitting by Graphpad Prism.

The results are shown in Figure 3 and Table 4, indicating that the affinity of humanized antibody 2C6.9-hz21 to human Claudin 18.2 is significantly higher than that of IMAB362. In the same experiment, the affinity of humanized antibody 2C6.9-hz11 was close to that of antibody 2C6.9-hz21 (the result was omitted).

Table 4: Detection of the afinity of humanized antibody 2C6.9-hz21 binding to L929-Claudin 18.2 cells using Cellular ELISA

| Antibody name | EC50(nM) | Max signal value (OD450) |
|---|---|---|
| 2C6.9-hz21 | 0.12 | 0.92 |
| IMAB362 | 0.15 | 0.53 |

The specificity of candidate antibodies was detected by FACS. The specific procedures were as follows: HEK293T, HEK293T-human Claudin 18.1 and HEK293T-human Claudin18.2 cells were detached and then centrifuged. After being washed twice with PBS, the cells were resuspended with PBS containing 1 % BSA. 300000 cells for each cell line were added with the candidate antibodies at a final concentration of 1000nM, mixed well, and incubated for 1h at 4°C, protected from light. Then the cells were washed for three times with PBS and FITC-labeled anti-human Fc secondary antibody (BioLegend, 409322) was added, followed by 0.5h of incubation at 4°C in the dark. Detection was performed by flow cytometry (Beckman, Cytoflex) after being washed with PBS for three times.

As shown in Figure 4, 2C6.9-hz21 can specifically bind to human Claudin 18.2 but not to human Claudin 18.1.

6.6 Determination of complement dependent cytotoxicity (CDC) of humanized antibody 2C6.9

2C6.9 belongs to IgG1 subtype, which can activate classical complement pathway effectively, and induce

complement dependent cytotoxicity (CDC). Guinea pig serum (purchased from Zhengzhou Baiji, catalog number S0001) which is rich in complements was used in our research in order to determine the CDC activity of 2C6.9. The specific procedures are as follows: HEK293T-Claudin 18.2 cells were harvested and centrifuged, followed by adjustment of cell density. $5\times10^4$/well of cells were plated on a plate and incubated overnight. DMEM containing 20% guinea pig serum was prepared the next day and was used to dilute 2C6.9 and IMAB362. Starting from 20 μg/mL, 10 concentrations were prepared with two-fold dilution. The original cell culture media for HEK293T-Claudin 18.2 cells was removed, and the antibody dilutions were added to the corresponding wells, 100 μL/well. 10 μL/well lysis buffer was provided as the positive control. The reactions were left to stand in incubator at 37°C, 5% $CO_2$ and incubated for 3h. Subsequently, CellTiter-Glo Luminescent (CTG, Purchased from Promega, Item No.: G7573) was added at 50 μl/well for staining followed by a 30-second mixing and left to stand for 1 min at room temperature. The fluorescence signal value was then determined by a microplate reader (MD, SpectraMax M2), the results of which were imported into Graphpad Prism for curve fitting.

As shown in Figure 5 and Table 6, the CDC activity of 2C6.9-hz21 was stronger than that of the control antibody IMAB362.

Table 6: CDC activity determination of anti-Claudin18.2 humanized antibody 2C6.9-hz21

| Antibody name | EC50 value (ng/mL) |
|---|---|
| 2C6.9-hz21 | 1363 |
| IMAB362 | 3317 |

6.7 Determination of the antibody-dependent cell-mediated cytotoxicity (ADCC) of humanized antibody 2C6.9

2C6.9 belongs to IgG1 subtype and has relative strong antibody-dependent cell-mediated cytotoxicity (ADCC). NK cell mediated killing assay was used to determine the ADCC activity of 2C6.9. The specific procedures were as follows: HEK293T-Claudin 18.2 cells were harvested and centrifuged, followed by adjustment of cell density. $1\times10^4$/well of cells were plated on a plate and incubated overnight. The medium was removed on the next day. NK92MI-CD16a cells (Huabo Biopharm) were centrifuged, resuspended in MEMA medium and adjusted to $1\times10^6$/mL, then 50 μL/well cells were added to the corresponding wells. 2C6.9-hz21 and IMAB362 antibodies were diluted with MEMA medium. For HEK293T-Claudin18.2 cells, 10 antibody concentrations were tested, starting from 40 μg/mL with 5-fold dilution. For NUGC-4 cells, 11 antibody concentrations were tested, starting from 2 mg/mL with 5-fold dilution. 50 μL/well of the diluted antibodies were added to the corresponding wells and the reactions were left to stand in incubator at 37°C, 5% $CO_2$ and incubated for 5.5h. Lysis buffer was then added to the positive control well and incubated for another 0.5h. 50 μL/well of Lactate Dehydrogenase (LDH) detection agent (DOJINDO LABORATORISE, CK12) were added to the wells and the absorbance at 490nm were taken every 10min on a microplate reader (MD, SpectraMax M2). The results were imported into Graphpad Prism for curve fitting.

As shown in Figure 6 and Table 7, the ADCC activity of 2C6.9-hz21 on HEK293T-Claudin 18.2 is stronger than that of IMAB362.

Table 7: ADCC activity determination of anti-human Claudin18.2 humanized antibody 2C6.9-hz21

| Antibody name | EC50 value (ng/mL) | Max. cytotoxicity (%) |
|---|---|---|
| 2C6.9-hz21 | 67.67 | 30 |
| IMAB362 | 57.66 | 20 |

Example 7. Preparation of ADC targeting Claudin 18.2 (2C6.9-ADC)

2C6.9-TL001 is prepared by conjugating of TL001 obtained in Example 3 with humanized monoclonal antibody 2C6.9. The preparation method is as follows:

(1) Conjugation: to 30 mg of 2C6.9-hz21 antibody was added 20 mM PB+105 mM NaCl+100 mM disodium edetate solution (pH 7.7), pH was adjusted to 7.7 with 2M Tris solution, the mixture was diluted with 20 mM PB+105 mM NaCl pH7.7 solution (the final concentration of disodium edetate was 5 mM, and the final concentration of the antibody was 15 mg/mL), mixed evenly, 10 mM TCEP solution was added and mixed well, the resulting mixture was allowed to stand at room temperature for a while (30 or 90 minutes); followed by an addition of TL001 dissolved in dimethyl sulfoxide (molar ratio to antibody: 5:1 or 9:1), mixed well, and stood at room temperature for 2 hours to obtain the conjugated

sample, which was named as 2C6.9-TL001.

(2) Buffer replacement: 30 KDa 50-ml ultrafiltration tube (Millipore) was used for buffer exchange of 2C6.9-TL001, the replacement solution was 10 mM histidine-hydrochloride histidine + 8% sucrose (pH 6.0) buffer, with a replacement multiple of 15; the sample was collected and added with 10% Tween-20, wherein the final concentration of Tween-20 in the sample was 0.02% (M/V).

(3) Test:

After the replacement, 2C6.9-TL001 was subjected to LC-MS molecular weight analysis under the following conditions:

Chromatographic determination conditions:

Liquid chromatographic column: Thermo MAbPac RP 3.0*100 mm;

Mobile phase A: 0.1%FA/98%$H_2O$/2%ACN;

Mobile phase B: 0.1%FA/2%$H_2O$/98%ACN;

Flow rate: 0.25 mL/min;

Temperature of sample chamber: 8°C; Column temperature: 60°C; Sample size: 1 μL;

| Time (min) | 2 | 20 | 22 | 25 | 26 | 30 |
|---|---|---|---|---|---|---|
| Mobile phase A (%) | 75 | 60 | 5 | 5 | 75 | 75 |
| Mobile phase B (%) | 25 | 40 | 95 | 95 | 25 | 25 |

Switching valve: 0-3 min to waste, 3-22 min to MS, 22-30 min to waste

Mass spectrometry conditions:

Mass spectrum model: AB Sciex Triple TOF 5600+;

Parameters: GS1 35; GS2 35; CUR 30; TEM 350; ISVF 5500; DP 200; CE 10; m/z 600-4000; Time bins to sum 40.

The theoretical molecular weight and measured molecular weight of the light chain and heavy chain of 2C6.9-TL001 obtained by conjugation of TL001 and 2C6.9-hz21 (the heavy chain is calculated by the main glycoform G0F) are shown in the table below:

| Peptide chain | | mAb | DAR1 | DAR2 | DAR3 | DAR4 |
|---|---|---|---|---|---|---|
| LC | Theoretical value (Da) | 24090.8 | 25641.6 | 27192.3 | 28743.1 | 30293.8 |
| | Measured value (Da) | 24091.6 | 25642.1 | Not detected | Not detected | Not detected |
| HC | Theoretical value (Da) | 49955.2 | 51505.9 | 53056.7 | 54607.4 | 56158.2 |
| | Measured value (Da) | 49957.5 | 51507.9 | 53058.5 | 54609.2 | 56157.9 |

When the conjugation feed ratio between TL001 and 2C6.9-hz21 is 5: 1, the light chain (LC) of antibody 2C6.9-TL001 is conjugated with 0 to 1 toxin (the proportions of LC and DAR1 are 57.8% and 42.2% respectively), and the heavy chain (HC) is conjugated with 0 to 4 toxins (the proportions of HC, DAR1, DAR2, DAR3, and DAR4 are 22.5%, 30.3%, 25.0%, 21.9%, and 0.3% respectively). Therefore, the toxin-to-antibody ratio (DAR) is 3.79, and the calculation formula is: DAR=light chain DAR1*2+heavy chain (DAR1*1+DAR2*2+DAR3*3+DAR4*4)*2.

When the conjugation feed ratio between TL001 and 2C6.9-hz21 is 9:1, the antibody light chain (LC) of 2C6.9-TL001 is conjugated with 0 to 1 toxin (the proportions of LC and DAR1 are 7.5% and 92.5% respectively), and the heavy chain (HC) is conjugated with 0 to 4 toxins (the proportions of HC, DAR1, DAR2, DAR3, and DAR4 are 2.5%, 10.2%, 9.8%, 76.4%, and 1.1% respectively). Therefore, the toxin-to-antibody ratio (DAR) is 7.12.

When the conjugation feed ratio between TL001 and 2C6.9-hz21 is 9:1, after conjugation and cation chromatography, the light chain of 2C6.9-TL001 is conjugated with 0-1 toxin (LC and DAR1 ratios were 1.5% and 24.0%, respectively), and the heavy chain is conjugated with 0-4 toxins (HC, DAR1, DAR2, DAR3, and DAR4 ratios were 0.7%, 2.1%, 14.3%, 54.5%, and 2.8%, respectively). The resulting drug-to-antibody ratio (DAR) was calculated as 7.40.

The ADC molecular structure of 2C6.9-TL001 is given below:

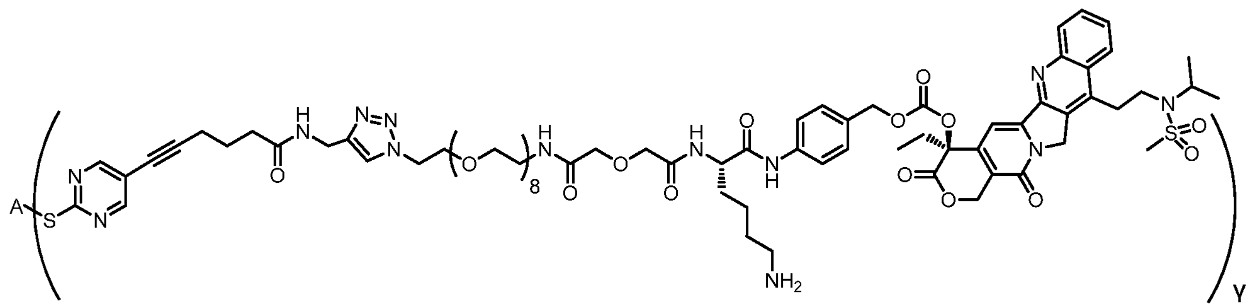

wherein γ is an integer from 1 to 10, and A is 2C6.9-hz21.

The conjugates were subjected to SEC detection by SEC-HPLC.

Chromatographic conditions:

Liquid chromatographic column: TSKgel G3000SWxl, 300*7.8 mm, 5 μm;

Mobile phase: 90 mmol/L Na2HPO4, 30 mmol/L NaH2PO4, 200 mM NaCl, 5% acetonitrile;

Flow rate: 0.8 mL/min; detection wavelength: 280 nm; column temperature: room temperature; temperature of sample chamber: 8°C;

Sample size: 40 μL; isocratic operation: 30 min.

The SEC chromatograms of 2C6.9-TL001 (DAR: 3.79) and 2C6.9-TL001 (DAR: 7.12) are shown in Figures 7-8, the SEC chromatogram of 2C6.9-TL001 (DAR:7.40) is shown in Figure 13. According to the retention time and peak area ratio of the SEC, it is confirmed that the molecular weight of the main conjugating products is about 150 kd, i.e., 2C6.9-TL001 obtained by the conjugation of TL001 and 2C6.9-hz21 still maintains the entire structure of the antibody.

The same preparation method was used to conjugate the TL002 and TL003 prepared in Examples 4 and 5 with the humanized monoclonal antibody 2C6.9 to prepare 2C6.9-TL002 and 2C6.9-TL003, the preparation and detection method are as described above. The DAR values of the obtained 2C6.9-TL002 and 2C6.9-TL003 are 6.95 and 7.03 respectively.

The ADC molecular structure of 2C6.9-TL002 is given below:

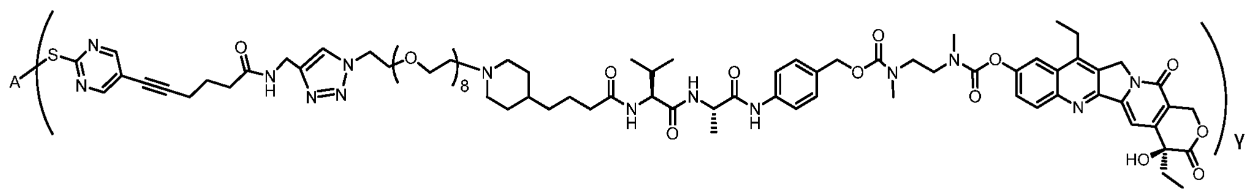

wherein γ is an integer from 1 to 10, and A is 2C6.9-hz21.

The molecular structure of 2C6.9-TL003 is given below:

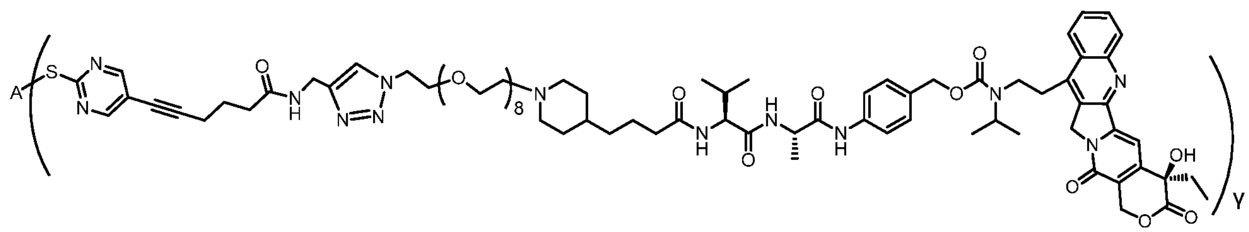

wherein γ is an integer from 1 to 10, and A is 2C6.9 antibody.

Example 8. Determination of the affinity of 2C6.9-TL001

A cell-based ELISA method was carried out to determine the affinity of 2C6.9-TL001 (DAR: 7.12) to Claudin

18.2 on the surface of cell membrane. The specific procedures were as follows: L929-Claudin 18.2 adherent cell lines were detached by 2 mM EDTA, resuspended to 2*10^5 cells/mL, and 100μL of the resuspension was placed into a 96-well plate and incubated at 37°C overnight; the next day, the medium was removed, and the plate was washed with PBS once. 100 μL/well 4% formaldehyde was added to the plate at room temperature for 30 min; formaldehyde was then removed, followed by being washed twice with PBS, and 100 μL PBS (containing 2% BSA) was added and incubated for 2h; the blocking solution was removed, the 2C6.9-TL001 to be tested was diluted with PBS (containing 2% BSA), starting from 9.375 μg/mL with 4-fold dilution, for a total of 9 concentrations, and then added to the plate at 100 μL/well, and incubated at 37°C for 2h; the plate was washed with 250 μL PBST for 5 times, and was allowed to stand for 2 min each time; PBS (2% BSA) was used to dilute horseradish peroxidase (HRP)-labeled anti-human IgG second antibody (HRP-anti-Human IgG, Jackson immunoresearch) at the ratio of 1:10000, the dilutions were added into a plate by 100 μL/well and incubated at 37°C for 1h; after being washed with 250 μL PBST for 6 times, the cells was allowed to stand for 2 min each time; 100 μL TMB chromogenic solution (Thermo) was added into the corresponding well to develop 20 min at 37°C; 50 μL 2 mol/L $H_2SO_4$ was added for termination, and OD450 nm absorbance values were obtained by a microplate reader (MD), and the values were imported into Graphpad Prism for curve fitting. The experimental results are shown in Figure 9, and the $EC_{50}$ value of the affinity of 2C6.9-TL001 (DAR: 7.12) to Claudin 18.2 on the cell membrane surface was 39.94 ng/mL. The antibody 2C6.9-hz21, after being conjugated with TL001, still has excellent Claudin 18.2 affinity.

Example 9. Detection of killing activity of 2C6.9-ADC on Claudin18.2 high-expression tumor cell lines

The killing activity of 2C6.9-TL001 on Claudin 18.2 high-expression cell lines was detected using HEK293T-Claudin 18.2 and HEK293T-Claudin 18.1 cells. The specific experimental steps were as follows: the day before the experiment, the cells were diluted with DMEM+10% FBS, the suspension was placed into the plate by 100 μL/well and 1*10^4 cells/well to stand overnight; the next day, ADC molecules were 4-fold diluted with DMEM basic medium, starting from 150 μg/mL (DAR:7.12) or 262.5 μg/mL (DAR:3.79) for a total of 11 concentrations, the dilutions were added into corresponding wells, 100 μL each well, until the final serum concentration was 5%; the cells were incubated at 37°C in a 5% $CO_2$ incubator for 48h; CCK8 (Rhinogen) was then added (20 μL/well), the cells were placed into a 5% $CO_2$ incubator for 0.5-2.5h at 37°C, the OD450 nm absorbance values were obtained by a microplate reader (MD) every half an hour, and the values were imported into Graphpad Prism for curve fitting.

As shown in Figures 10A, 10B and 10C, 2C6.9-TL001 can effectively kill HEK293T-Claudin 18.2 cells. When the DAR value is 7.12, its $EC_{50}$ value is 473 ng/mL; when the DAR value is 3.79, its $EC_{50}$ value is 794.1 ng/mL. In the meantime, the $EC_{50}$ of 2C6.9-TL001 (DAR: 7.12) for killing HEK293T-Claudin18 is 3900 ng/mL, and the killing activity of 2C6.9-TL001 (DAR: 7.12) on Claudin 18.2 cells is significantly stronger than that of Claudin 18.1 cells (about 8 times the difference), indicating that the killing effect of 2C6.9-TL001 is specific to Claudin 18.2.

The killing activities of 2C6.9-TL002 and 2C6.9-TL003 on HEK293T-Claudin 18.2 and HEK293T-Claudin 18.1 cells were detected by the same experimental method described above. The experimental results in Figures 10D and 10E show that, 2C6.9-TL002 and 2C6.9-TL003 can effectively kill HEK293T-Claudin 18.2 cells, and $EC_{50}$ values were 628.9 ng/mL and 540.2 ng/mL respectively; the $EC_{50}$ values of 2C6.9-TL002 and 2C6.9-TL003 for killing HEK293T-Claudin18.1 were 30590 ng/mL and 8258 ng/mL respectively. The killing activity of 2C6.9-TL002 and 2C6.9-TL003 on HEK293T-Claudin 18.2 cells was significantly stronger than that on Claudin 18.1, indicating that the killing effect was Claudin 18.2 specific.

Example 10. Detection of killing activity of 2C6.9-ADC on endogenously expressed Claudin 18.2 cell lines

Gastric cancer cell line NUGC-4 was selected to detect the killing activity of 2C6.9-TL001 on endogenously expressed Claudin 18.2 cells. Specific experimental steps were as follows: the day before the experiment, the cells were diluted with RPMI 1640+10% FBS, the suspension was placed into a plate by 100 μL/well and 1*10^4 cells/well to stand overnight; the next day, 2C6.9-TL001 was 3-fold diluted with RPMI 1640 medium, starting from 1000 μg/mL (DAR 7.12) or 1750 μg/mL (DAR: 3.79) for a total of 11 concentrations, the dilutions were added into corresponding wells, 100 μL per well, and the final serum concentration reached 5%; the cells were inbubated at 37°C in a 5% $CO_2$ incubator for 72h; CCK8 (Rhinogen) was then added 20 μL/well, the cells were incubated at 37°C in a 5% $CO_2$ incubator for 0.5-2.5h, the OD450 nm absorbance values were obtained by a microplate reader (MD) every half an hour, the values were imported into Graphpad Prism for curve fitting. The experimental results were shown in Figures 11A and 11B, 2C6.9-TL001 can effectively kill NUGC-4 cells: when the DAR value is 7.12, its $EC_{50}$ value is 2.383 μg/mL; when the DAR value is 3.79, its $EC_{50}$ value is 10.01 μg/mL.

The killing activities of 2C6.9-TL002 and 2C6.9-TL003 on NUGC-4 cells were detected by a similar experimental method to the method as described above. The initial concentration of ADC is 500 μg/mL with 4-fold dilution for a total of 11 concentrations. As shown in Figure 11C, 2C6.9-TL002 and 2C6.9-TL003 can effectively kill NUGC-4 cells, and

the $EC_{50}$ values are respectively 54.92 μg/mL and 123.94 μg/mL.

Example 11. Detection of 2C6.9-hz21 antibody internalization

NUGC-4 was selected to detect the internalization activity of antibody 2C6.9. Specific experimental steps were as follows: NUGC-4 cells were digested with trypsin and counted, resuspended in PBS (containing 1% BSA), the cell density was adjusted to $3 \times 10^6$/mL; 2C6.9-hz21 antibody with a final concentration of 100 μg/mL was added to 100 μL of the resuspended cells, the isotypic human IgG was set as negative control, and incubated on ice for 1h; after the incubation, the cells were washed with precooled PBS three times, resuspended with NUGC-4 cell culture medium (1640+10% FBS), and divided into two parts: one part was incubated at 37°C in a cell incubator for 4h (endocytosis group), and the other part was incubated on ice for 4h (affinity group); after the incubation, the cells were washed with precooled PBS three times, resuspended in 50 uL PBS (containing 1% BSA), anti-human fluorescent secondary antibody (Biolegend) was added, and incubated at 4°C for half an hour; after the incubation, the cells were washed with precooled PBS three times, detection was perfomed by flow cytometry (Beckman), the antibody internalization ratio was calculated according to the formula: endocytosis (%)=[1-($MFI_{37°C\ antibody\ group}$- $MFI_{37°C\ control\ group}$)/($MFI_{antibody\ group\ on\ ice}$ - $MFI_{control\ group\ on\ ice}$)] ×100%. The experimental results showed that the 4-hour internalization ratio of 2C6.9-hz21 on NUGC-4 cells was 37.79%, indicating that 2C6.9-hz21 conjugates have the potential to internalize the drugs into cells and kill tumor cells.

Example 12. Detection *of in vivo* efficacy of 2C6.9-ADC

A cancer cell line-derived xenograft (CDX) model and a patient-derived xenograft (PDX) model are used to evaluate the antitumor effect of the ADC molecules.

12.1 NCI-N87-Claudin18.2 + Balb/c Nude mice CDX model

NCI-N87-Claudin18.2 cells were cultured in RPMI1640 medium containing 10% fetal bovine serum at 37°C and 5% $CO_2$. The cells in exponential growth phase were collected and resuspended in PBS, and then inoculated subcutaneously into female Balb/c Nude mice (Beijing Vital River Laboratory Animal Technology Co., Ltd.) at an amount of $5\times10^6$ cells per mouse (suspended in 0.1 mL PBS), to establish a subcutaneous transplantation tumor model. When the average tumor volume reached 70 to 100 $mm^3$, the mice were randomly grouped according to the tumor volume, 7 mice/group. The day of grouping was recorded as day 0, and the groups were Human IgG1 isotype control antibody (negative control) group (IgG1 for short), 2C6.9-TL001 (DAR: 7.12) 1 mg / kg group and 3 mg/kg group, and 2C6.9-TL002 (DAR: 6.95) 1 mg/kg group and 3 mg/ kg group. All samples were injected via the tail vein twice a week for a total of 6 times.

After administration, the tumor diameter was measured with a vernier caliper twice a week, and the tumor volume was calculated according to the following formula: $V = 0.5\ a \times b^2$, wherein a and b represent the long diameter and short diameter of the tumor, respectively. Deaths were observed and recorded every day.

The tumor growth inhibition rate TGI (%) was calculated by the following formula to evaluate the antitumor effect:

$$TGI\ (\%) = [1-(V_{Tend}-V_{Tstart})/(V_{Cend}-V_{Cstart})]*100\%$$

wherein

$V_{Tend}$: mean tumor volume at the end of the experiment in the treatment group
$V_{Tstart}$: mean tumor volume at the start of the administration in the treatment group
$V_{Cend}$: mean tumor volume at the end of the experiment in the negative control group
$V_{Cstart}$: mean tumor volume at the start of the administration in the negative control group

The relative tumor proliferation rate T/C (%) was calculated by the following formula to evaluate the antitumor effect:

$$T/C(\%)(\text{tumor volume})=(T_t/T_0)/(C_t/C_0)\times100\%$$

wherein

$T_0$: average tumor volume of the treatment group at the initial (i.e., P0)
$T_t$: average tumor volume of the treatment group at each measurement
$C_0$: average tumor volume of negative control group at initial (i.e., P0)
$C_t$: average tumor volume of negative control group at each measurement.

The experimental results were shown in Table 8 and Figures 12A and 12B, 2C6.9-TL001 (DAR: 7.12) showed significant inhibitory effect on the tumor growth of the NCI-N87-Claudin18.2 gastric cancer transplanted tumor model in a dose-dependent manner. Compared with the negative control group, after 6 doses (day 21), the tumor inhibition rate (TGI) of 2C6.9-TL001 1-mg/kg group was up to 96.03%, and 4 mice had partial tumor regression; while in the 3-mg/kg group, the TGI reached 133.50%, and 3 mice had partial tumor regression and 4 mice had complete tumor regression. The TGI of the 2C6.9-TL002 3-mg/kg group was 40.11%, and 1-mg/kg group had no obvious antitumor effect. There was no significant weight loss seen in all treatment groups during the observation period, and the animals were well tolerated.

Table 8: NCI-N87-Claudin18.2 + Balb/c Nude mice CDX model

| Group | Dosage (mg/kg) | Day 21 | | | | |
|---|---|---|---|---|---|---|
| | | Tumor volume ($mm^3$) ($\bar{x}\pm$SEM) | TGI (%) | T/C (%) | Tumor regression (PR/CR) | P value |
| IgG1 | 3 | 479.07±28.15 | -- | -- | 0/0 | -- |
| 2C6.9-TL001 | 1 | 143.91±28.23 | 96.03 | 29.96 | 4/0 | <0.0001 |
| 2C6.9-TL001 | 3 | 12.96±4.62 | 133.50 | 2.70 | 3/4 | <0.0001 |
| 2C6.9-TL002 | 1 | 461.02±36.32 | 5.28 | 95.94 | 0/0 | ns |
| 2C6.9-TL002 | 3 | 339.28±43.91 | 40.11 | 70.62 | 1/0 | <0.0001 |

Note: TGI: tumor growth inhibition rate; T/C: relative tumor proliferation rate; PR: partial regression of tumors; CR: complete regression of tumors regress; P value is the significant difference result of comparison with IgG1 group; N/A: not applicable; ns: p>0.05, no statistical differences.

12.2 *In vivo* efficacy comparison of 2C6.9-TL001 and 2C6.9 monoclonal antibody + chemotherapy in CDX model

NCI - N8 7 -Claudin 18.2 subcutaneous transplanted tumor models were established according to Example 12.1 and grouped. The day of grouping was recorded as day 0. The groups were respectively human IgG1 isotype control antibody (negative control) group (IgG1 for short), paclitaxel group (albumin binding type), 2C6.9 mAb combined with paclitaxel group, and 2C6.9-TL001 (DAR: 7.12) group. All samples were injected via tail veins twice a week for a total of 3 weeks. The dosage is shown in Table 9.

The experimental results are shown in Table 9 and Figure 12C. Compared with the negative control group, all of the three administration groups can significantly inhibit tumor growth after 11 days of drug administration, especially the 2C6.9-TL001 (DAR: 7.12) group. The tumor inhibition rate (TGI) of 2C6.9-TL001 (DAR: 7.12) group is up to 121.68%, and 6 out of 7 mice have partial tumor regression.

Table 9: *In vivo* efficacy comparison of 2C6.9-TL001 and 2C6.9 monoclonal antibody + chemotherapy in CDX model

| Group | Dosage (mg/kg) | Day 11 | | | |
|---|---|---|---|---|---|
| | | Tumor volume ($mm^3$) ($\bar{x}\pm$SEM) | TGI (%) | Tumor regression (PR/CR) | P value |
| IgG1 | 3 | 314.03±34.45 | N/A | 0/0 | N/A |
| Paclitaxel | 12 | 226.21±28.26 | 42.40 | 0/0 | ns |
| 2C6.9+ paclitaxel | 10+12 | 175.03±14.30 | 67.43 | 0/0 | <0.0001 |

(continued)

| Group | Dosage (mg/kg) | Day 11 | | | |
|---|---|---|---|---|---|
| | | Tumor volume ($mm^3$) ($\bar{x}\pm$SEM) | TGI (%) | Tumor regression (PR/CR) | P value |
| 2C6.9-TL001 | 3 | 62.76±9.98 | 121.68 | 6/0 | <0.0001 |
| Note: TGI: tumor growth inhibition rate; T/C: relative tumor proliferation rate; PR: partial tumor regression; CR: complete tumor regression. P value is the significant difference result of comparison with IgG1 group; N/A: not applicable; ns: p>0.05, no statistical differences. | | | | | |

After 21 days of administration, compared with the negative control group, all of the three administration groups were able to significantly inhibit tumor growth, and the 2C6.9-TL001 (DAR: 7.12) group was the most significant. The tumor inhibition rate (TGI) of the 2C6.9-TL001 (DAR: 7.12) group was up to 125.73%, and 5 out of the 7 mice hade complete tumor regression, and 2 mice had partial tumor regression. The experimental results are shown in Table 10 and Figure 12D.

Table 10: *In vivo* efficacy comparison of 2C6.9-TL001 and 2C6.9 monoclonal antibody + chemotherapy in CDX model

| Group | Dosage (mg/kg) | Day 21 | | | |
|---|---|---|---|---|---|
| | | Tumor volume ($mm^3$) ($\bar{x}\pm$SEM) | TGI (%) | Tumor regression (PR/CR) | P value |
| IgG1 | 3 | 456.60±36.43 | N/A | 0/0 | N/A |
| Paclitaxel | 12 | 276.31±23.46 | 51.60 | 0/0 | <0.0001 |
| 2C6.9+ paclitaxel | 10+12 | 207.52±21.25 | 71.43 | 0/0 | <0.0001 |
| 2C6.9-TL001 | 3 | 17.75±3.97 | 125.73 | 2/5 | <0.0001 |
| Note: TGI: tumor growth inhibition rate; T/C: relative tumor proliferation rate; PR: partial tumor regression; CR: complete tumor regression. P value is the significant difference result of comparison with IgG1 group; N/A: not applicable. | | | | | |

12.3 Comparison *of in vivo* efficacy of 2C6.9-TL001 having different DAR values in CDX model

NUGC-4 cells were cultured with RPMI1640 culture medium containing 10% fetal bovine serum (FBS) at 37°C with 5% $CO_2$. The cells in the exponential growth phase were collected, resuspended in PBS, and inoculated subcutaneously in female Balb/c Nude mice (Beijing Vital River Laboratory Animal Technology Co., Ltd.) at the cell volume of $5\times10^6$/mouse (suspended in 0.1 mL PBS) to establish a subcutaneous transplantation tumor model. When the average tumor volume reached about 70-100 $mm^3$, the mice were randomly grouped according to the tumor volume, 7 mice for each group. The day of grouping was recorded as day 0, and the groups were respectively human IgG1 isotype control antibody (negative control) group (IgG1 for short), 2C6.9-TL001 (DAR: 3.79) 5.25 mg/kg group, 2C6.9-TL001 (DAR: 3.79) 17.5 mg/kg group, 2C6.9-TL001 (DAR: 7.12) 3 mg/kg group, and 2C6.9-TL001 (DAR: 7.12) 10 mg/kg group (ADC drugs with different DAR values were administered at the designed dosage with the same toxin loading). All samples were injected via tail veins twice a week for a total of 3 weeks. The dosages were shown in Table 11.

The experimental results are shown in Table 11 and Figures 12E and 12F. Under the same toxin loading, the efficacy of 2C6.9-TL001 with a high DAR value (7.12) is basically equivalent to that with a low DAR value (3.79) after 21 days of drug administration. There is no significant weight loss seen in all treatment groups during the observation period, and the animals were well tolerated.

Table 11: Comparison *of in vivo* efficacy of 2C6.9-TL001 having different DAR values in CDX model

| Group | Dosage (mg/kg) | Day 21 | | |
|---|---|---|---|---|
| | | Tumor volume ($mm^3$) ($\bar{x}\pm$SEM) | TGI (%) | P value |
| IgG1 | 17.5 | 1227.10±168.01 | N/A | N/A |
| 2C6.9-TL001(DAR:3.79) | 5.25 | 736.12±92.59 | 43.76 | <0.0001 |
| 2C6.9-TL001(DAR:3.79) | 17.5 | 477.42±67.90 | 66.94 | <0.0001 |
| 2C6.9-TL001(DAR:7.12) | 3 | 670.96±113.92 | 49.60 | <0.0001 |

(continued)

| Group | Dosage (mg/kg) | Day 21 | | |
|---|---|---|---|---|
| | | Tumor volume (mm$^3$) ($\bar{x}\pm$SEM) | TGI (%) | P value |
| 2C6.9-TL001(DAR:7.12) | 10 | 250.04±28.73 | 87.17 | <0.0001 |
| Note: TGI: tumor growth inhibition rate; T/C: relative tumor proliferation rate; PR: partial tumor regression; CR: complete tumor regression. P value is the significant difference result of comparison with IgG1 group; N/A: not applicable. | | | | |

12.4 HuPrime ® gastric cancer GA0006+ Balb/c Nude mice PDX model

Tumor tissue was collected from tumor-bearing mice in a HuPrime® gastric cancer xenotransplantation model GA0006 (Crown Bioscience (Taicang) Inc.; high expression Claudin18.2 stomach tumor from a 57-year-old female patient), it was cut into 3×3×3 mm diameter pieces and inoculated subcutaneously in the right anterior scapulae of Balb/c Nude mice. When the average tumor volume was about 150-250 mm$^3$, the mice were randomly divided into three groups according to the tumor size, 7 mice per group, the grouping day was recorded as Day 0. There were three groups: human IgG1 homotype control antibody (negative control) group (IgG1 10 mg/kg), 2C6.9-TL001-DAR7.12 3-mg/kg group, and 10-mg/kg group. All samples were injected into the tail vein twice a week for a total of 5 times. After drug administration, the tumor volume and body weight of the mice were observed and regularly measured in the way described in Example 12.1.

The experimental results in Table 12 and Figures 12G and 12H show that 2C6.9-TL001-DAR7.12 has significantly inhibitory effect on the tumor growth of GA0006 gastric cancer PDX model in a dose-dependent manner. Compared with the negative control group, the tumor inhibition rate (TGI) of the 3-mg/kg group was up to 94.72% after 5 doses (Day 17), and the tumors in 4 mice were partially subsided; the TGI of the 10-mg/kg group was as high as 124.49%, and the tumors in 7 mice all subsided, indicating that 2C6.9-TL001 can effectively inhibit tumor growth. There is no significant weight loss seen in all treatment groups during the observation period, and the animals were well tolerated.

Table 12: HuPrime® gastric cancerGA0006+Balb/c Nude mice PDX model

| Group | Dosage (mg/kg) | Day 17 | | | | |
|---|---|---|---|---|---|---|
| | | Tumor volume (mm$^3$) ($\bar{x}\pm$SEM) | TGI (%) | T/C (%) | Tumor regression (PR/CR) | P value |
| IgG1 | 10 | 780.46±93.30 | N/A | N/A | 0/0 | N/A |
| 2C6.9-TL001 | 3 | 187.00±46.07 | 94.72 | 23.96 | 4/0 | <0.0001 |
| 2C6.9-TL001 | 10 | 0.00±0.00 | 124.49 | 0.00 | 0/7 | <0.0001 |
| Note: TGI:tumor growth inhibition rate; T/C: relative tumor proliferation rate; PR: partial tumor regression; CR: complete tumor regression. P value is the significant difference result of comparison with IgG1 group; N/A: not applicable. | | | | | | |

The experimental results are shown in Table 13 and Figures 12I and 12J. On day 24 after administration, 2C6.9-TL001-DAR7.12 showed a significant and dose-dependent inhibition of tumor growth in the PDX model of GA0006 gastric cancer. Compared with the negative control group, the tumor inhibition rate (TGI) of the 3-mg/kg group was up to 103.76%, and the tumors in 6 mice partially regressed; the TGI of the 10 mg/kg group reached 113.70%, and the tumors in 7 mice all regressed, indicating that 2C6.9-TL001 can efficiently inhibit tumor growth. There is no significant weight loss seen in all treatment groups during the observation period, and the animals were well tolerated.

Table 13: HuPrime® gastric cancer GA0006+Balb/c Nude mouse PDX model

| Group | Dosage (mg/kg) | Day 24 | | | | |
|---|---|---|---|---|---|---|
| | | Tumor volume (mm$^3$) ($\bar{x}\pm$SEM) | TGI (%) | T/C (%) | Tumor regression (PR/CR) | P value |
| IgG1 | 10 | 1273.78±124.49 | N/A | N/A | 0/0 | N/A |
| 2C6.9-TL001 | 3 | 111.83±42.26 | 103.76 | 8.78 | 6/0 | <0.0001 |

(continued)

| Group | Dosage (mg/kg) | Day 24 | | | | |
|---|---|---|---|---|---|---|
| | | Tumor volume ($mm^3$) ($\bar{x}\pm$SEM) | TGI (%) | T/C (%) | Tumor regression (PR/CR) | P value |
| 2C6.9-TL001 | 10 | 0.00±0.00 | 113.70 | 0.00 | 0/7 | <0.0001 |

Note: TGI: tumor growth inhibition rate; T/C: relative tumor proliferation rate; PR: partial tumor regression; CR: complete tumor regression. P value is the significant difference result of comparison with IgG1 group; N/A is not applicable.

12.5 *In vivo* efficacy evaluation against NCI-N87-Claudin18.2 in Balb/c Nude mouse CDX model

A NCI-N87-Claudin18.2 subcutaneous transplantation tumor model was established according to the method in Example 12.1. When the average tumor volume reached about 140 mm3, the mice were randomly grouped according to the tumor volume, 8 mice for each group. The day of grouping was recorded as Day 0, and the groups were respectively Human IgG1 isotype control antibody (negative control) group (IgG1 for short), 2C6.9-TL001 (DAR: 7.40) 0.3 mg/kg group, 1 mg/kg group, and 3 mg/kg group. All samples were injected into the tail vein twice a week, 6 times in total, dosages were shown in Table 14.

The results are shown in Table 14 and Figures 12K and 12L. When the observation was extended to day 31 after drug withdrawal, compared with the negative control group, the tumor inhibition rate (TGI) of the 2C6.9-TL001 0.3 mg/kg group was 34.04% , while the TGI of the 1 mg/kg and 3 mg/kg groups were up to 122.57% (partial regression of tumors was observed in all mice) and 184.22% (total regression of tumors was observed in 4 mice and partial regression of tumors was observed in 4 mice) respectively. There is no significant weight loss seen in all treatment groups during the observation period, and the animals were well tolerated.

Table 14: *In vivo* efficacy evaluation against NCI-N87-Claudin18.2 in Balb/c Nude mouse CDX model

| Group | Dosage (mg/kg) | Day 31 | | | | |
|---|---|---|---|---|---|---|
| | | Tumor volume ($mm^3$) ($\bar{x}\pm$SEM) | TGI (%) | T/C (%) | Tumor regression (PR/CR) | P value |
| IgG1 | 3 | 592.50±24.38 | N/A | N/A | 0/0 | N/A |
| 2C6.9-TL001 | 0.3 | 437.74±16.68 | 34.04 | 74.04 | 0/0 | <0.001 |
| 2C6.9-TL001 | 1 | 107.06±9.63 | 122.57 | 18.13 | 8/0 | <0.0001 |
| 2C6.9-TL001 | 3 | 21.83±2.26 | 184.22 | 3.69 | 4/4 | <0.0001 |

Note: TGI: tumor growth inhibition rate; T/C: relative tumor proliferation rate; PR: partial tumor regression; CR: complete tumor regression. P value is the significant difference result of comparison with IgG1 group; N/A: not applicable.

*12.6In vivo* efficacy evaluation against HEK293T-Claudin18.2 in Balb/c Nude mouse CDX model

HEK293T-Claudin18.2 cells (human embryonic kidney cells) were cultured with DMEM medium containing 3 μg/mL puromycin and 10% fetal bovine serum (FBS) at 37°C with 5% $CO_2$. The cells in the exponential growth phase were collected, resuspended in PBS, and inoculated subcutaneously into female Balb/c Nude mice (Zhejiang Vital River Laboratory Animal Technology Co., Ltd.) at a cell volume of $1\times10^7$/mouse (suspended in 0.1 mL PBS) to establish a subcutaneous transplantation tumor model. When the average tumor volume reached about 120-140 $mm^3$, the mice were randomly grouped according to the tumor volume, 8 mice for each group. The day of grouping was recorded as Day 0, and the groups were respectively Human IgG1 isotype control antibody (negative control) group (referred to as IgG1), 2C6.9-TL001 (DAR: 7.40) 0.3 mg/kg group, 1 mg/kg group, and 3 mg/kg group. All samples were injected into the tail vein twice a week, 6 times in total, dosages were shown in Table 15.

The results are shown in Table 15 and Figures 12M and 12N. Compared with the negative control group, 2C6.9-TL001 (DAR: 7.40) showed a significant and dose-dependent inhibition of tumor growth in the HEK293T-Claudin18.2 human embryonic kidney cell transplantation tumor model after 6 doses (day 21 after the first dose). The tumor inhibition rate (TGI) of the 2C6.9-TL001 0.3 mg/kg group was 70.99% (total tumor regression was observed in 1 mouse), while

the TGI of the 1 mg/kg and 3 mg/kg groups were 94.98% (partial tumor regression was observed in 2 mice) and 182.81% (partial tumor regression was observed in 2 mice and total tumor regression was observed in 6 mice), respectively.

Table 15: *In vivo* efficacy evaluation against HEK293T-Claudin18.2 in Balb/c Nude mouse CDX model

| Group | Dosage (mg/kg) | Day 21 | | | | |
|---|---|---|---|---|---|---|
| | | Tumor volume ($mm^3$) ($\bar{x}\pm$SEM) | TGI (%) | T/C (%) | Tumor regression (PR/CR) | P value |
| IgG1 | 3 | 1547.48±245.85 | N/A | N/A | 0/0 | N/A |
| 2C6.9-TL001 | 0.3 | 507.95±99.30 | 70.99 | 32.83 | 0/1 | <0.01 |
| 2C6.9-TL001 | 1 | 156.48±23.87 | 94.98 | 10.13 | 2/0 | <0.0001 |
| 2C6.9-TL001 | 3 | 14.36±3.63 | 182.81 | 0.92 | 2/6 | <0.0001 |

Note: TGI: tumor growth inhibition rate; T/C: relative tumor proliferation rate; PR: partial tumor regression; CR: complete tumor regression. P value is the significant difference result of comparison with IgG1 group; N/A: not applicable.

12.7 *In vivo* efficacy evaluation against NUGC-4 in Balb/c Nude mouse CDX model

NUGC-4 cells were cultured with RPMI1640 medium containing 10% fetal bovine serum (FBS) at 37°C and 5% $CO_2$. The cells in the exponential growth phase were collected, resuspended in PBS, and inoculated subcutaneously into female Balb/c Nude mice (Zhejiang Vital River Laboratory Animal Technology Co., Ltd.) at a cell volume of $5\times10^6$/mouse (suspended in 0.1 mL PBS) to establish a subcutaneous transplantation tumor model. When the average tumor volume reached about 80 $mm^3$, the mice were randomly grouped according to the tumor volume, 8 mice for each group. The day of grouping was recorded as Day 0, and the groups were respectively Human IgG1 isotype control antibody (negative control) group (referred to as IgG1), 2C6.9-TL001 (DAR: 7.40) 3 mg/kg group, and 10 mg/kg group. All samples were injected into the tail vein twice a week, 6 times in total, dosages were shown in Table 16.

The results are shown in Table 16 and Figures 12O and 12P. Compared with the negative control group, 2C6.9-TL001 (DAR: 7.40) showed a significant and dose-dependent inhibition of tumor growth in the NUGC-4 gastric cancer transplantation tumor model after 6 doses (day 21 after the first dose). The 2C6.9-TL001 3 mg/kg group showed a tumor inhibition rate (TGI) of 56.62%, while the TGI of the 10 mg/kg group was 90.28% (partial tumor regression was observed in 2 mice).

Table 16: *In vivo* efficacy evaluation against NUGC-4 in Balb/c Nude mice CDX model

| Group | Dosage (mg/kg) | Day 21 | | | | |
|---|---|---|---|---|---|---|
| | | Tumor volume ($mm^3$) ($\bar{x}\pm$SEM) | TGI (%) | T/C (%) | Tumor regression (PR/CR) | P value |
| IgG1 | 10 | 782.66±47.61 | N/A | N/A | 0/0 | N/A |
| 2C6.9-TL001 | 3 | 385.67±35.09 | 56.62 | 48.95 | 0/0 | <0.0001 |
| 2C6.9-TL001 | 10 | 148.23±25.52 | 90.28 | 19.09 | 2/0 | <0.0001 |

Note: TGI: tumor growth inhibition rate; T/C: relative tumor proliferation rate; PR: partial tumor regression; CR: complete tumor regression. P value is the significant difference result of comparison with IgG1 group; N/A: not applicable.

In conclusion, 2C6.9-TL001 can effectively inhibit the growth of Claudin18.2 positive tumor in either CDX model or PDX model in a dose-dependent manner, and is safe to use.

Although the embodiments of this invention have been described in detail, those skilled in the art would understand: following the guidance of the teachings of all disclosure, modifications and variations of the details can be made, and all these changes are all fall within the scope of the present invention. The scope of the present invention is given by the claims appended hereto and any equivalent thereof.

## Claims

1. An antibody-drug conjugate (ADC), the structure of which is shown in formula (I),

$$(D\text{-}L)_{\gamma}\text{-}A \qquad \text{formula (I)}$$

wherein, D is a fragment of a bioactive molecule; L is a linker;

y is an integer from 1 to 10; preferably, y is an integer from 1 to 8 (for example, 1, 2, 3, 4, 5, 6, 7 or 8); A is an antibody or antigen-binding fragment thereof that specifically binds to human CLDN18.2, and the antibody or antigen-binding fragment thereof comprises:

(1) the following VH and/or VL, wherein CDRs are defined according to the IMGT numbering system:

(1-1): VH comprising the following 3 CDRs: CDR-H1 having the sequence of SEQ ID No: 1, CDR-H2 having the sequence of SEQ ID No: 2 or 21, and CDR-H3 having the sequence of SEQ ID No: 3; and/or VL comprising the following 3 CDRs: CDR-L1 having the sequence of SEQ ID No: 4, CDR-L2 having the sequence of SEQ ID No: 5, and CDR-L3 having the sequence of SEQ ID No: 6;
or
(1-2): compared with the VH or VL described in (1-1), at least one CDR contains a mutation, which is the substitution, deletion or addition of one or more amino acids, or any combination thereof (for example, the substitution, deletion or addition of 1, 2 or 3 amino acids, or any combination thereof); the antibody or antigen-binding fragment thereof containing the mutation is still capable of specifically binding to human CLDN 18.2;
or

(2) the following VH and/or VL, wherein CDRs are defined according to the AbM numbering system:

(2-1): VH comprising the following 3 CDRs: CDR-H1 having the sequence of SEQ ID No: 7, CDR-H2 having the sequence of SEQ ID No: 8 or 22, and CDR-H3 having the sequence of SEQ ID No: 9; and/or VL comprising the following 3 CDRs: CDR-L1 having the sequence of SEQ ID No: 10, CDR-L2 having the sequence of SEQ ID No: 11, and CDR-L3 having the sequence of SEQ ID No: 12;
or
(2-2): compared with the VH or VL described in (2-1), at least one CDR contains a mutation, which is the substitution, deletion or addition of one or more amino acids, or any combination thereof (for example, the substitution, deletion or addition of 1, 2 or 3 amino acids, or any combination thereof); the antibody or antigen-binding fragment thereof containing the mutation is still capable of specifically binding to human CLDN 18.2;
preferably, the substitution is a conservative substitution;
preferably, the VH and/or VL of the antibody or antigen-binding fragment thereof include the framework regions (FRs) of a human or murine immunoglobulin.

2. The ADC according to claim 1, wherein:

(1) the antibody or antigen-binding fragment thereof comprises the VH as set forth in SEQ ID NO: 13 or 14; and/or the VL as set forth in SEQ ID NO: 15;
(2) compared with the VH described in (1), the VH comprised in the antibody or antigen-binding fragment thereof has at least 70%, at least 80%, at least 85%, at least 90%, at least 91%, at least 92%, at least 93%, at least 94%, at least 95%, at least 96%, at least 97%, at least 98%, at least 99%, or 100% identity; and/or, compared with the VL described in (1), the VL compirsed in the antibody or antigen-binding fragment thereof has at least 70%, at least 80%, at least 85%, at least 90%, at least 91%, at least 92%, at least 93%, at least 94%, at least 95%, at least 96%, at least 97%, at least 98%, at least 99%, or 100% identity;
or
(3) compared with the VH described in (1), the VH comprised in the antibody or antigen-binding fragment thereof has the substitution, deletion or addition of one or more amino acids, or any combination thereof (for example, the substitution, deletion or addition of 1, 2, 3, 4 or 5 amino acids, or any combination thereof); and/or, compared with the VL described in (1), the VL comprised in the antibody or antigen-binding fragment thereof has the substitution, deletion or addition of one or more amino acids, or any combination thereof (for example, the

substitution, deletion or addition of 1, 2, 3, 4 or 5 amino acids, or any combination thereof); preferably, the substitution is a conservative substitution.

**3.** The ADC according to claim 1 or 2, wherein the antibody comprises:

(1) the CH (heavy chain constant region) of human immunoglobulin or a variant thereof, wherein the variant comprises a substitution, deletion or addition of one or more amino acids compared with the wild type sequence from which it is derived (for example, the substitution, deletion or addition of up to 20, up to 15, up to 10, or up to 5 amino acids; for example, the substitution, deletion or addition of 1, 2, 3, 4, or 5 amino acids); and/or
(2) the CL (light chain constant region) of human immunoglobulin or a variant thereof, wherein the variant comprises a substitution, deletion or addition of one or more amino acids compared with the wild type sequence from which it is derived (for example, the substitution, deletion or addition of up to 20, up to 15, up to 10, or up to 5 amino acids; for example, the substitution, deletion or addition of 1, 2, 3, 4 or 5 amino acids);

preferably, the CH is an IgG heavy chain constant region, such as an IgG1, IgG2, IgG3 or IgG4 heavy chain constant region;
preferably, the antibody comprises the heavy chain constant region of human IgG1;
preferably, the antibody comprises the CH as set forth in SEQ ID NO: 16, or a variant thereof which has a conservative substitution of up to 20 amino acids compared with SEQ ID NO: 16 (for example, a conservative substitution of up to 15, 10 or 5 amino acids; for example, a conservative substitution of 1, 2, 3, 4, or 5 amino acids);
preferably, the CL is a constant region of light chain κ;
preferably, the antibody comprises the CL as set forth in SEQ ID NO: 17, or a variant thereof which has a conservative substitution of up to 20 amino acids compared with SEQ ID NO: 17 (for example, a conservative substitution of up to 15, 10 or 5 amino acids; for example, a conservative substitution of 1, 2, 3, 4, or 5 amino acids);
more preferably, the antibody comprises a CH as set forth in SEQ ID NO: 16 and/or a CL as set forth in SEQ ID NO: 17.

**4.** The ADC according to any one of claims 1 to 3, wherein the antibody is:

(1) the heavy chain comprising the VH of the sequence as set forth in SEQ ID NO: 13 and the CH of the sequence as set forth in SEQ ID NO: 16, and the light chain comprising the VL of the sequence as set forth in SEQ ID NO: 15 and the CL of the sequence as set forth in SEQ ID NO: 17;
or
(2) the heavy chain comprising the VH of the sequence as set forth in SEQ ID NO: 14 and the CH of the sequence as set forth in SEQ ID NO: 16, and the light chain comprising the VL of the sequence as set forth in SEQ ID NO: 15 and the CL of the sequence as set forth in SEQ ID NO: 17.

**5.** The ADC according to any one of claims 1 to 4, wherein the antibody or antigen-binding fragment thereof comprises:

(1) a heavy chain, comprising an amino acid sequence selected from the group consisting of:

(1-1) the sequence as set forth in SEQ ID NO: 18 or SEQ ID NO: 19;
(1-2) a sequence having the substitution, deletion or addition of one or more amino acids (e.g., the substitution, deletion or addition of 1, 2, 3, 4 or 5 amino acids) compared to the sequence as set forth in SEQ ID NO: 18 or SEQ ID NO: 19; or
(1-3) a sequence having at least 80%, at least 85%, at least 90%, at least 91%, at least 92%, at least 93%, at least 94%, at least 95%, at least 96%, at least 97%, at least 98%, at least 99%, or 100% identity to the sequence as set forth in SEQ ID NO: 18 or SEQ ID NO: 19;
and

(2) a light chain, comprising an amino acid sequence selected from the group consisting of:

(2-1) the sequence as set forth in SEQ ID NO:20;
(2-2) a sequence having the substitution, deletion or addition of one or more amino acids (e.g., the substitution, deletion or addition of 1, 2, 3, 4 or 5 amino acids) compared to the sequence as set forth in SEQ ID NO: 20; or

(2-3) a sequence having at least 80%, at least 85%, at least 90%, at least 91%, at least 92%, at least 93%, at least 94%, at least 95%, at least 96%, at least 97%, at least 98%, at least 99%, or 100% identity to the sequence as set forth in SEQ ID NO: 20;
preferably, the substitutions described in (1-2) and (2-2) are conservative substitutions.

**6.** The ADC according to any one of claims 1 to 5, wherein the antibody or antigen-binding fragment thereof is selected from ScFv, Fab, Fab', $(Fab')_2$, Fv fragment, disulfide bond-linked Fv(dsFv), diabody, bispecific antibody, and multispecific antibody.

**7.** The ADC according to any one of claims 1 to 6, which has a structure as shown in formula (II),

$$\{D-[L_1-(L_2)_{m1}-(L_3)_{m2}-(L_4)_{m3}-E]\}_\gamma-A \qquad \text{Formula (II)}$$

wherein,

Li is

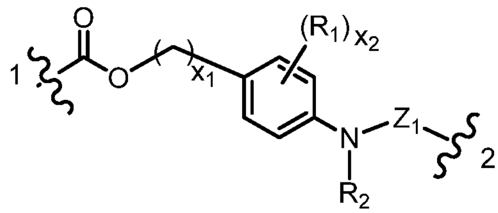

,

wherein $R_1$ and $R_2$ each independently are hydrogen (such as protium or deuterium), halogen, carboxylic acid group, sulfonic acid group, cyano, $C_{1-6}$ alkyl, halogenated $C_{1-6}$ alkyl, cyano- substituted $C_{1-6}$ alkyl (e.g., $-CH_2CN$), $C_{1-6}$ alkoxy, $C_{2-10}$ alkenyl or $C_{2-10}$ alkynyl; $Z_1$ is an amino acid or a peptide composed of 2-10 amino acids; $x_1$ and $x_2$ each independently are 0, 1, 2, 3, 4, 5 or 6; $L_1$ is connected to D at position 1, and connected to $L_2$ at position 2;
$L_2$ is

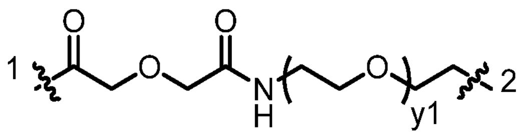

,

wherein $y_1$ is 0, 1, 2, 3, 4, 5, 6, 7, 8, 9 or 10; $L_2$ is connected to $L_1$ at position 1, and connected to $L_3$ at position 2;
$L_3$ is selected from a 5-12 membered aromatic heterocycle;
$L_4$ is

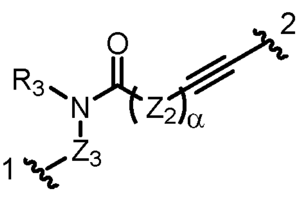

,

wherein $Z_2$ is selected from $C_{1-6}$ alkylene, $C_{2-10}$ alkenylene, $C_{2-10}$ alkynylene, and $C_{3-8}$ cycloalkylene; $R_3$ is selected from hydrogen (such as protium or deuterium ) and $C_{1-6}$ alkyl; $Z_3$ does not exist or is selected from $C_{1-6}$ alkylene; alternatively, $R_3$ and $Z_3$ together with the nitrogen atom to which they are attached form a 4-8 membered heterocyclyl; $\alpha$ is 0,1,2,3,4,5 or 6, and $L_4$ is connected to E at position 2 and connected to $L_3$ at position 1;
E is

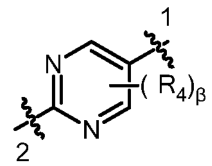

,

wherein each $R_4$ is hydrogen (such as protium or deuterium), $\beta$ is 0, 1, 2, 3, 4, 5 or 6, and E is connected to A at position 2, and connected to $L_4$ at position 1;

mi, $m_2$ and $m_3$ are each independently 0, 1, 2, 3, 4, 5, 6, 7, 8, 9 or 10;

A is as defined in any one of claims 1 to 6;

D and $\gamma$ are as defined in claim 1.

**8.** The ADC according to any one of claims 1 to 6, which has a structure as shown in formula (III):

$$\{D-[(L_1')_{m4}-L_1-(L_5)_{m5}-(L_3)_{m2}-(L_4)_{m3}-E]\}_{\gamma}-A \qquad \text{Formula (III)}$$

wherein

$L_1$' is

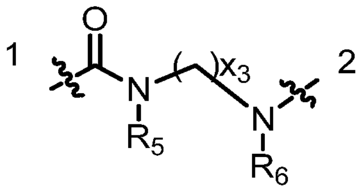

,

wherein $R_5$ and $R_6$ each independently are hydrogen (such as protium or deuterium) or $C_{1-6}$ alkyl; $x_3$ is 1, 2, 3, 4, 5 or 6; and if $L_1$' is present, it connects with D at position 1 and connects with $L_1$ at position 2;

$L_1$ is

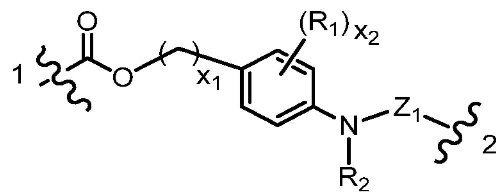

,

wherein $R_1$ and $R_2$ each independently are hydrogen (such as protium or deuterium), halogen, carboxylic acid group, sulfonic acid group, cyano, $C_{1-6}$ alkyl, halogenated $C_{1-6}$ alkyl, cyano- substituted $C_{1-6}$ alkyl (e.g., $-CH_2CN$), $C_{1-6}$ alkoxy, $C_{2-10}$ alkenyl or $C_{2-10}$ alkynyl; $Z_1$ is an amino acid or a peptide composed of 2-10 amino acids; $x_1$ and $x_2$ each independently are 0, 1 , 2, 3, 4, 5 or 6; and, at position 1, $L_1$ is connected to $L_1$' (when $L_1$' exists) at position 1, or to D (when $L_1$' does not exist) at position 1; $L_1$ is connected to $L_5$ at position 2;

$L_5$ is

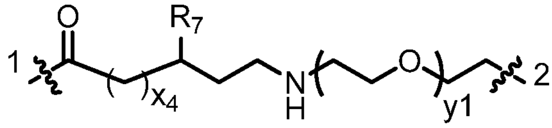

,

wherein $R_7$ is hydrogen or $C_{1-6}$ alkyl, or $R_7$ is connected to the N atom on the $\gamma$-C thereof to form a 5-6 membered heterocyclyl; $x_4$ is 1, 2, 3, 4, 5 or 6; $y_1$ is 0, 1, 2, 3, 4, 5, 6, 7, 8, 9 or 10; and $L_5$ is connected to $L_1$ at position 1, and connected to $L_3$ at position 2;

$L_3$ is selected from a 5-12 membered aromatic heterocycle;

L4 is

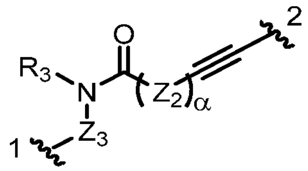

,

wherein $Z_2$ is selected from $C_{1-6}$ alkylene, $C_{2-10}$ alkenylene, $C_{2-10}$ alkynylene and $C_{3-8}$ cycloalkylene; $R_3$ is selected from hydrogen (such as protium and deuterium) and $C_{1-6}$ alkyl; $Z_3$ does not exist or is selected from $C_{1-6}$ alkylene; or, $R_3$ and $Z_3$ together with the nitrogen atom to which they are attached form a 4-8 membered heterocyclic radical; $\alpha$ is 0, 1, 2, 3, 4, 5 or 6; and $L_4$ is connected to E at position 2, and connected to $L_3$ at position 1;
E is

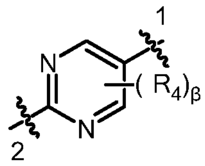

,

wherein each $R_4$ independently is hydrogen (such as protium or deuterium), $\beta$ is 0, 1, 2, 3, 4, 5, or 6, and E is connected to A at position 2, and connected to $L_4$ at position 1;
mi, $m_2$, $m_3$ and $m_4$ each independently are 0, 1, 2, 3, 4, 5, 6, 7, 8, 9 or 10;
A is as defined in any one of claims 1 to 6;
D and $\gamma$ are as defined in claim 1.

**9.** The ADC according to any one of claims 7 to 8, wherein $L_1$ is

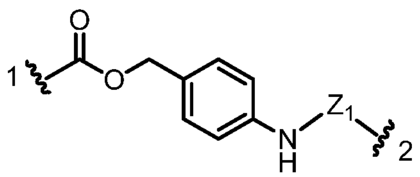

,

wherein $Z_1$ is an amino acid or a peptide composed of 2-5 amino acids, wherein the amino acid is selected from Lys, Cit, Val, D-Val, Phe, Leu, Gly, Ala, and Asn; preferably, $Z_1$ is selected from Cit, Lys, Cit-Val, and Ala-Val;

preferably, $L_1$ is

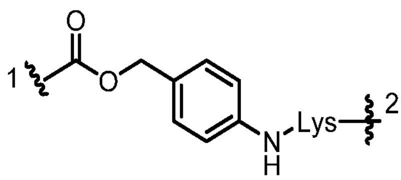

;

preferably, $L_1$ is

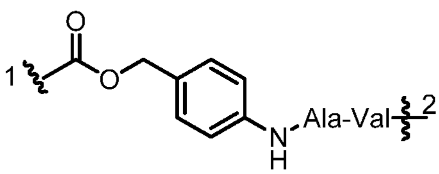

.

**10.** The ADC according to any one of claims 7 to 9, wherein $L_2$ is

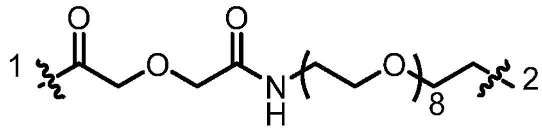

,

and $m_1$ is 1.

**11.** The ADC according to any one of claims 7 to 10, wherein $L_3$ is a 5-6 membered aromatic heterocycle, and $m_2$ is 1; preferably, $L_3$ is triazole, and $m_2$ is 1.

**12.** The ADC according to any one of claims 7 to 11, wherein $L_4$ is

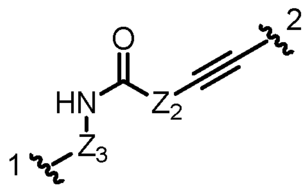

,

$Z_2$ is $C_{1-6}$ alkylene, $Z_3$ is $C_{1-6}$ alkylene; and $m_3$ is 1;
preferably, $L_4$ is

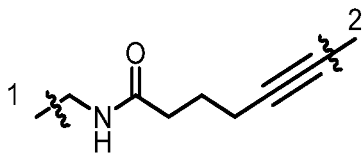

,

and $m_3$ is 1.

**13.** The ADC according to any one of claims 8 to 12, wherein $L_1$' is

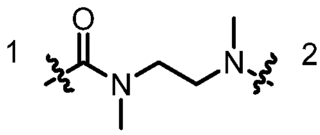

.

**14.** The ADC according to any one of claims 8 to 13, wherein $L_5$ is

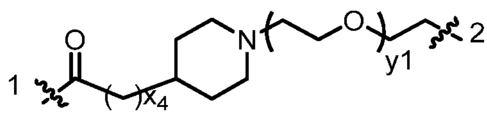

,

wherein $x_4$ is 1, 2, 3, 4, 5 or 6; $y_1$ is 1, 2, 3, 4, 5, 6, 7, 8, 9 or 10;
preferably, $L_5$ is

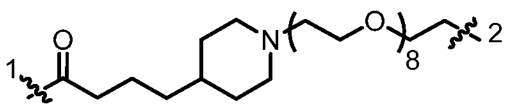

.

**15.** The ADC according to any one of claims 1 to 14, wherein D is

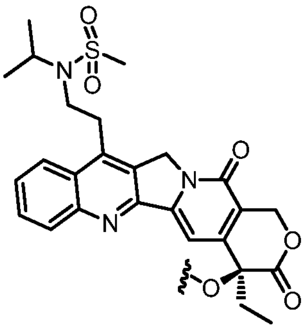
,
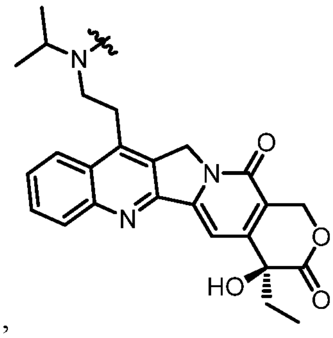
or
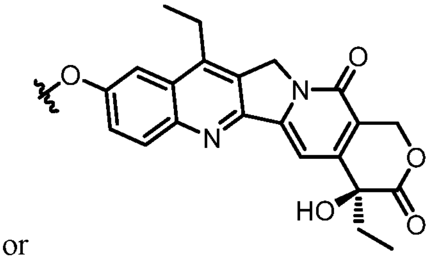
;

preferably, D is

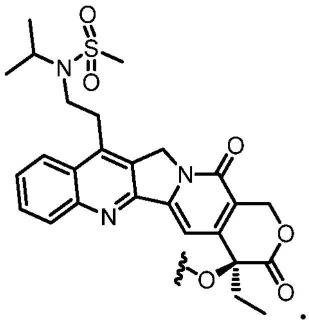
.

**16.** The ADC according to claim 7, wherein the D-[$L_1$-$(L_2)_{m1}$-$(L_3)_{m2}$-$(L_4)_{m3}$-E]- in formula (II) is

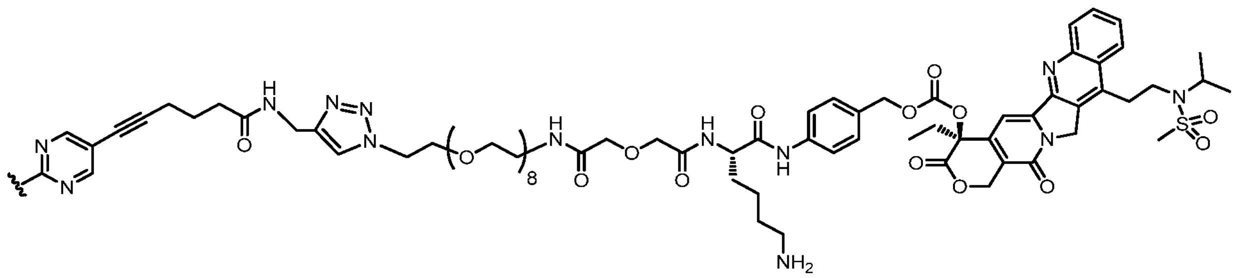
.

**17.** The ADC according to claim 8, wherein the D-[$(L_1')_{m4}$-$L_1$-$(L_5)_{m5}$-$(L_3)_{m2}$-$(L_4)_{m3}$-E]-in formula (III) is

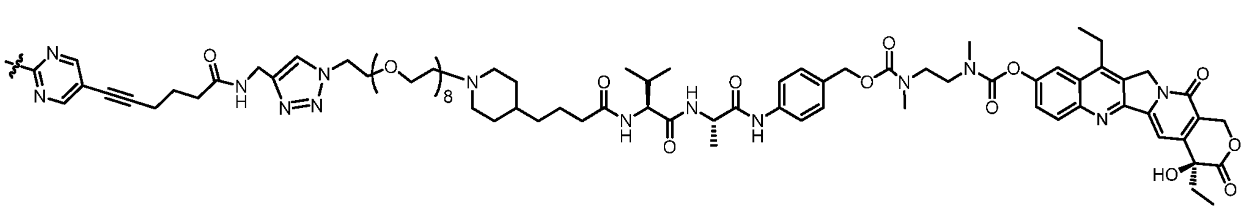

or

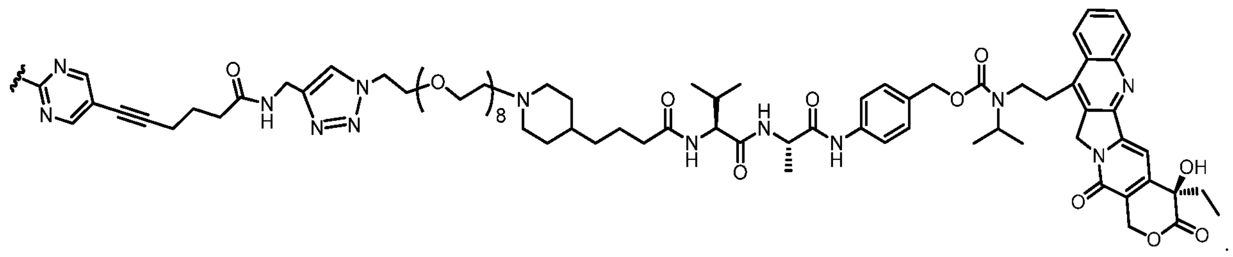

18. The ADC according to any one of claims 1 to 17, which is selected from:

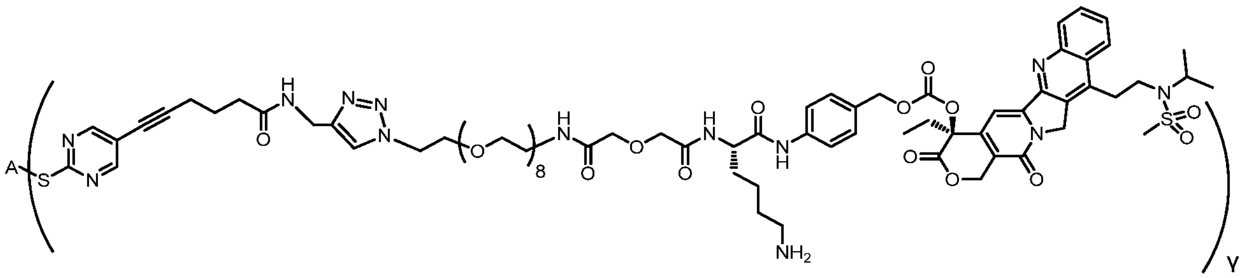

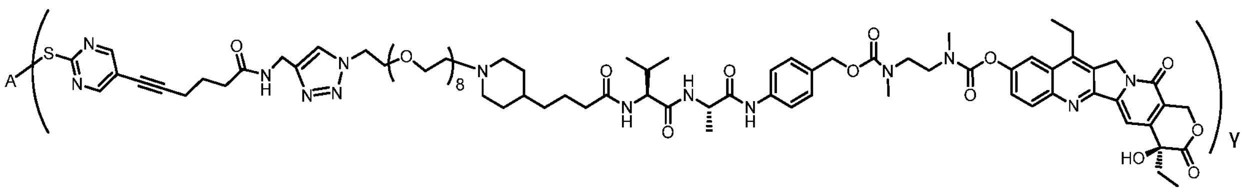

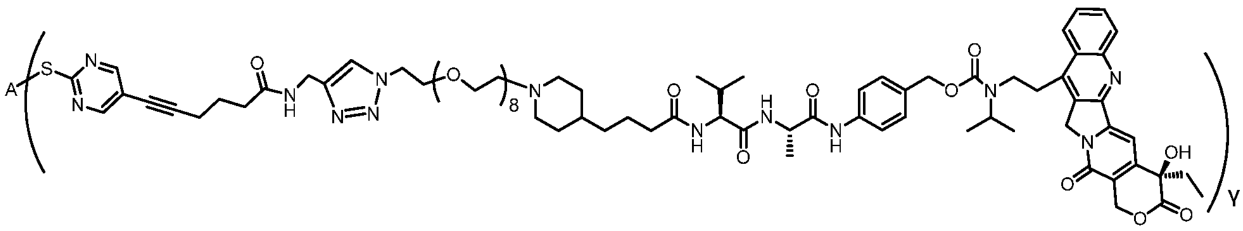

wherein $\gamma$ is an integer from 1 to 10; for example, $\gamma$ is 1, 2, 3, 4, 5, 6, 7, 8, 9 or 10.

19. The ADC according to any one of claims 1 to 18, wherein the antibody or antigen-binding fragment thereof has a label; preferably, the antibody or antigen-binding fragment thereof has a detectable label, such as enzymes (i.e., horseradish peroxidase), radionuclides, fluorescent dyes, luminescent substances (e.g., chemiluminescent substances) or biotin.

20. A composition, comprising the ADC according to any one of claims 1 to 19, and the molar ratio of the fragment of the bioactive molecule to the antibody or antigen-binding fragment thereof that specifically binds to CLDN18.2 (DAR value) is a decimal or integer from 1 to 10 (for example, a decimal or integer from 3 to 8, such as 1.0, 1.5, 2.0, 2.5, 3.0, 3.1, 3.2, 3.3, 3.4, 3.5, 3.6, 3.7 , 3.79, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1 , 6.2, 6.3, 6.4, 6.5, 6.6, 6.7, 6.8, 6.9, 6.95, 7.0, 7.03, 7.1, 7.12, 7.2, 7.3, 7.4, 7.5, 7.6, 7.7, 7.8, 7.9 or 8.0).

21. A pharmaceutical composition, comprising the ADC according to any one of claims 1 to 19, or the composition according to claim 20, and a pharmaceutically acceptable carrier and/or excipient.

22. Use of the ADC according to any one of claims 1 to 19, the composition according to claim 20, or the pharmaceutical composition according to claim 21 in the preparation of a medicament for the prevention and/or treatment and/or adjuvant treatment of tumors;

optionally, the medicament further includes an additional anti-tumor active ingredient; preferably, the ADC, composition or pharmaceutical composition is administered separately, simultaneously or sequentially with the additional anti-tumor active ingredient; preferably, the additional anti-tumor active ingredient is a bioactive polypeptide or an active fragment thereof, or a chemotherapeutic drug; more preferably, the bioactive polypeptide is selected from immune checkpoint inhibitors (e.g., PD-1 antibody, PD-L1 antibody, CTLA-4 antibody, and LAG-3 antibody), or cytokine (e.g., interferon, IL-2, IL-15, GM-CSF, IL-7, IL-12, IL-18, and IL-21); preferably, the chemotherapeutic drug is one or more selected from epirubicin, oxaliplatin, capecitabine, 5-fluorouracil, leucovorin, paclitaxel, and albumin-bound paclitaxel.

**23.** Use according to claim 22, wherein the tumor is selected from solid tumors, hematological tumors, or metastatic, refractory or recurrent lesions of cancers;

preferably, the tumor or cancer is selected from esophageal cancer, gastrointestinal cancer, pancreatic cancer, thyroid cancer, colorectal cancer, kidney cancer, lung cancer (such as non-small cell lung cancer, NSCLC), liver cancer, gastric cancer, gastric adenocarcinoma, gastroesophageal junction (GEJ) adenocarcinoma, head and neck cancer, bladder cancer, breast cancer, uterine cancer, cervical cancer, ovarian cancer, prostate cancer, testicular cancer, germ cell tumor, bone cancer, skin cancer, thymus cancer, cholangiocarcinoma, gallbladder cancer, melanoma, mesothelioma, lymphoma, myeloma (such as multiple myeloma), sarcoma, glioblastoma, and leukemia;
preferably, the tumor is selected from gastric cancer, gastric adenocarcinoma, gastroesophageal junction (GEJ) adenocarcinoma, esophageal cancer, gastrointestinal cancer, pancreatic cancer, and lung cancer (such as NSCLC);
preferably, the tumor is gastric cancer, gastric adenocarcinoma or gastroesophageal junction (GEJ) adenocarcinoma, such as a locally advanced unresectable or metastatic tumor of gastric cancer, gastric adenocarcinoma or gastroesophageal junction (GEJ) adenocarcinoma;
preferably, the tumor is CLDN 18.2 positive, and more preferably, the tumor is HER2 negative.

**24.** A method of preventing and/or treating a tumor, and/or delaying tumor progression, and/or reducing or inhibiting tumor recurrence in a subject, wherein the method comprises a step of administering to a subject in need thereof an effective amount of the antibody-drug conjugate (ADC) according to any one of claims 1 to 19, the composition according to claim 20, or the pharmaceutical composition according to claim 21;

optionally, the method further comprises a step of applying a second therapy to the subject, wherein the second therapy is selected from surgery, chemotherapy, radiotherapy, immunotherapy, gene therapy, DNA therapy, RNA therapy, nanotherapy, viral therapy, adjuvant therapy, and any combination thereof; the method and the second therapy are applied separately, in combination, simultaneously, or sequentially;
preferably, the immunotherapy includes the administration of a bioactive polypeptide, and more preferably, the bioactive polypeptide is selected from immune checkpoint inhibitors (e.g., PD-1 antibody, PD-L1 antibody, CTLA-4 antibody, and LAG-3 antibody), or cytokine (e.g., interferon, IL-2, IL-15, GM-CSF, IL-7, IL-12, IL-18, and IL-21);
preferably, the chemotherapy is one or more selected from epirubicin, oxaliplatin, capecitabine, 5-fluorouracil, folinic acid, paclitaxel, and albumin-bound paclitaxel;
preferably, the tumor is selected from solid tumors, hematological tumors, and the metastatic, refractory or recurrent lesions of cancers;
preferably, the tumor or cancer is selected from esophageal cancer, gastrointestinal cancer, pancreatic cancer, thyroid cancer, colorectal cancer, kidney cancer, lung cancer (such as NSCLC), liver cancer, gastric cancer, gastric adenocarcinoma, gastroesophageal junction (GEJ) adenocarcinoma, head and neck cancer, bladder cancer, breast cancer, uterine cancer, cervical cancer, ovarian cancer, prostate cancer, testicular cancer, germ cell tumor, bone cancer, skin cancer, thymus cancer, cholangiocarcinoma, gallbladder cancer, melanoma, mesothelioma, lymphoma, myeloma (such as multiple myeloma), sarcoma, glioblastoma, and leukemia;
preferably, the tumor is selected from gastric cancer, gastric adenocarcinoma, gastroesophageal junction (GEJ) adenocarcinoma, esophageal cancer, gastrointestinal cancer, pancreatic cancer, lung cancer (such as NSCLC);
preferably, the tumor is gastric cancer, gastric adenocarcinoma or gastroesophageal junction (GEJ) adenocarcinoma, such as a locally advanced unresectable or metastatic tumor of gastric cancer, gastric adenocarcinoma or gastroesophageal junction (GEJ) adenocarcinoma;
preferably, the tumor is CLDN 18.2 positive, and more preferably, the tumor is HER2 negative.

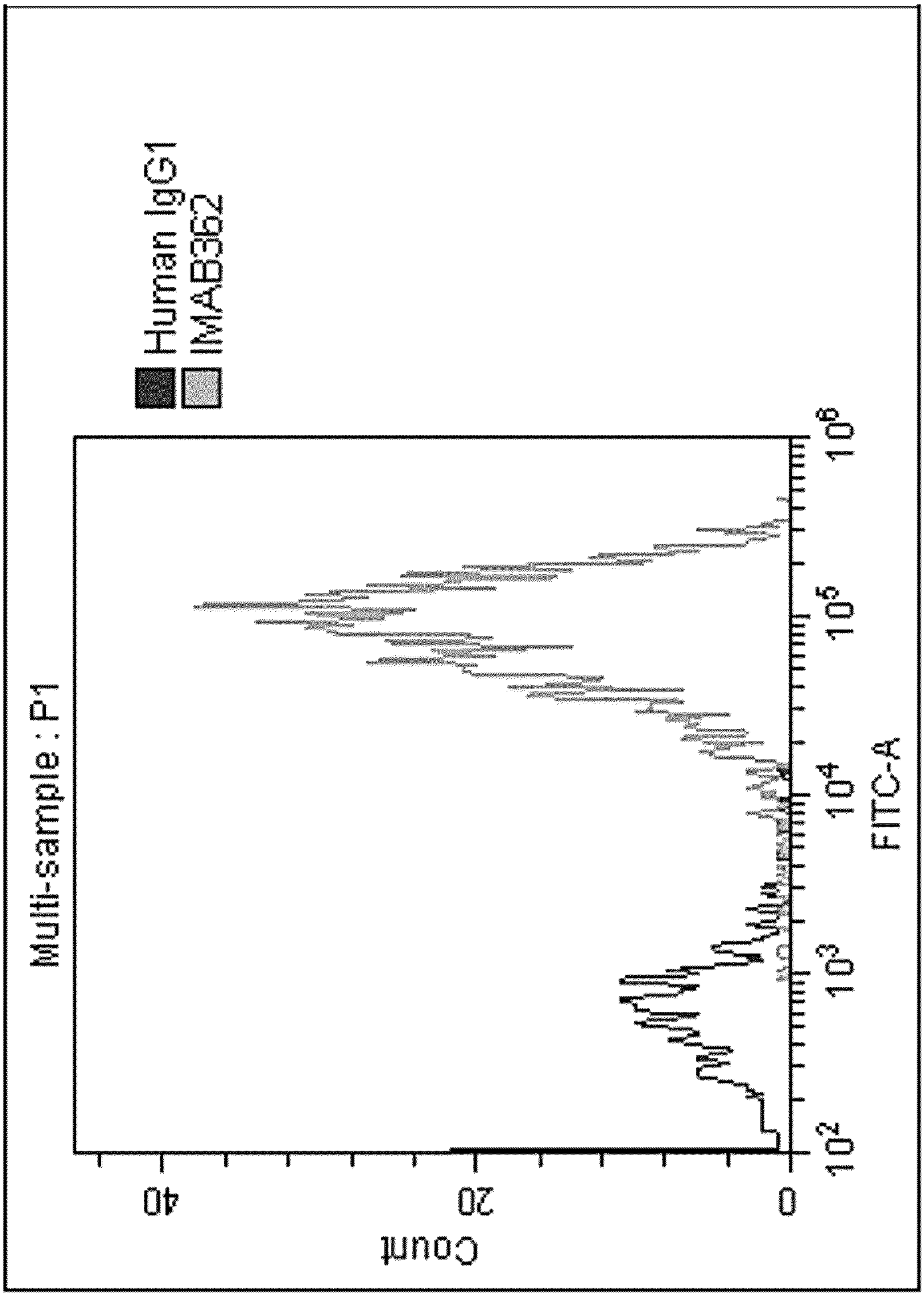
Multi-sample : P1
Human IgG1
IMAB362
Count
0
20
40
FITC-A
$10^2$
$10^3$
$10^4$
$10^5$
$10^6$

Fig. 1A

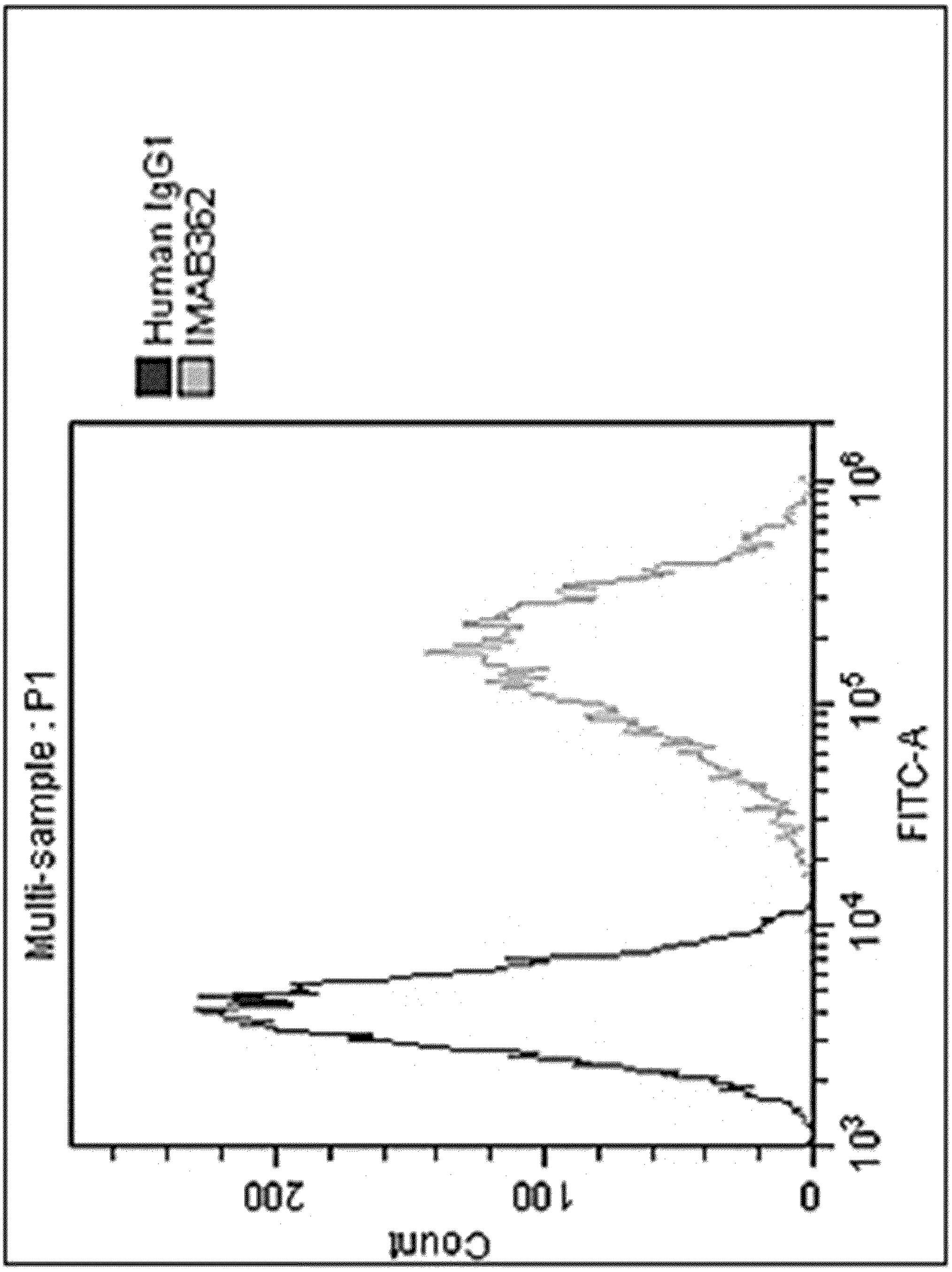
Multi-sample : P1
Human IgG1
IMAB362
Count
0
100
200
FITC-A
$10^3$
$10^4$
$10^5$
$10^6$

Fig. 1B

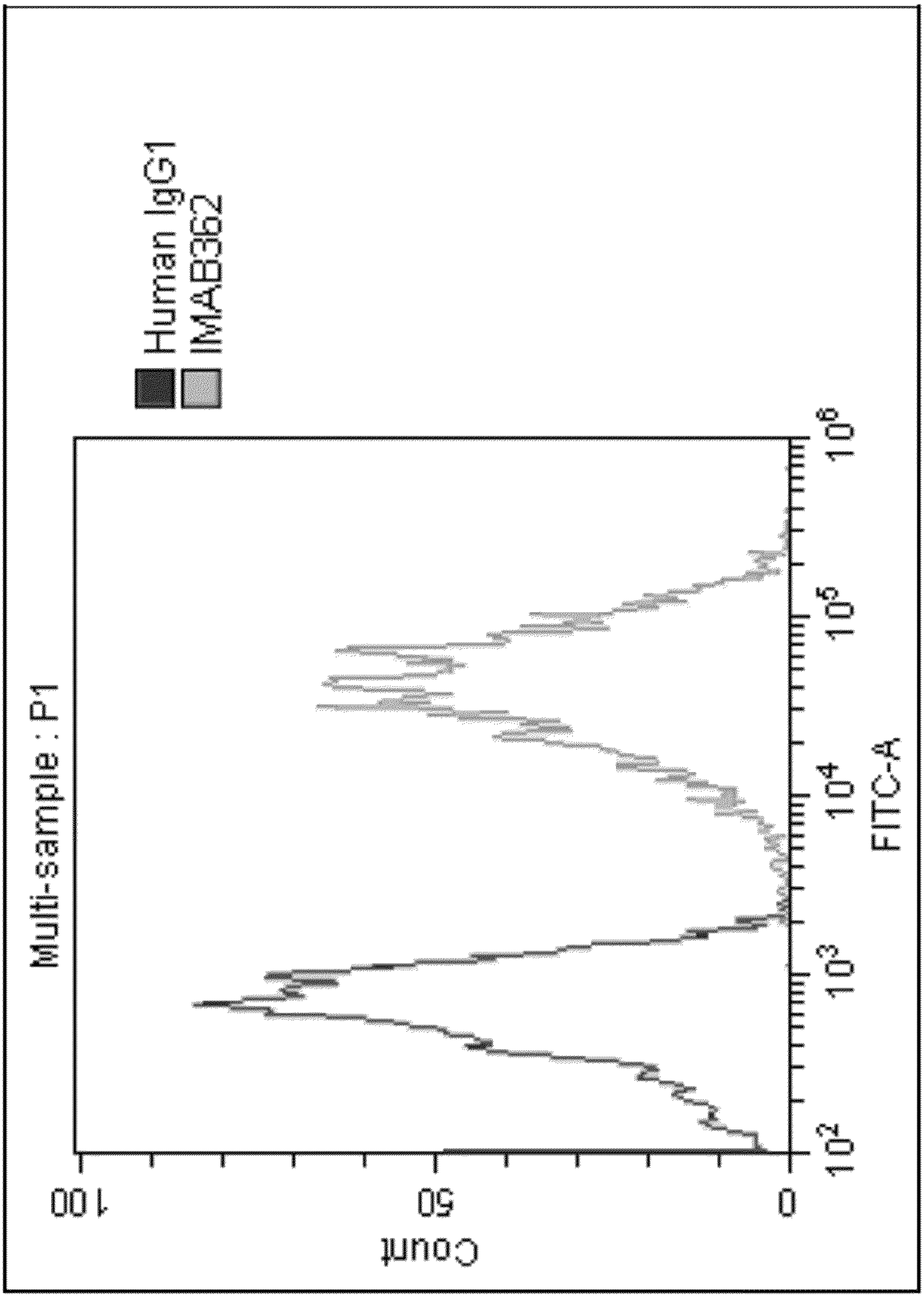
Multi-sample : P1
Human IgG1
IMAB362
Count
100
50
0
$10^2$
$10^3$
$10^4$
$10^5$
$10^6$
FITC-A

Fig. 1C

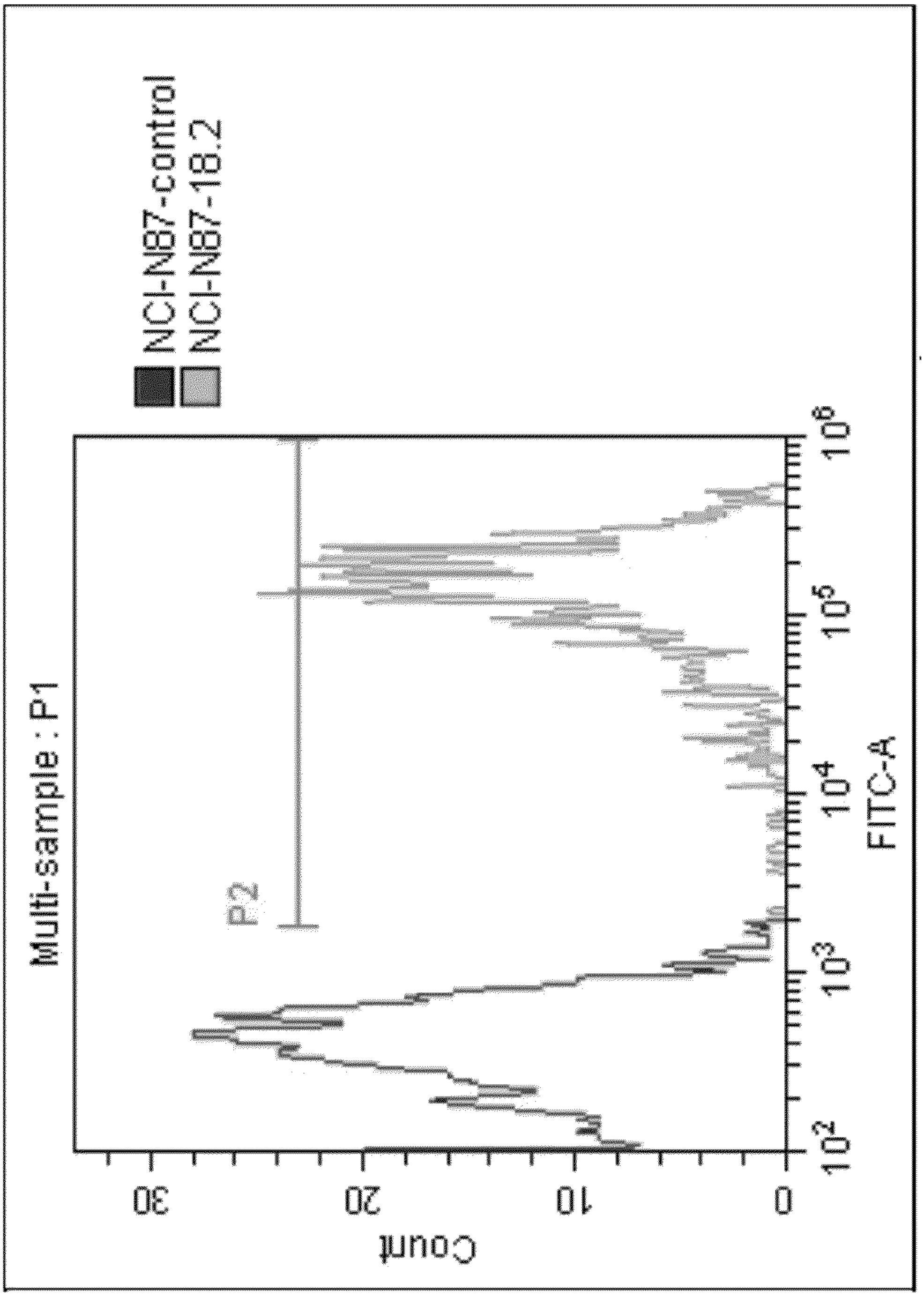
Multi-sample : P1
NCI-N87-control
NCI-N87-18.2
P2
Count
0
10
20
30
FITC-A
$10^2$
$10^3$
$10^4$
$10^5$
$10^6$

Fig. 1D

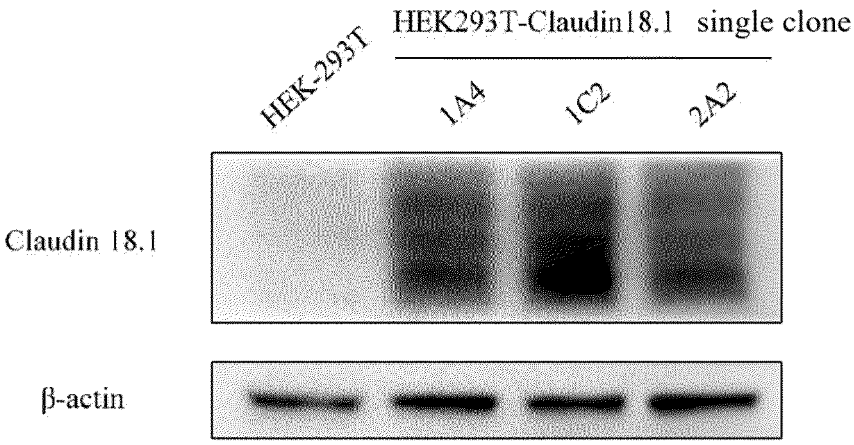
HEK-293T
HEK293T-Claudin18.1 single clone
1A4
1C2
2A2
Claudin 18.1
β-actin

Fig. 1E

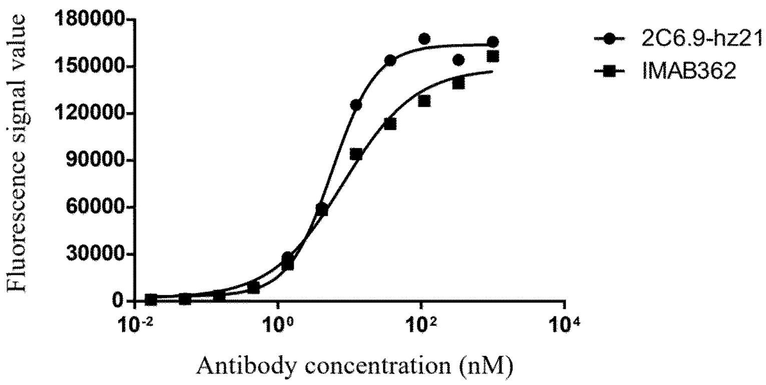
Fluorescence signal value
180000
150000
120000
90000
60000
30000
0
10-2
100
102
104
Antibody concentration (nM)
2C6.9-hz21
IMAB362

Fig. 2

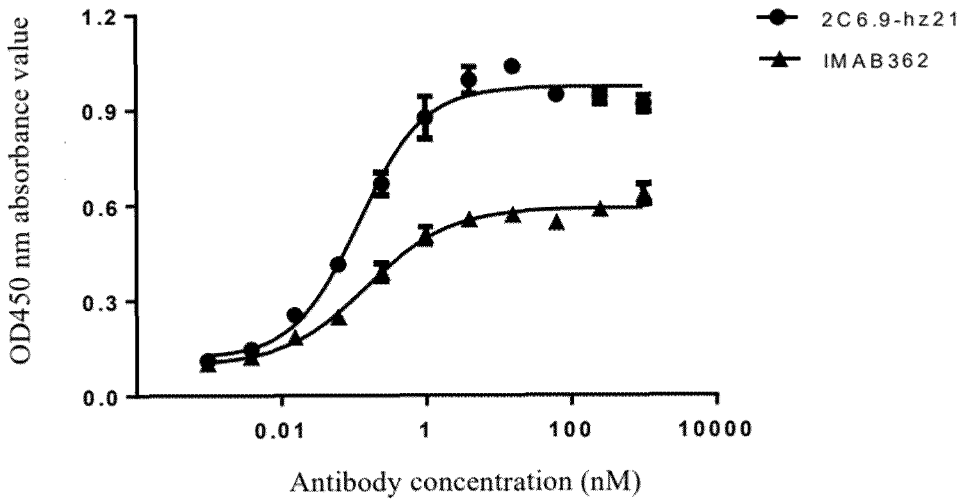
OD450 nm absorbance value
1.2
0.9
0.6
0.3
0.0
0.01
1
100
10000
Antibody concentration (nM)
2C6.9-hz21
IMAB362

Fig. 3

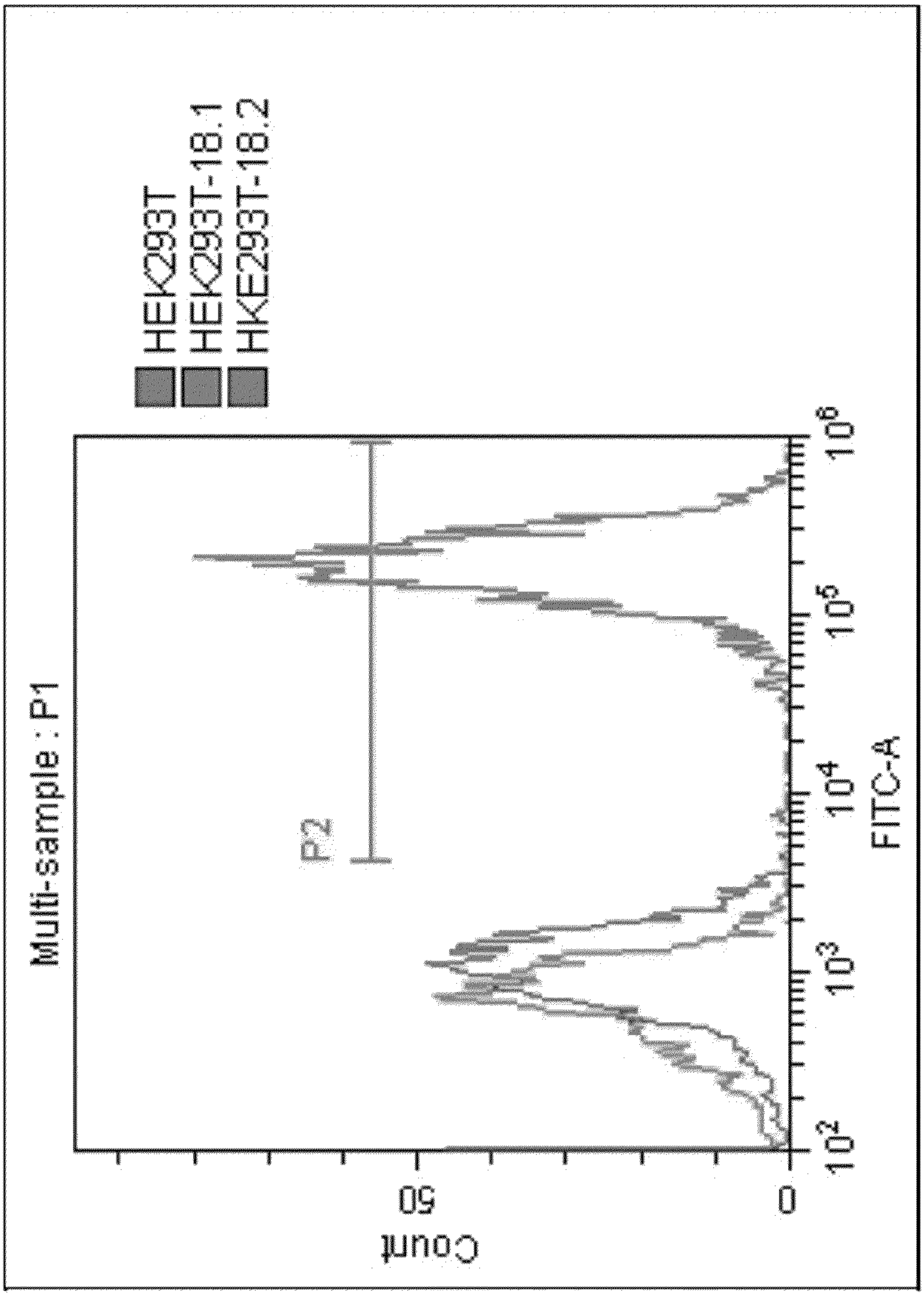
Multi-sample : P1
HEK293T
HEK293T-18.1
HKE293T-18.2
P2
Count
50
0
$10^2$
$10^3$
$10^4$
$10^5$
$10^6$
FITC-A

Fig. 4

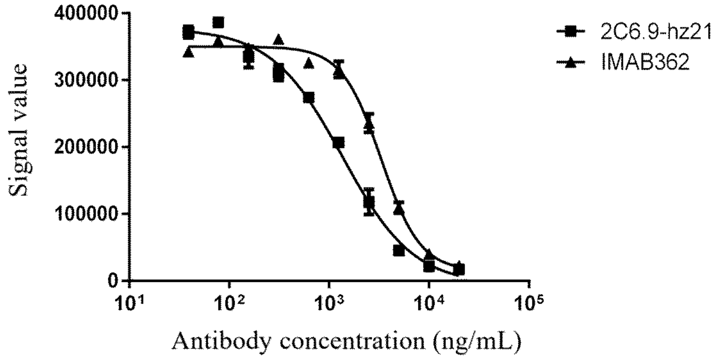

Signal value
400000
300000
200000
100000
0
10¹
10²
10³
10⁴
10⁵
Antibody concentration (ng/mL)
2C6.9-hz21
IMAB362


Fig. 5

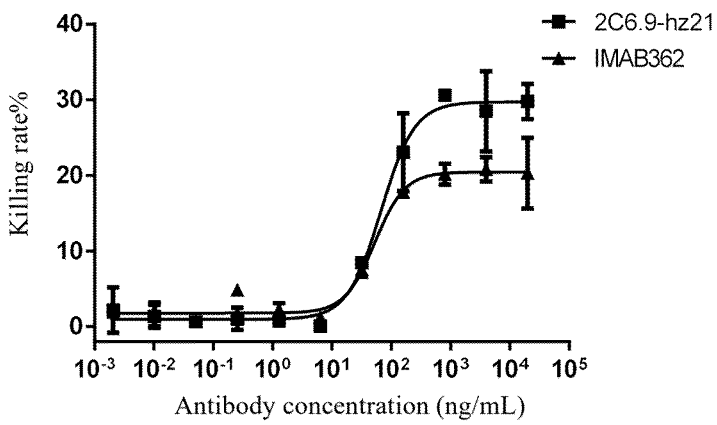

Killing rate%
40
30
20
10
0
10⁻³
10⁻²
10⁻¹
10⁰
10¹
10²
10³
10⁴
10⁵
Antibody concentration (ng/mL)
2C6.9-hz21
IMAB362


Fig. 6

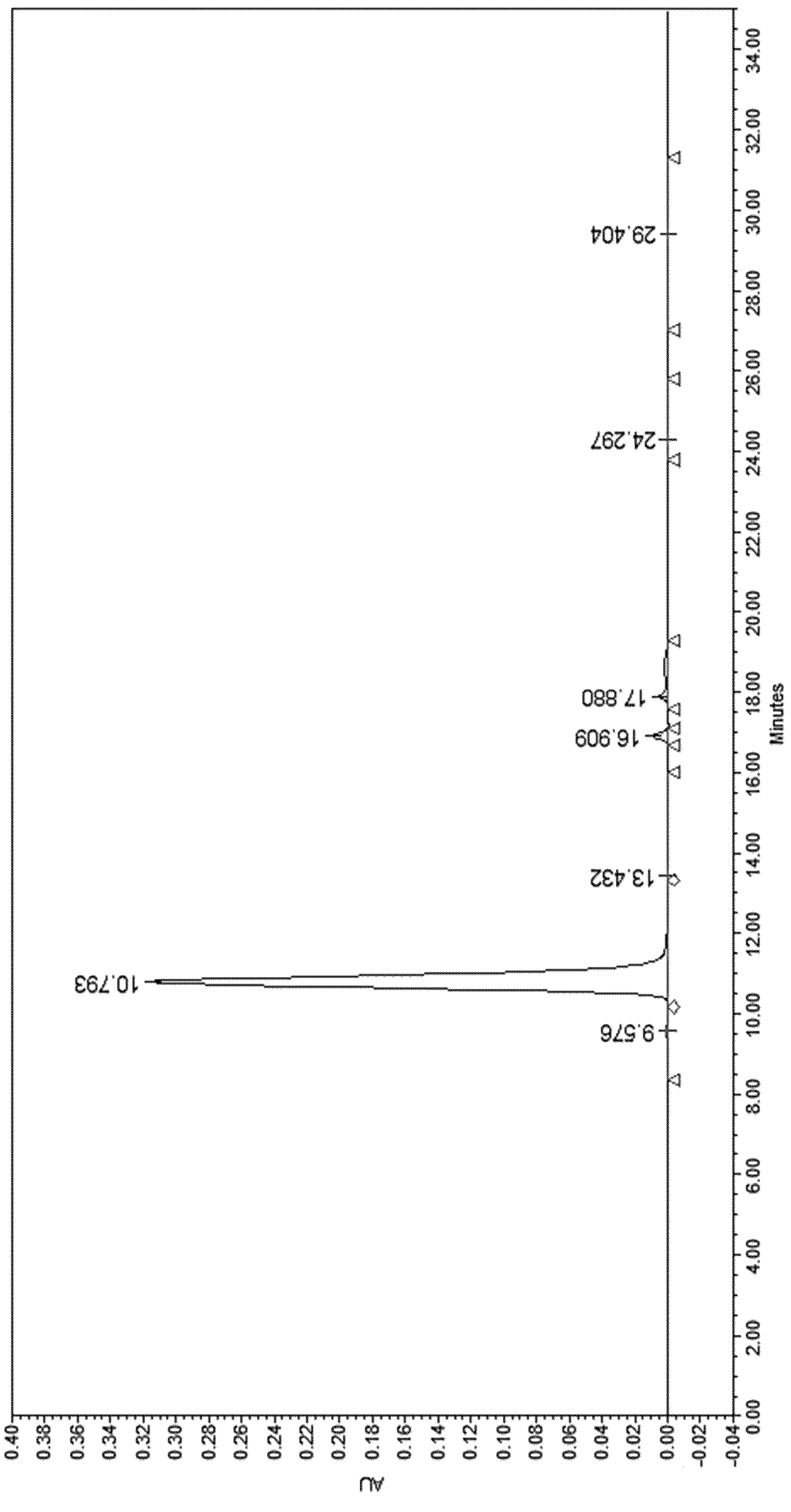


Fig. 7

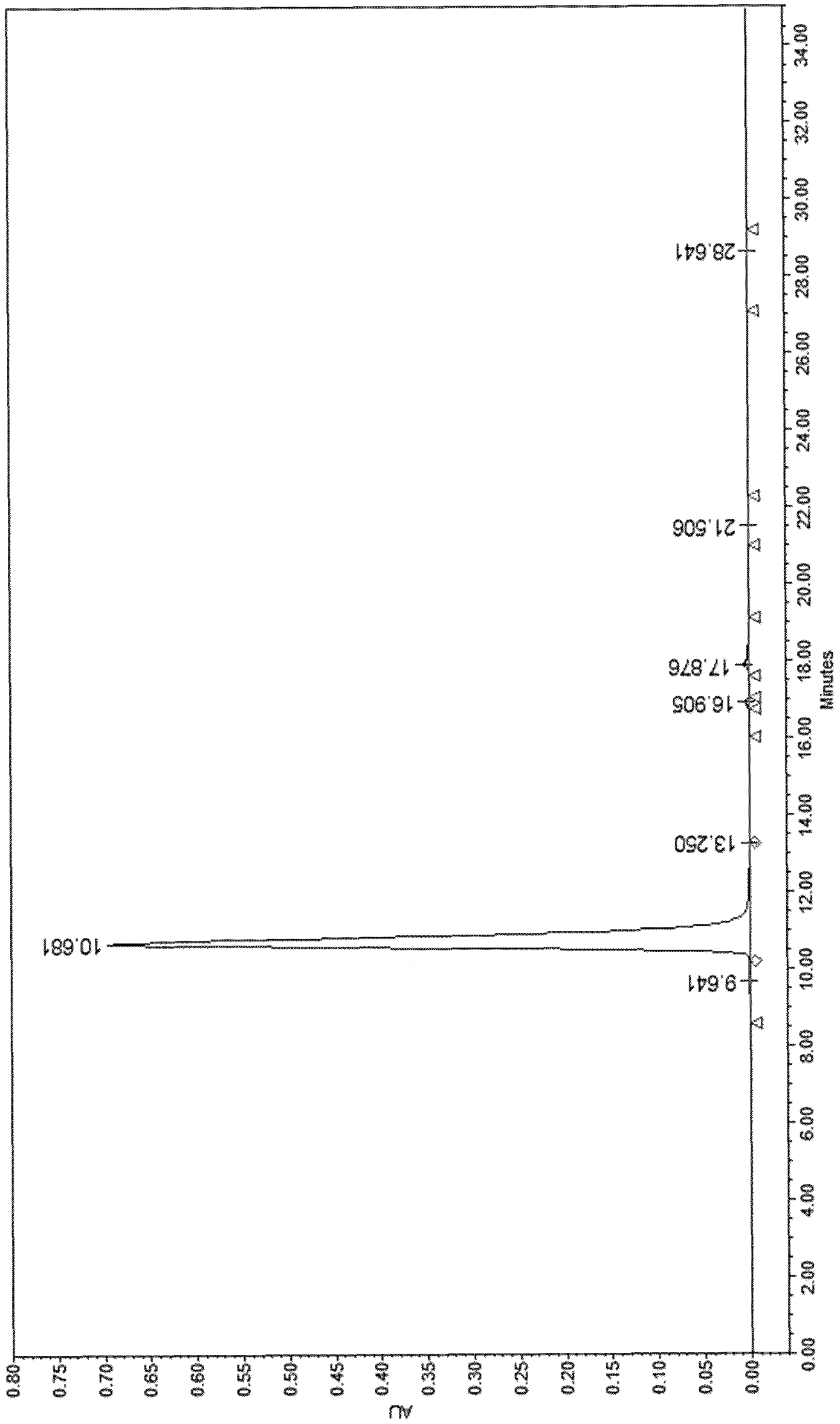


Fig. 8

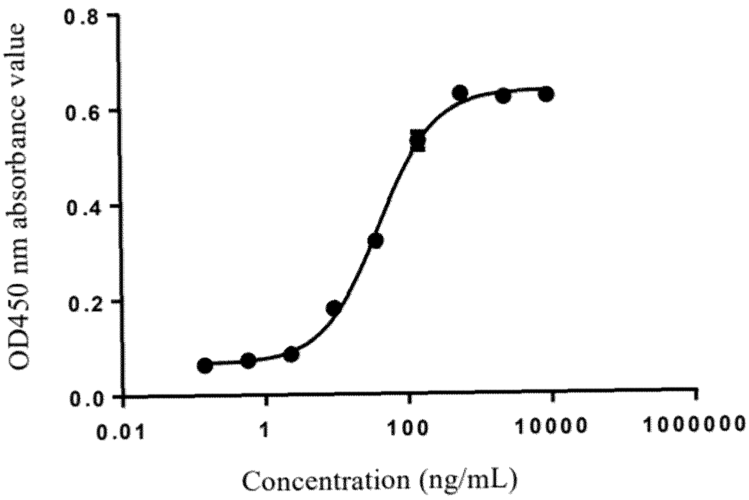
0.8
0.6
0.4
0.2
0.0
OD450 nm absorbance value
0.01
1
100
10000
1000000
Concentration (ng/mL)

Fig. 9

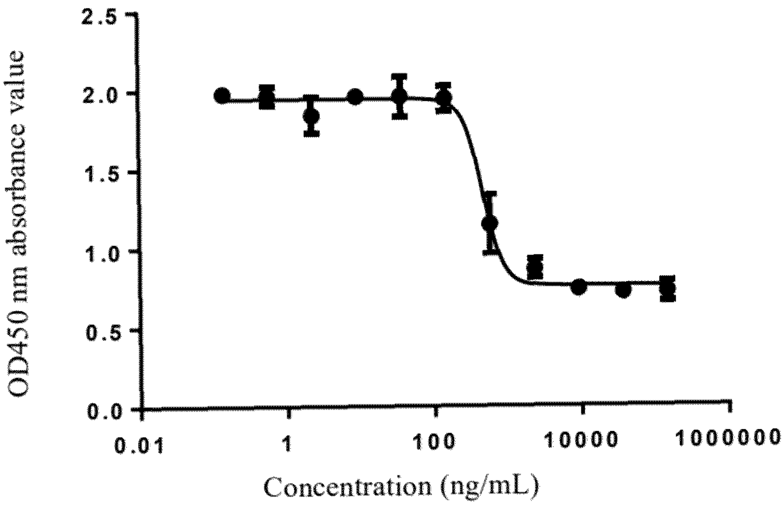
2.5
2.0
1.5
1.0
0.5
0.0
OD450 nm absorbance value
0.01
1
100
10000
1000000
Concentration (ng/mL)

Fig. 10A

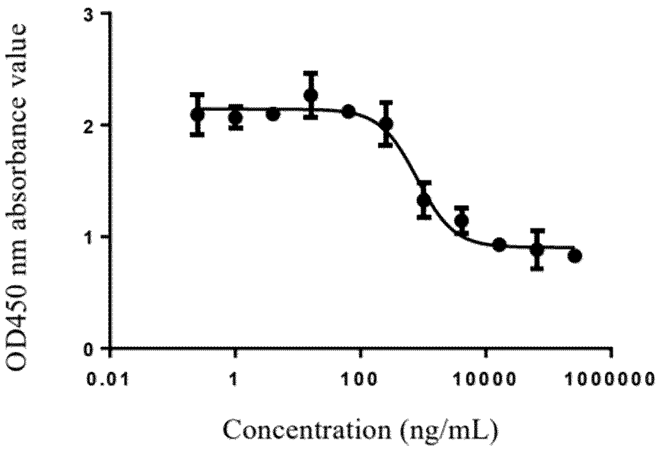


Fig. 10B

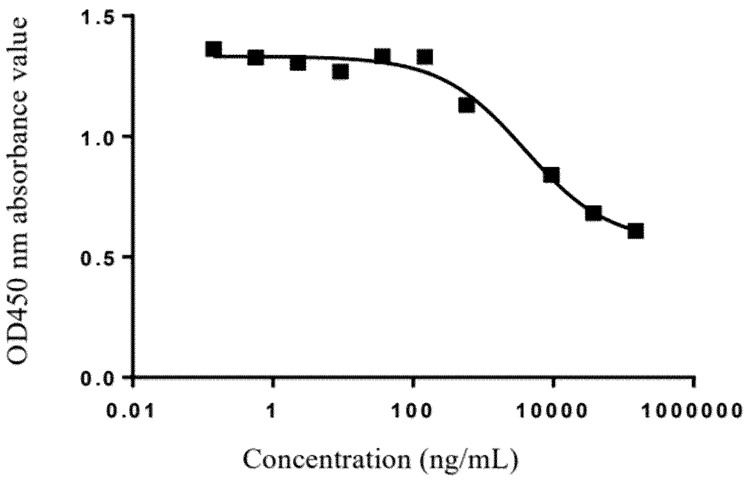


Fig. 10C

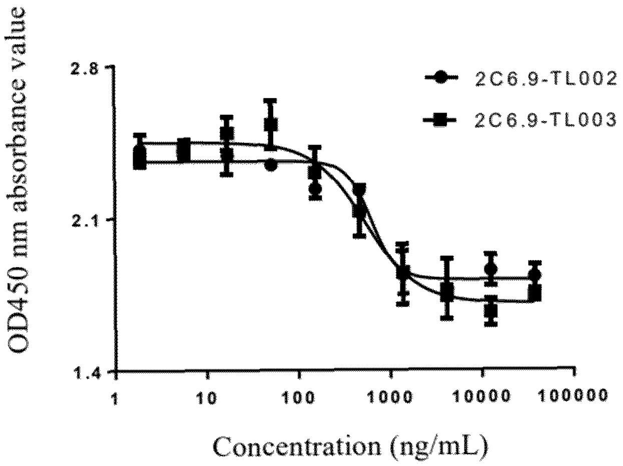


Fig. 10D

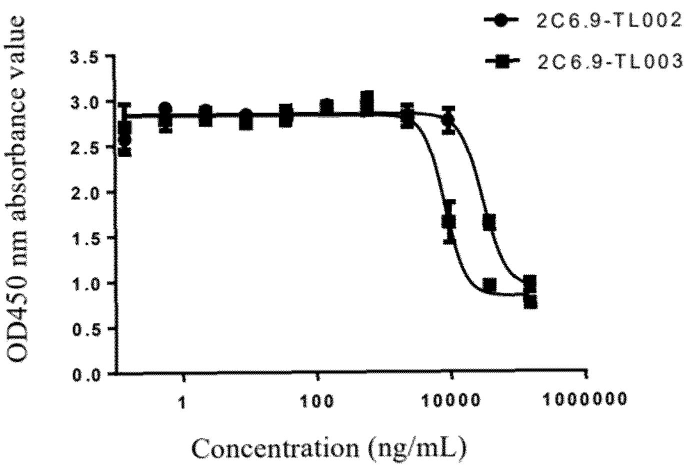


Fig. 10E

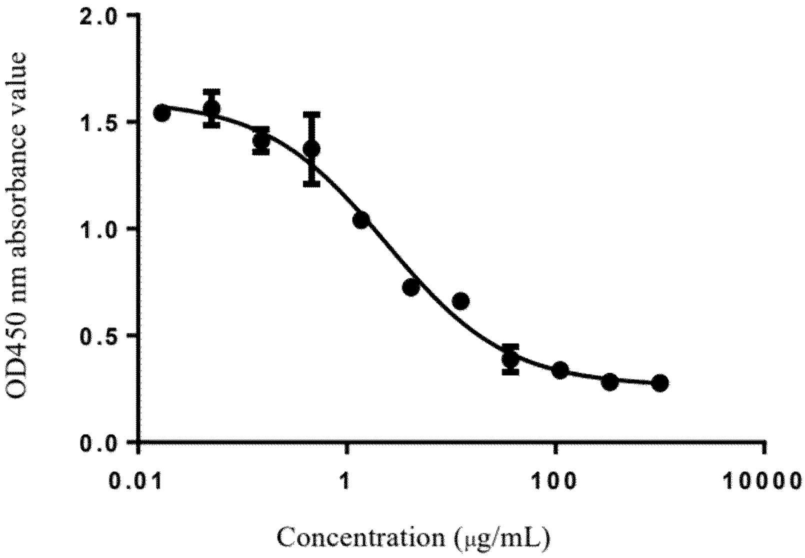


Fig. 11A

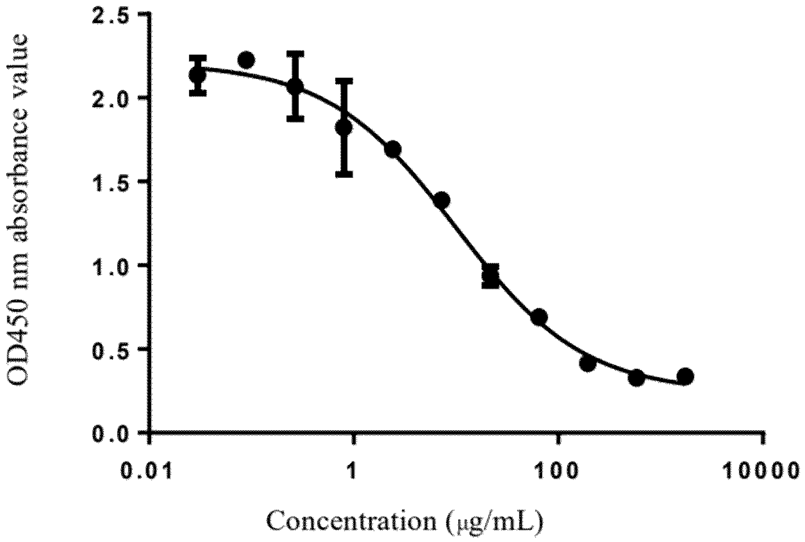


Fig. 11B

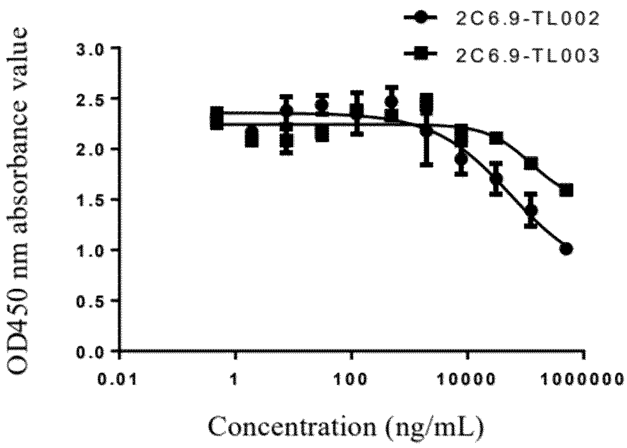


Fig. 11C

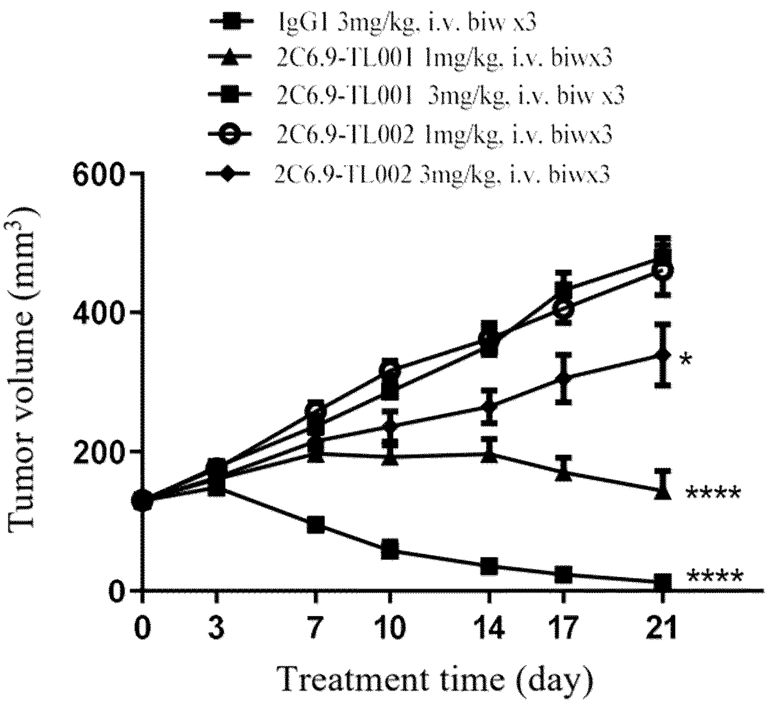


Fig. 12A

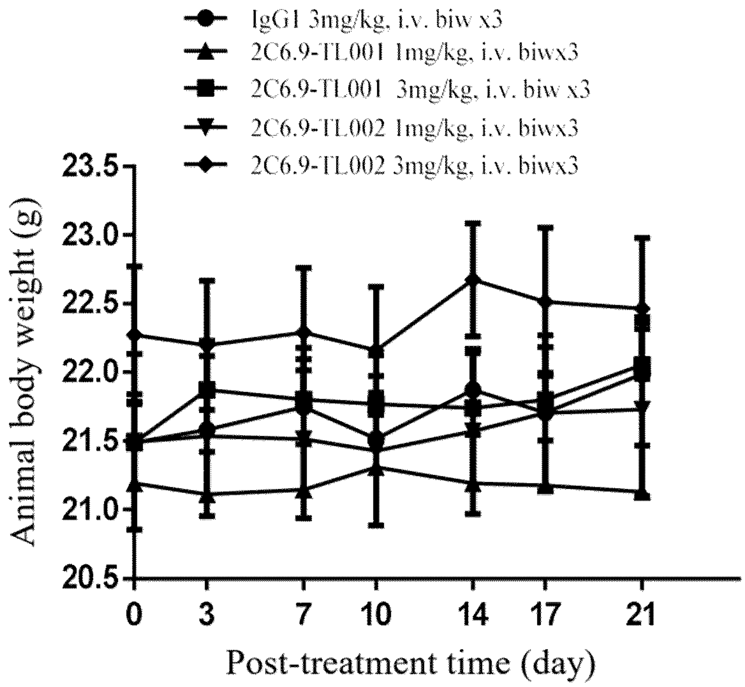


Fig. 12B

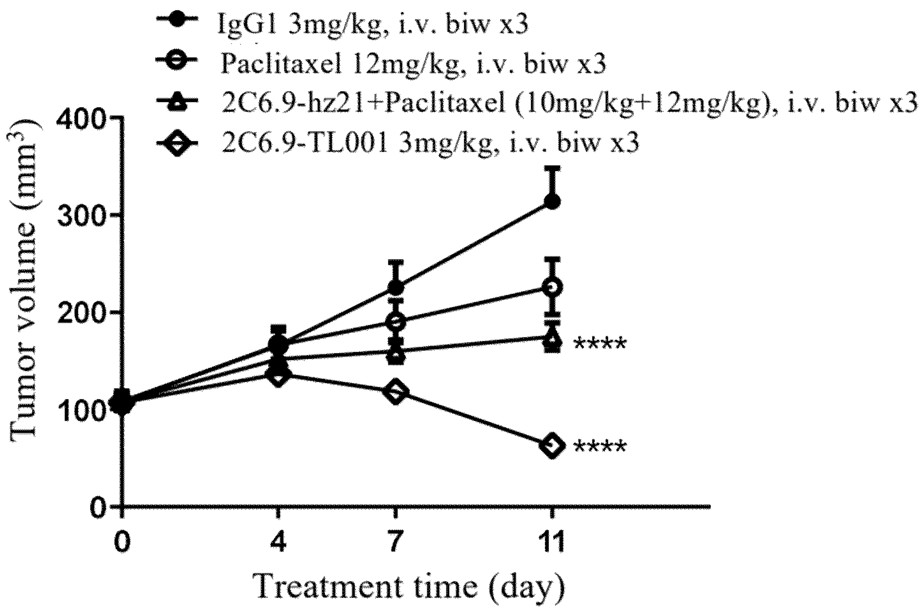


Fig. 12C

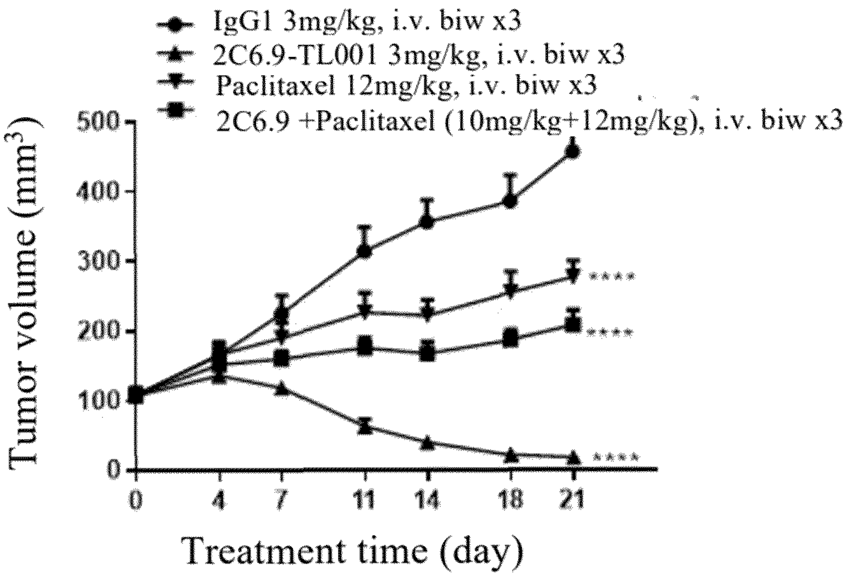


Fig. 12D

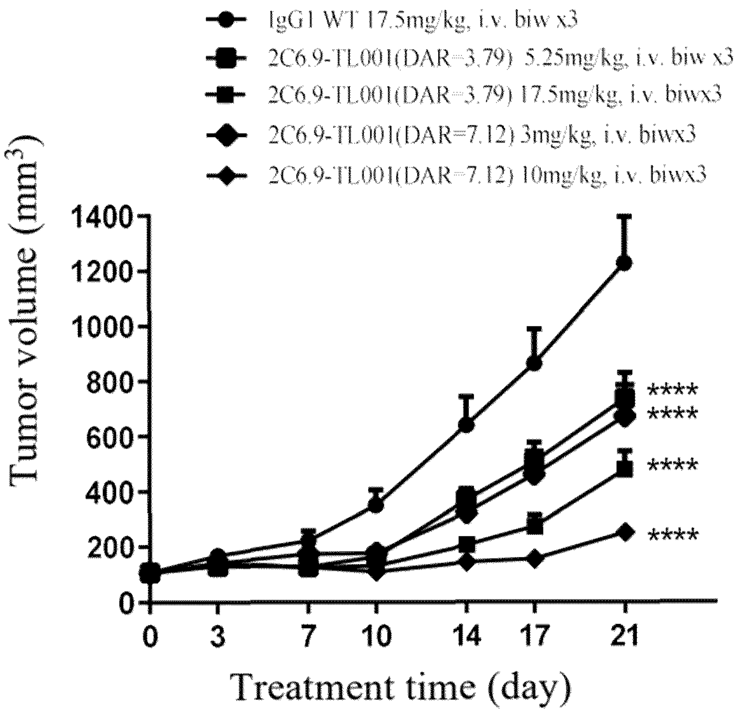


Fig. 12E

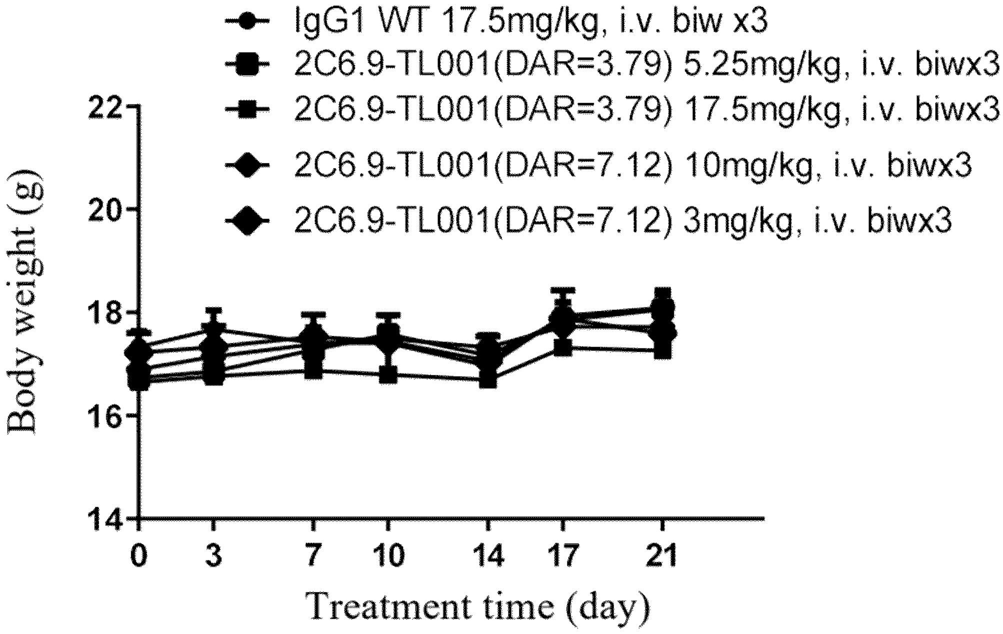


Fig. 12F

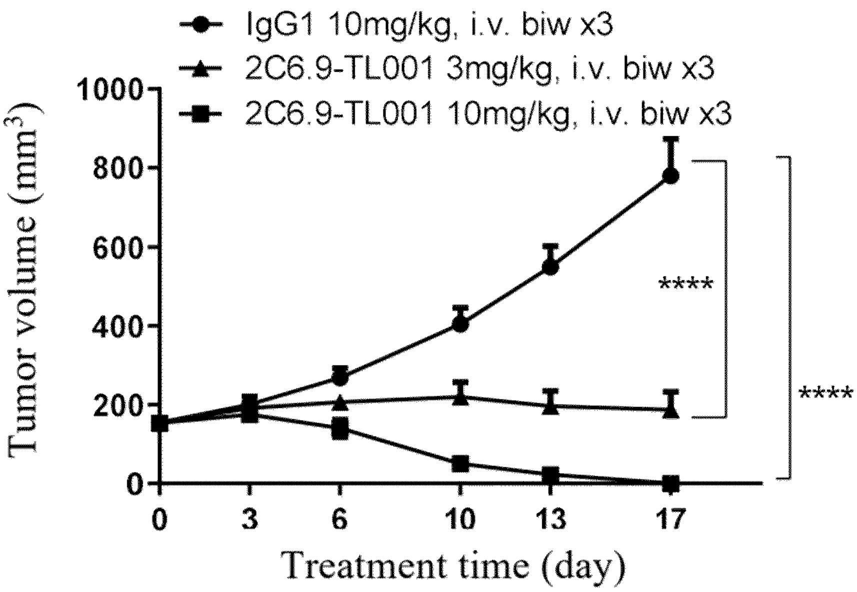


Fig. 12G

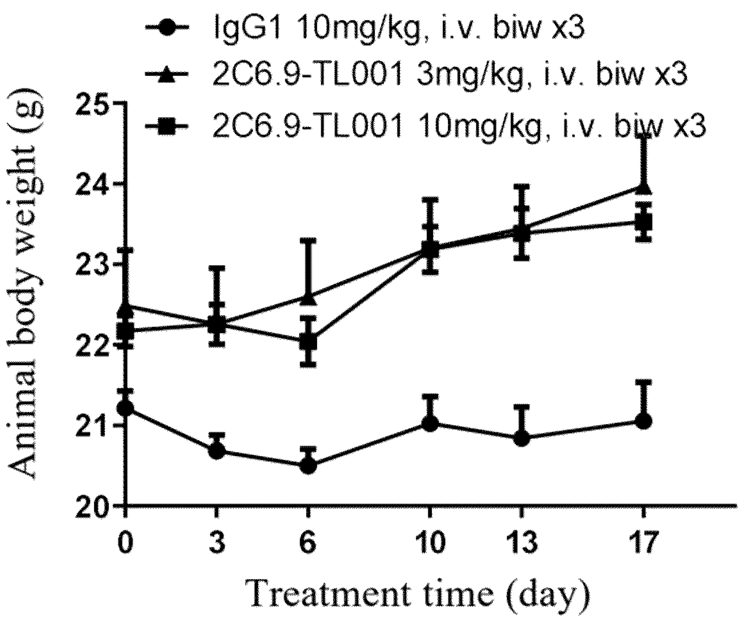


Fig. 12H

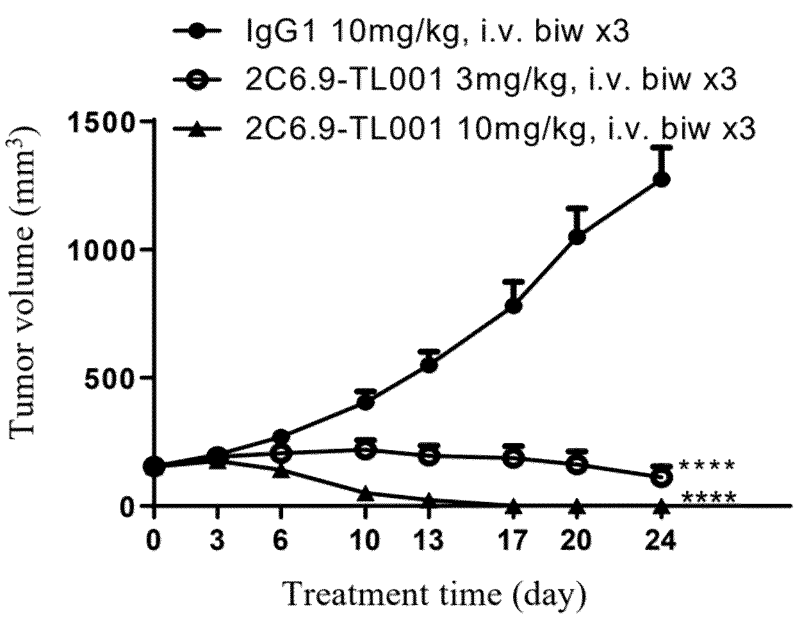


Fig. 12I

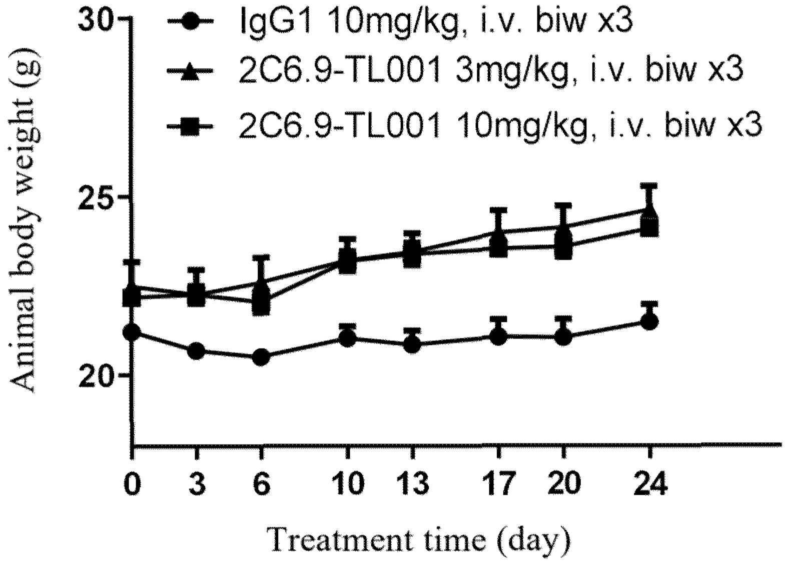


Fig. 12J

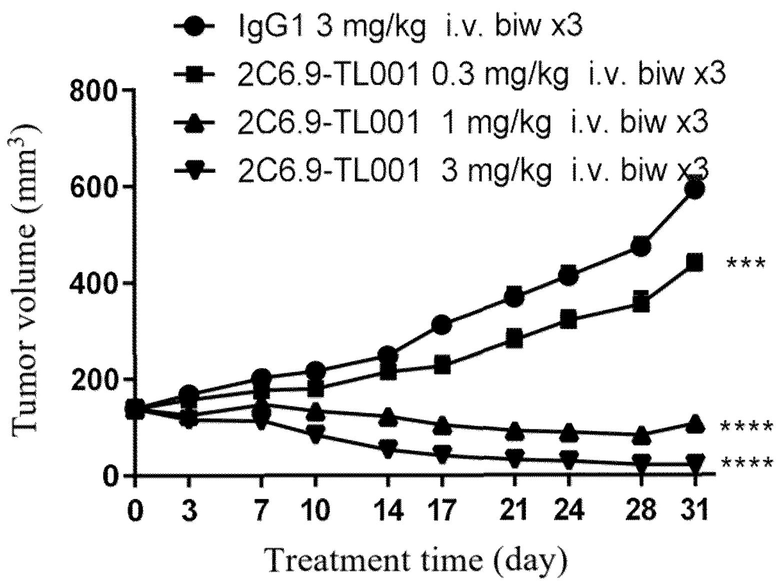


Fig. 12K

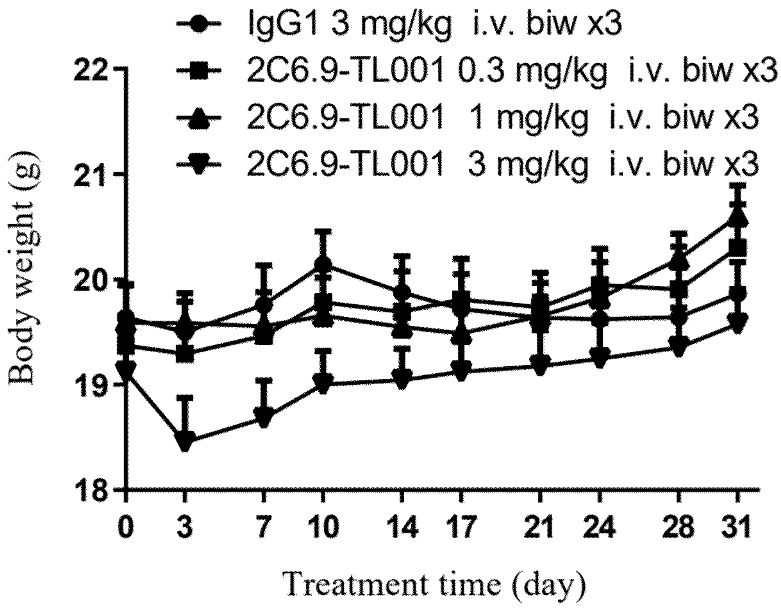


Fig. 12L

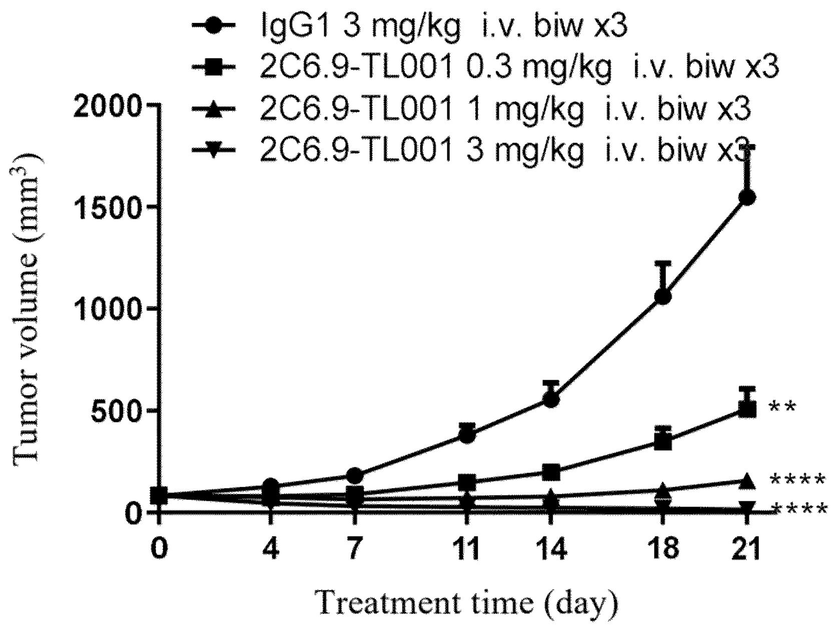


Fig. 12M

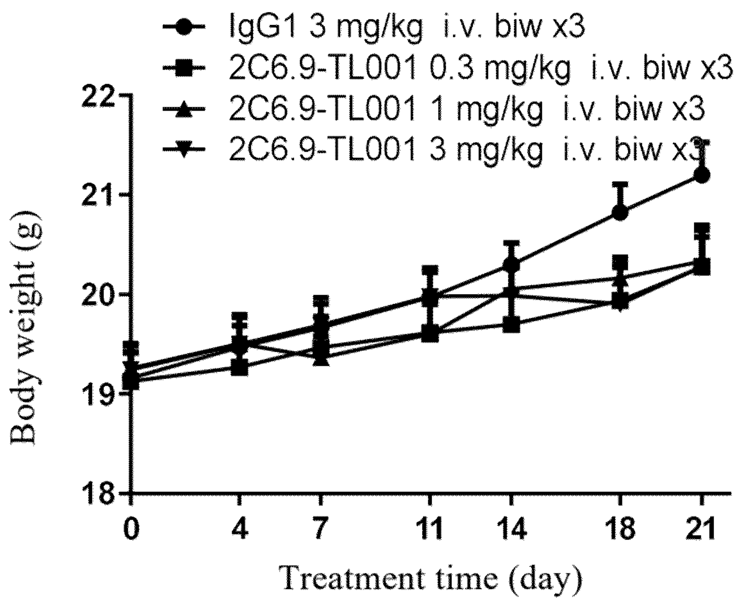


Fig. 12N

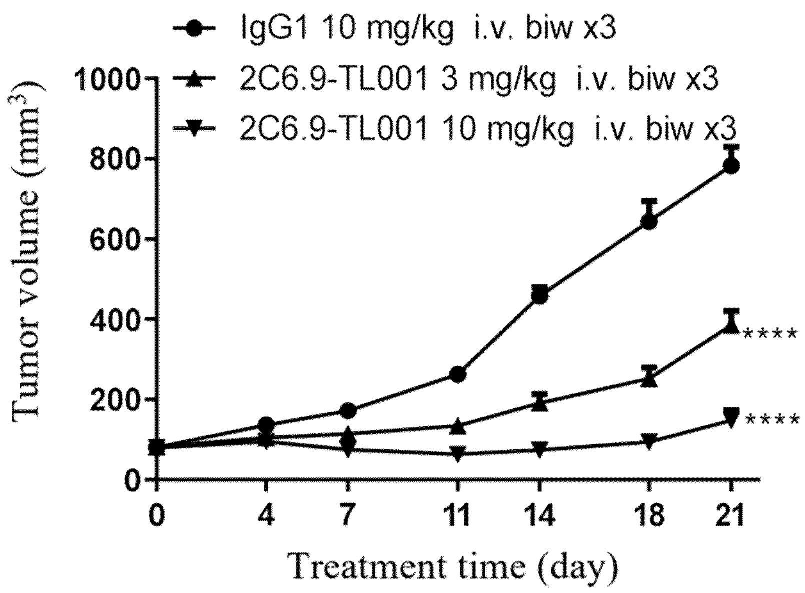


Fig. 12O

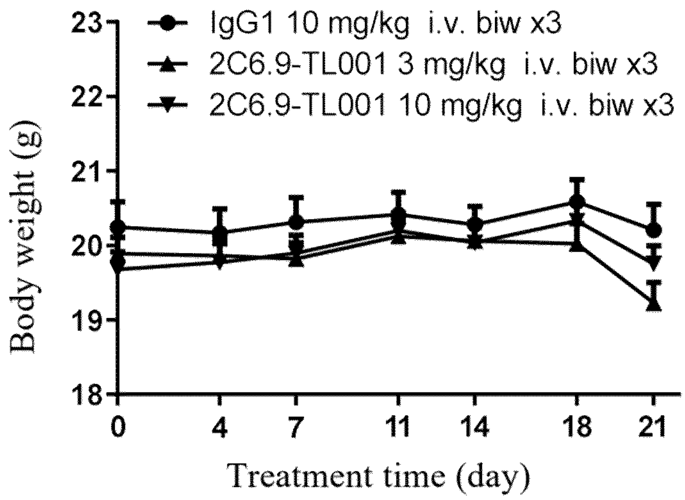
IgG1 10 mg/kg i.v. biw x3
2C6.9-TL001 3 mg/kg i.v. biw x3
2C6.9-TL001 10 mg/kg i.v. biw x3
Body weight (g)
23
22
21
20
19
18
0
4
7
11
14
18
21
Treatment time (day)

Fig. 12P

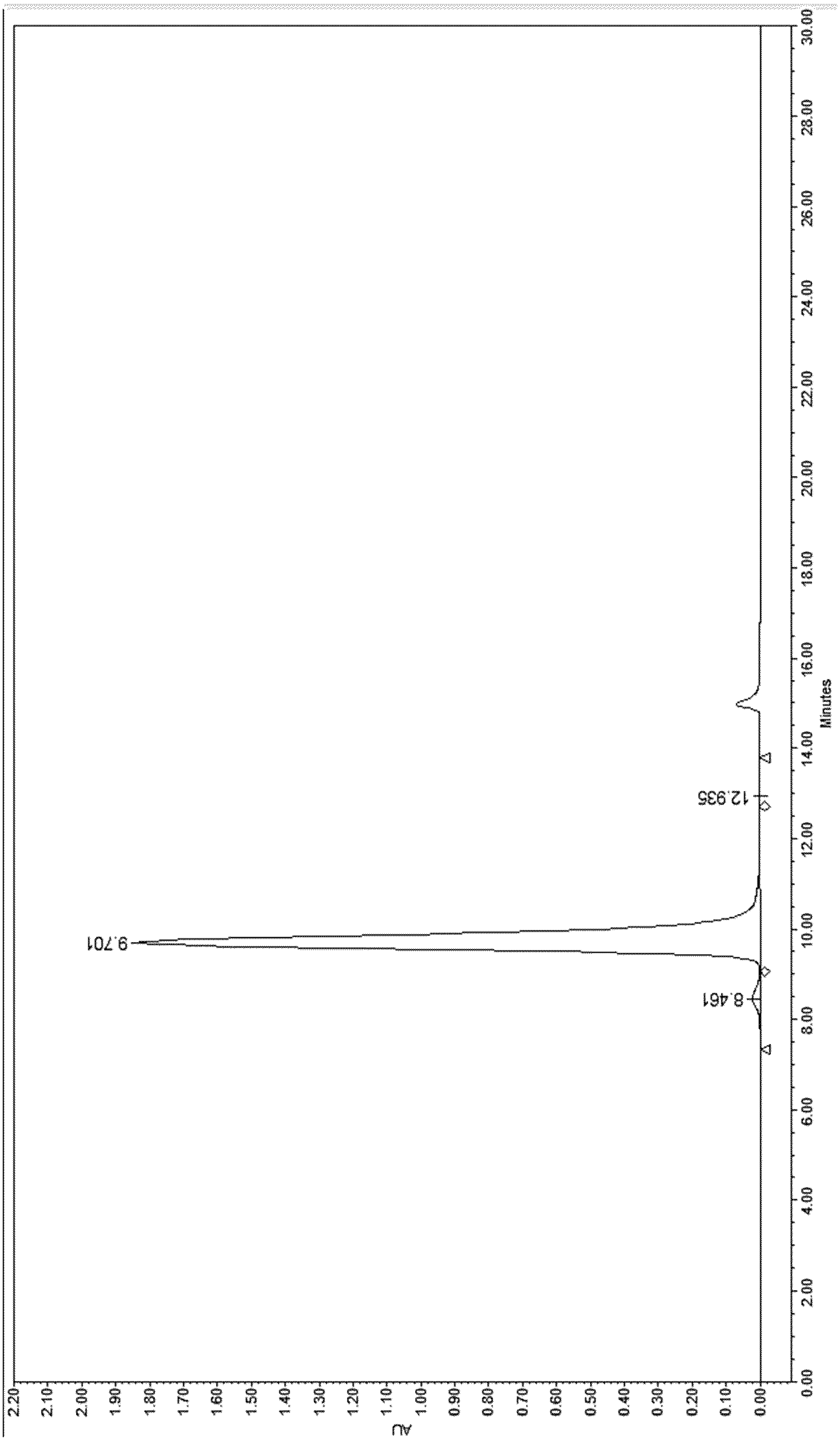


Fig. 13

**INTERNATIONAL SEARCH REPORT**

International application No.
**PCT/CN2021/093348**

**A. CLASSIFICATION OF SUBJECT MATTER**

A61K 47/68(2017.01)i; A61K 31/4745(2006.01)i; A61K 45/06(2006.01)i; A61K 49/00(2006.01)i; A61K 51/10(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; SIPOABS; DWPI; USTXT; WOTXT; EPTXT; CNKI; ISI Web of Science; STNext; Genbank; EMBL; Uniprot; 中国专利生物序列检索系统, Chinese Patent Biological Sequence Retrieval System: 四川科伦博泰生物, 肖亮, 紧密连接蛋白, 密蛋白, 密蛋白18.2, 密蛋白18A2, 抗体, 缀合物, 偶联物, 序列1-22, sequence 1-22, Sichuan kelun-biotech, Xiao liang, Claudin, CLDN, Claudin 18.2, CLDN18.2, Claudin 18A2, CLDN 18A2, antibody, conjugate, ADC

**C. DOCUMENTS CONSIDERED TO BE RELEVANT**

| Category* | Citation of document, with indication, where appropriate, of the relevant passages | Relevant to claim No. |
|---|---|---|
| PX | WO 2020135201 A1 (SICHUAN KELUN BIOTECH BIOPHARMACEUTICAL CO., LTD. et al.) 02 July 2020 (2020-07-02)<br>claims 1-11, 18 and 25-27 | 1-6, 19-24 |
| A | CN 111110862 A (SHANGHAI L&L VISION BIOPHARMACEUTICALS CO., LTD.) 08 May 2020 (2020-05-08)<br>claims 1, 6, 8 and 9 | 1-24 |
| A | WO 2019149116 A1 (SICHUAN KELUN BIOTECH BIOPHARMACEUTICAL CO., LTD.) 08 August 2019 (2019-08-08)<br>entire document | 1-24 |
| A | WO 2018006882 A1 (CARSGEN THERAPEUTICS CO., LTD. et al.) 11 January 2018 (2018-01-11)<br>entire document | 1-24 |
| A | WO 2020023679 A1 (ACCURUS BIOSCIENCES INC.) 30 January 2020 (2020-01-30)<br>entire document | 1-24 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 July 2021** | **02 August 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | ᅟNo. |

Form PCT/ISA/210 (second sheet) (January 2015)

**INTERNATIONAL SEARCH REPORT**

International application No.
**PCT/CN2021/093348**

**C. DOCUMENTS CONSIDERED TO BE RELEVANT**

| Category* | Citation of document, with indication, where appropriate, of the relevant passages | Relevant to claim No. |
|---|---|---|
| A | WO 2019154120 A1 (SICHUAN KELUN BIOTECH BIOPHARMACEUTICAL CO., LTD.) 15 August 2019 (2019-08-15)<br>entire document | 1-24 |
| A | WO 2019174617 A1 (BEIJING XUANYI PHARMASCIENCES CO., LTD.) 19 September 2019 (2019-09-19)<br>entire document | 1-24 |

**INTERNATIONAL SEARCH REPORT**

International application No.
**PCT/CN2021/093348**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2021/093348**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:

[1] Claim 24 relates to methods for preventing or treating tumors, delaying tumor progression, and reducing or inhibiting tumor recurrence, which is a method of treatment of the human body by therapy and therefore does not warrant an international search according to the criteria set out in PCT Rule 39.1(iv).

[2] A reasonably anticipated modification to claim 24 is as follows.

[3] Claim 24: the use of the antibody-drug adjuvant of any one of claims 1-19, the composition of claim 20, or the pharmaceutical composition of claim 21 in the preparation of a drug for preventing and/or treating a tumor, and/or delaying tumor progression, and/or reducing or inhibiting tumor recurrence.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/093348**

| Patent document cited in search report | Publication date (day/month/year) | Patent family member(s) | Publication date (day/month/year) |
|---|---|---|---|
| WO 2020135201 A1 | 02 July 2020 | None | |
| CN 111110862 A | 08 May 2020 | CN 110606891 A | 24 December 2019 |
| | | WO 2019242505 A1 | 26 December 2019 |
| | | CN 111867630 A | 30 October 2020 |
| | | CN 111848809 A | 30 October 2020 |
| | | CN 111518214 A | 11 August 2020 |
| WO 2019149116 A1 | 08 August 2019 | CN 111447939 A | 24 July 2020 |
| | | CN 110090308 A | 06 August 2019 |
| WO 2018006882 A1 | 11 January 2018 | AU 2017294276 A1 | 28 February 2019 |
| | | KR 20190038564 A | 08 April 2019 |
| | | US 2019233511 A1 | 01 August 2019 |
| | | CN 109790222 A | 21 May 2019 |
| | | RU 2019101430 A | 10 August 2020 |
| | | JP 2019531084 A | 31 October 2019 |
| | | EP 3483182 A1 | 15 May 2019 |
| | | BR 112019000327 A2 | 13 August 2019 |
| | | EP 3483182 A4 | 29 July 2020 |
| | | CA 3030257 A1 | 11 January 2018 |
| | | SG 11201900171 A1 | 27 February 2019 |
| | | IN 201937004589 A | 15 March 2019 |
| | | HK 40002280 A0 | 20 March 2020 |
| WO 2020023679 A1 | 30 January 2020 | None | |
| WO 2019154120 A1 | 15 August 2019 | CN 111542324 A | 14 August 2020 |
| WO 2019174617 A1 | 19 September 2019 | KR 20200130831 A | 20 November 2020 |
| | | CN 112166123 A | 01 January 2021 |
| | | EP 3765522 A1 | 20 January 2021 |
| | | CA 3087423 A1 | 19 September 2019 |
| | | AU 2019235033 A1 | 30 July 2020 |
| | | BR 112020014591 A2 | 01 December 2020 |
| | | US 20210009686 A1 | 14 January 2021 |
| | | SG 11202006217 A1 | 29 July 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010410633
- CN 2019126495 W
- US 3817837 A
- US 3850752 A
- US 3939350 A
- US 3996345 A
- US 4277437 A
- US 4275149 A
- US 4366241 A
- CN 104341529 A
- WO 2015141862 A1
- US 4816567 A
- US 5225539 A, Winter
- US 5530101 A
- US 5585089 A
- US 5693762 A
- US 6180370 B, Queen
- WO 0243478 A
- CN 101312989 B


### Non-patent literature cited in the description


- **MARTIN ACR ; CHEETHAM JC ; REES AR.** Modelling antibody hypervariable loops: A combined algorithm. *Proc Natl Acad Sci USA,* 1989, vol. 86, 9268-9272
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **CHOTHIA et al.** *Nature,* 1989, vol. 342, 878-883
- **LEFRANC et al.** *Dev. Comparat. Immunol.,* 2003, vol. 27, 55-77
- **KABAT.** Sequences of Proteins of Immunological Interest. National Institutes of Health, 1987
- Protein sequence and structure analysis of antibody variable domains[M. **MARTIN AC R.** Antibody engineering. Springer, 2010, 33-51
- Fundamental Immunology. Raven Press, 1989
- **HOLLIGER et al.** *Nat Biotechnol,* 2005, vol. 23, 1126-1136
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **PLUCKTHUN.** The Pharmacology of Monoclonal Antibodies. Springer-Verlag, 1994, vol. 113, 269-315
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **HOLLIGER P. et al.** *Proc. Natl. Acad. Sci. USA,* 1993, vol. 90, 6444-6448
- **POLJAK RJ et al.** *Structure,* 1994, vol. 2, 1121-1123
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **GODING.** Monoclonal Antibodies: Principles and Practice. Academic Press, 1996, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.,* 1980, vol. 107, 220
- **D. WILKINSON.** The Scientist. The Scientist, Inc, 17 April 2000, vol. 14, 25-28
- **MORRISON et al.** *Proc. Natl. Acad. Sci. USA,* 1984, vol. 81, 6851-6855
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **CLARK.** *Immunol. Today,* 2000, vol. 21, 397-402

- **KABAT, EA et al.** Sequences of Proteins of Immunological Interest. U.S. Department of Health and Human Services, 1991
- **KABAT, EA et al.** Sequences of Proteins of Immunological Interest. US Department of Health and Human Services, 1991
- Antibody Engineering: Methods and Protocols. Humana Press, 2004, vol. 248
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGERMANN et al.** *Year in Immunology,* 1993, vol. 7, 33
- **DUCHOSAL et al.** *Nature,* 1992, vol. 355, 258
- **LONBERG et al.** *Nature,* 1994, vol. 368 (6474), 856-859
- **HOOGENBOOM et al.** *J. Mol. Biol.,* 1991, vol. 227, 381
- **VAUGHAN et al.** *Nature Biotech,* 1996, vol. 14, 309
- **SMITH TF ; WATERMAN MS.** *Adv.Appl.Math.,* 1981, vol. 2, 482
- **HIGGINS DG ; SHARP PM.** *CABIOS,* 1989, vol. 5, 151
- **ALTSCHUL SF et al.** *Nature Genet.,* 1994, vol. 6, 119
- **MALMQVIST M.** *Nature,* 1993, vol. 361, 186-187
- **DAVIES et al.** *Annual Rev Biochem,* 1990, vol. 59, 439-473
- **NEEDLEMAN et al.** *J. Mol. Biol.,* 1970, vol. 48, 443-453
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc. Natl Acad. Set USA,* 1997, vol. 94, 412-417
- Immunology-A Synthesis. Sinauer Associates, 1991
- Remington’s Pharmaceutical Sciences. Gennaro AR, 1995
- **MOHAMMADI Z.** *Mol Biotechnol.,* September 2015, vol. 57 (9), 793-800